(19) Europäisches Patentamt
European Patent Office
Office européen des brevets

(11) Veröffentlichungsnummer: **0 332 579 B1**

(12) # EUROPÄISCHE PATENTSCHRIFT

(45) Veröffentlichungstag der Patentschrift: **10.08.94**

(51) Int. Cl.5: **C07D 401/12**, C07D 213/81, A01N 43/54, A01N 43/58, A01N 43/36

(21) Anmeldenummer: **89810160.5**

(22) Anmeldetag: **01.03.89**

(54) **Mittel zum Schutz von Pflanzen gegen Krankheiten.**

(30) Priorität: **09.03.88 CH 887/88**

(43) Veröffentlichungstag der Anmeldung:
**13.09.89 Patentblatt 89/37**

(45) Bekanntmachung des Hinweises auf die Patenterteilung:
**10.08.94 Patentblatt 94/32**

(84) Benannte Vertragsstaaten:
**AT BE CH DE ES FR GB IT LI NL**

(56) Entgegenhaltungen:
**EP-A- 0 139 613**
**DE-A- 2 745 833**

(73) Patentinhaber: **CIBA-GEIGY AG**
**Klybeckstrasse 141**
**CH-4002 Basel (CH)**

(72) Erfinder: **Zondler, Helmut, Dr.**
**Oberwilerstrasse 49**
**CH-4103 Bottmingen (CH)**
Erfinder: **Meyer, Alfred, Dr.**
**St. Galler-Ring 220**
**CH-4054 Basel (CH)**
Erfinder: **Eckhardt, Wolfgang, Dr.**
**Breslauerstrasse 14**
**D-7850 Lörrach (DE)**
Erfinder: **Kunz, Walter, Dr.**
**Buchenstrasse 9**
**CH-4104 Oberwil (CH)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue mit Heterocyclen substituierte Isonicotinsäureamide der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

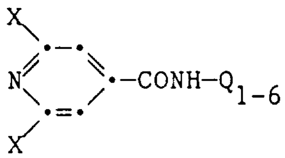

(I),

in welcher bedeuten:
X in ortho/ortho'-Stellung gleiche Atome Fluor, Chlor, Brom oder Jod, vorzugsweise Fluor, Chlor oder Brom und besonders bevorzugt Chlor;
$Q_1$ unsubstituiertes oder durch $R_1$, $R_2$ und $R_3$ substituiertes Pyrimidin-4-yl;
$Q_2$ unsubstituiertes oder durch $R_1$, $R_2$ und $R_3$ substituiertes Pyrimidin-2-yl;
$Q_3$ unsubstituiertes oder durch $R_1$, $R_2$ und $R_3$ substituiertes Pyrimidin-5-yl;
$Q_4$ unsubstituiertes oder durch $R_1$, $R_2$ und $R_3$ substituiertes Pyridin-2-yl, Pyridin-3-yl oder Pyridin-4-yl;
$Q_5$ unsubstituiertes oder durch $R_1$, $R_2$ und $R_3$ substituiertes Pyridazin-3-yl oder Pyridazin-4-yl;
$Q_6$ unsubstituiertes oder durch $R_1$, $R_2$ und $R_3$ substituiertes Pyrazin-2-yl;
$R_1$, $R_2$, $R_3$ Wasserstoff, Halogen, $C_1$-$C_6$-Alkyl, $C_1$-$C_6$-Haloalkyl mit 1 bis 3 Halogenatomen, $C_1$-$C_6$-Alkoxy, $C_2$-$C_6$-Alkoxyalkyl, $C_1$-$C_6$-Thioalkyl, $C_1$-$C_6$-Haloalkoxy mit 1 bis 5 Halogenatomen, $C_3$-$C_6$-Alkenyloxy, $C_3$-$C_6$-Alkinyloxy, $C_3$-$C_6$-Thioalkenyl, $C_3$-$C_6$-Cycloalkyl, mit Methyl substituiertes $C_3$-$C_6$-Cycloalkyl, Nitro, Cyano, die Reste $CH(OR_4)_2$ oder $COOR_4$, wobei $R_4$ $C_1$-$C_4$-Alkyl darstellt; $R_1$, $R_2$, $R_3$ ferner Phenyl, Phenoxy, Thiophenyl, Benzyl, Benzyloxy oder Thiobenzyl; oder mindestens einmal mit $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Phenyl, Phenoxy, Thiophenyl, Benzyl, Benzyloxy oder Thiobenzyl; sowie $N(R_5)R_6$, wobei $R_5$ und $R_6$ unabhängig voneinander $C_1$-$C_6$-Alkyl darstellt; darüber hinaus Piperidinyl, Pyrrolidinyl, Morpholinyl, Imidazolyl oder Triazolyl, oder durch $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Piperidinyl, Pyrrolidinyl, Morpholinyl, Imidazolyl oder Triazolyl.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar:
Methyl, Ethyl sowie die Isomeren von Propyl, Butyl, Pentyl oder Hexyl, wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1) oder Pentinyl-(4).

Cycloalkyl bedeutet wahlweise Cyclopropyl, Cyclobutyl, Cyclopentyl oder Cyclohexyl, vorzugsweise Cyclopropyl, Cyclopentyl oder Cyclohexyl.

Ein engerer Umfang der Erfindung wird dargestellt durch die Formel I, in welcher bedeuten:
X in ortho/ortho'-Stellung gleiche Atome Fluor, Chlor, Brom oder Jod;
$Q_1$ unsubstituiertes oder durch $R_1$, $R_2$ und $R_3$ substituiertes Pyrimidin-4-yl;
$Q_2$ unsubstituiertes oder durch $R_1$, $R_2$ und $R_3$ substituiertes Pyrimidin-2-yl;
$Q_3$ unsubstituiertes oder durch $R_1$, $R_2$ und $R_3$ substituiertes Pyrimidin-5-yl;
$Q_4$ unsubstituiertes oder durch $R_1$, $R_2$ und $R_3$ substituiertes Pyridin-2-yl, Pyridin-3-yl oder Pyridin-4-yl;
$Q_5$ unsubstituiertes oder durch $R_1$, $R_2$ und $R_3$ substituiertes Pyridazin-3-yl oder Pyridazin-4-yl;
$Q_6$ unsubstituiertes oder durch $R_1$, $R_2$ und $R_3$ substituiertes Pyrazin-2-yl;
$R_1$, $R_2$, $R_3$ Wasserstoff, Halogen, $C_1$-$C_6$-Alkyl, $C_1$-$C_6$-Haloalkyl mit 1 bis 3 Halogenatomen, $C_1$-$C_6$-Alkoxy, $C_2$-$C_6$-Alkoxyalkyl, $C_1$-$C_6$-Thioalkyl, $C_1$-$C_6$-Haloalkoxy mit 1 bis 5 Halogenatomen, $C_3$-$C_6$-Alkenyloxy, $C_3$-$C_6$-Alkinyloxy, $C_3$-$C_6$-Thioalkenyl, $C_3$-$C_6$-Cycloalkyl, Nitro, Cyano oder $COOR_4$, wobei $R_4$ $C_1$-$C_4$-Alkyl darstellt; ferner Phenyl, Phenoxy, Thiophenyl, Benzyl, Benzyloxy oder Thiobenzyl; oder mindestens einmal mit $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano

substituiertes Phenyl, Phenoxy, Thiophenyl, Benzyl, Benzyloxy oder Thiobenzyl; sowie $N(R_5)R_6$, wobei $R_5$ und $R_6$ unabhängig voneinander $C_1$-$C_6$-Alkyl darstellt; darüber hinaus Piperidinyl, Pyrrolidinyl, Morpholinyl, Imidazolyl oder Triazolyl, oder durch $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Piperidinyl, Pyrrolidinyl, Morpholinyl, Imidazolyl oder Triazolyl.

Aufgrund ihrer besonderen pflanzenschützenden Eigenschaften lassen sich die Verbindungen der Formel I in folgende Gruppen einteilen:

A) Verbindungen, in denen $Q_1$ durch $R_1$, $R_2$ und $R_3$ substituiertes Pyrimidin-4-yl darstellt und X, $R_1$, $R_2$ und $R_3$ die unter Formel I angegebenen Bedeutungen besitzen;

B) Verbindungen, in denen $Q_2$ durch $R_1$, $R_2$ und $R_3$ substituiertes Pyrimidin-2-yl darstellt und X, $R_1$, $R_2$ und $R_3$ die unter Formel I angegebenen Bedeutungen besitzen;

C) Verbindungen, in denen $Q_3$ durch $R_1$, $R_2$ und $R_3$ substituiertes Pyrimidin-5-yl darstellt und X, $R_1$, $R_2$ und $R_3$ die unter Formel I angegebenen Bedeutungen besitzen;

D) Verbindungen, in denen $Q_4$ durch $R_1$, $R_2$ und $R_3$ substituiertes Pyridin-2-yl, Pyridin-3-yl oder Pyridin-4-yl darstellt und X, $R_1$, $R_2$ und $R_3$ die unter Formel I angegebenen Bedeutungen besitzen;

E) Verbindungen, in denen $Q_5$ durch $R_1$, $R_2$ und $R_3$ substituiertes Pyridazin-3-yl oder Pyridazin-4-yl darstellt und X, $R_1$, $R_2$ und $R_3$ die unter Formel I angegebenen Bedeutungen besitzen;

F) Verbindungen, in denen $Q_6$ durch $R_1$, $R_2$ und $R_3$ substituiertes Pyrazin-2-yl darstellt und X, $R_1$, $R_2$ und $R_3$ die unter Formel I angegebenen Bedeutungen besitzen.

Wegen hervorragender biologischer Aktivität sind von den obengenannten Verbindungsgruppen folgende Untergruppen von Verbindungen der Formel I bevorzugt:

$A_1$) Verbindungen, in denen X in ortho/ortho'-Stellung gleiche Atome Fluor, Chlor, Brom oder Jod darstellt, $Q_1$ Pyrimidin-4-yl bedeutet und $R_1$, $R_2$ und $R_3$ als Substituenten in 2-, 5- oder 6-Stellung stehen für Wasserstoff, Halogen, $C_1$-$C_6$-Alkyl, $C_1$-$C_3$-Haloalkyl mit 1 bis 3 Halogenatomen, $C_1$-$C_4$-Alkoxy, $C_2$-$C_4$-Alkoxyalkyl, $C_1$-$C_4$-Thioalkyl, $C_1$-$C_4$-Haloalkoxy mit 1 bis 3 Halogenatomen, $C_3$-$C_4$-Alkenyloxy, $C_3$-$C_4$-Alkinyloxy, $C_3$-$C_4$-Thioalkenyl, $C_3$-$C_5$-Cycloalkyl, Nitro, Cyano oder $COOR_4$, wobei $R_4$ $C_1$-$C_4$-Alkyl bedeutet, ferner stehen für Phenyl, Phenoxy, Thiophenyl, Benzyl, oder mit 1 bis 3 Halogenatomen substituiertes Phenyl oder Phenyloxy; darüber hinaus für $N(R_5)R_6$, wobei $R_5$ und $R_6$ unabhängig voneinander $C_1$-$C_4$-Alkyl darstellen; sowie für Piperidinyl, Pyrrolidinyl, Morpholinyl, Imidazolyl oder Triazolyl, oder durch $C_1$-$C_3$-Alkyl oder Halogen substituiertes Piperidinyl, Pyrrolidinyl, Morpholinyl, Imidazolyl oder Triazolyl.

$A_2$) Verbindungen, in denen X in ortho/ortho'-Stellung gleiche Atome Fluor, Chlor oder Brom darstellt, $Q_1$ Pyrimidin-4-yl bedeutet und $R_1$, $R_2$ und $R_3$ in 2-, 5-oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Thioallylen, Cyclopropyl, Nitro, Cyano oder $COOR_4$, wobei $R_4$ $C_1$-$C_4$-Alkyl bedeutet; stehen ferner für Phenyl, Phenoxy, Thiophenyl, Benzyl, Dimethylamino, Piperidinyl, Pyrrolidinyl, Morpholinyl, Imidazolyl oder 1,2,4-Triazolyl.

$A_3$) Verbindungen, in denen X Chlor darstellt, $Q_1$ Pyrimidin-4-yl bedeutet und $R_1$, $R_2$ und $R_3$ in 2-, 5-oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl bedeutet; stehen ferner für Phenyl, Phenoxy, Thiophenyl, Benzyl, Dimethylamino.

$B_1$) Verbindungen, in denen X in ortho/ortho'-Stellung gleiche Atome Fluor, Chlor, Brom oder Jod darstellt, $Q_2$ Pyrimidin-2-yl bedeutet und $R_1$, $R_2$ und $R_3$ in 4-, 5- oder 6-Stellung stehen für Wasserstoff, Halogen, $C_1$-$C_4$-Alkyl, $C_1$-$C_2$-Haloalkyl mit 1 bis 3 Halogenatomen, $C_1$-$C_3$-Alkoxy, $C_1$-$C_4$-Thioalkyl, $C_1$-$C_2$-Haloalkoxy mit 1 bis 3 Halogenatomen, $C_3$-$C_4$-Alkenyloxy, $C_3$-$C_4$-Alkinyloxy, $C_3$-$C_5$-Cycloalkyl, Nitro, Cyano oder $COOR_4$, wobei $R_4$ $C_1$-$C_4$-Alkyl bedeutet; oder worin $R_1$, $R_2$, $R_3$ für Phenoxy, Thiophenyl, Thiobenzyl stehen; ferner für $N(R_5)R_6$, wobei $R_5$ und $R_6$ unabhängig voneinander $C_1$-$C_2$-Alkyl darstellen; sowie für Piperidinyl oder Morpholinyl oder durch $C_1$-$C_3$-Alkyl oder Chlor substituiertes Morpholinyl.

$B_2$) Verbindungen, in denen X in ortho/ortho'-Stellung gleiche Atome Fluor, Chlor oder Brom darstellt, $Q_2$ Pyrimidin-2-yl bedeutet und $R_1$, $R_2$ und $R_3$ in 4-, 5- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl bedeutet; stehen ferner für Phenyl, Phenoxy, Thiophenyl, Benzyl, Dimethylamino.

$B_3$) Verbindungen, in denen X Chlor darstellt, $Q_2$ Pyrimidin-2-yl bedeutet und $R_1$, $R_2$ und $R_3$ in 4-, 5-oder 6-Stellung stehen für Wasserstoff, Fluor, Chlor, Brom, $C_1$-$C_4$-Alkyl, $C_1$-$C_2$-Haloalkyl mit 1 bis 3

Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl bedeutet; stehen ferner für Phenyl, Phenoxy, Thiophenyl, Benzyl, Dimethylamino.

$C_1$) Verbindungen, in denen X in ortho/ortho'-Stellung gleiche Atome Fluor, Chlor, Brom oder Jod darstellen, $Q_3$ Pyrimidin-5-yl bedeutet und $R_1$, $R_2$ und $R_3$ stehen für Wasserstoff, Halogen, $C_1$-$C_4$-Alkyl, $C_1$-$C_2$-Haloalkyl mit 1 bis 3 Halogenatomen, $C_1$-$C_5$-Alkoxy, $C_2$-$C_4$-Alkoxyalkyl, $C_1$-$C_4$-Thioalkyl, $C_1$-$C_3$-Haloalkoxy mit 1 bis 4 Halogenatomen, $C_3$-$C_4$-Alkenyloxy, $C_3$-$C_4$-Alkinyloxy, $C_3$-$C_5$-Cycloalkyl, Phenoxy, Thiophenyl oder Benzyloxy, mit Halogen substituiertes Phenoxy, Thiophenyl oder Benzyloxy; ferner für $N(R_5)R_6$, wobei $R_5$ und $R_6$ unabhängig voneinander $C_1$-$C_2$-Alkyl darstellen; sowie für Pyrrolidinyl oder Morpholinyl.

$C_2$) Verbindungen, in denen X in ortho/ortho'-Stellung gleiche Atome Fluor, Chlor oder Brom darstellt, $Q_3$ Pyrimidin-5-yl bedeutet und $R_1$, $R_2$ und $R_3$ in 2-, 4- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl bedeutet; stehen ferner für Phenyl, Phenoxy, Thiophenyl, Benzyl, Dimethylamino.

$C_3$) Verbindungen, in denen X Chlor darstellt, $Q_3$ Pyrimidin-5-yl bedeutet und $R_1$, $R_2$ und $R_3$ in 2-, 4- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl bedeutet; stehen ferner für Phenyl, Phenoxy, Thiophenyl, Benzyl, Dimethylamino.

$D_1$) Verbindungen, in denen X in ortho/ortho'-Stellung gleiche Atome Fluor, Chlor, Brom oder Jod darstellt, $Q_4$ Pyridin-2-yl bedeutet und $R_1$, $R_2$ und $R_3$ in 3-, 4-, 5- oder 6-Stellung stehen für Wasserstoff, Halogen, $NO_2$, $C_1$-$C_4$-Alkyl, $C_1$-$C_2$-Haloalkyl mit 1 bis 3 Halogenatomen, $C_1$-$C_4$-Alkoxy oder $C_1$-$C_4$-Thioalkyl.

$D_2$) Verbindungen, in denen X in ortho/ortho'-Stellung gleiche Atome Fluor, Chlor, Brom oder Jod darstellt, $Q_4$ Pyridin-3-yl bedeutet und $R_1$, $R_2$ und $R_3$ in 2-, 4-, 5- oder 6-Stellung stehen für Wasserstoff, Halogen, $NO_2$, $C_1$-$C_4$-Alkyl, $C_1$-$C_2$-Haloalkyl mit 1 bis 3 Halogenatomen, $C_1$-$C_4$-Alkoxy oder $C_1$-$C_4$-Thioalkyl.

$D_3$) Verbindungen, in denen X in ortho/ortho'-Stellung gleiche Atome Fluor, Chlor, Brom oder Jod darstellt, $Q_4$ Pyridin-4-yl bedeutet und $R_1$, $R_2$ und $R_3$ in 2-, 3-, 5- oder 6-Stellung stehen für Wasserstoff, Halogen, $NO_2$, $C_1$-$C_4$-Alkyl, $C_1$-$C_2$-Haloalkyl mit 1 bis 3 Halogenatomen, $C_1$-$C_4$-Alkoxy oder $C_1$-$C_4$-Thioalkyl.

$E_1$) Verbindungen, in denen X in ortho/ortho'-Stellung gleiche Atome Fluor, Chlor, Brom oder Jod darstellt, $Q_5$ Pyridazin-3-yl bedeutet und $R_1$, $R_2$ und $R_3$ in 4-, 5- oder 6-Stellung stehen für Wasserstoff, Halogen, $C_1$-$C_4$-Alkyl, $C_1$-$C_4$-Alkoxy, $C_1$-$C_4$-Thioalkyl, $C_1$-$C_2$-Haloalkyl mit 1 bis 3 Halogenatomen.

$E_2$) Verbindungen, in denen X in ortho/ortho'-Stellung gleiche Atome Fluor, Chlor, Brom oder Jod darstellt, $Q_5$ Pyridazin-4-yl bedeutet und $R_1$, $R_2$ und $R_3$ in 3-, 5- und 6-Stellung stehen für Wasserstoff, Halogen, $C_1$-$C_4$-Alkyl, $C_1$-$C_4$-Alkoxy oder $C_1$-$C_2$-Haloalkyl mit 1 bis 3 Halogenatomen.

$F_1$) Verbindungen, in denen X in ortho/ortho'-Stellung gleiche Atome Fluor, Chlor, Brom oder Jod darstellt, $Q_6$ Pyrazin-2-yl bedeutet und $R_1$, $R_2$ und $R_3$ in 3-, 5- und 6-Stellung stehen für Wasserstoff, Halogen, $C_1$-$C_4$-Alkyl, $C_1$-$C_4$-Alkoxy oder $C_1$-$C_2$-Haloalkyl mit 1 bis 3 Halogenatomen.

Die folgenden Verbindungen zeichnen sich durch besonders vorteilhafte pflanzenschützende Eigenschaften aus:

N-(2-Cyclopropyl-5-ethyl-6-chlor-pyrimidin-4-yl)-2,6-dichlor-isonicotinsäureamid;
N-(2-Cyclopropyl-5-methyl-6-chlor-pyrimidin-4-yl)-2,6-dichlor-isonicotinsäureamid;
N-(2,5-Dimethyl-6-chlor-pyrimidin-4-yl)-2,6-dichlor-isonicotinsäureamid;
N-(2,5-Diethyl-6-chlor-pyrimidin-4-yl)-2,6-dichlor-isonicotinsäureamid;
N-(2-Clor-6-thiomethyl-pyrimidin-4-yl)-2,6-dichlor-isonicotinsäureamid;
N-(2-Methoxy-5-chlor-pyrimidin-4-yl)-2,6-dichlor-isonicotinsäureamid;
N-(2,5-Dichlor-pyrimidin-4-yl)-2-6-dichlor-isonicotinsäureamid;
N-(2,6-Dimethyl-pyrimidin-4-yl)-2,6-dichlor-isonicotinsäureamid;
N-(5-Methyl-6-chlor-pyrimidin-4-yl)-2,6-dichlor-isonicotinsäureamid;
N-(5-n-Propyl-6-chlor-pyrimidin-4-yl)-2,6-dichlor-isonicotinsäureamid;
N-(5-Ethyl-6-chlor-pyrimidin-4-yl)-2,6-dichlor-isonicotinsäureamid;
N-(2,5,6-Trichlor-pyrimidin-4-yl)-2,6-dichlor-isonicotinsäureamid;
N-(2-Dimethylamino-6-methoxy-pyrimidin-4-yl)-2,6-dichlor-isonicotinsäureamid;
N-(2-Ethyl-5-methyl-6-chlor-pyrimidin-4-yl)-2,6-dichlor-isonicotinsäureamid;

N-(2-Trifluormethoxy-5-chlor-pyrimidin-4-yl)-2,6-dichlor-isonicotinsäureamid;
N-(2-Methoxy-5-fluor-pyrimidin-4-yl)-2,6-dichlor-isonicotinsäureamid;
N-(2-Chlor-5-fluor-pyrimidin-4-yl)-2,6-dichlor-isonicotinsäureamid;
N-(2-Isopropoxy-5-chlor-pyrimidin-4-yl)-2,6-dichlor-isonicotinsäureamid;
N-(2-Isopropyl-6-chlor-pyrimidin-4-yl)-2,6-dichlor-isonicotinsäureamid;
N-(2-Chlor-6-n-propyl-pyrimidin-4-yl)-2,6-dichlor-isonicotinsäureamid;
N-(2-n-Propoxy-5-chlor-pyrimidin-4-yl)-2,6-dichlor-isonicotinsäureamid;
N-(2-Isopropyl-6-ethylthio-pyrimidin-4-yl)-2,6-dichlor-isonicotinsäureamid;
N-(2-Isopropyl-6-isopropylthio-pyrimidin-4-yl)-2,6-dichlor-isonicotinsäureamid;
N-(2-Chlor-6-methyl-pyrimidin-4-yl)-2,6-dichlor-isonicotinsäureamid;
N-Pyridin-2-yl-2,6-dichlor-isonicotinsäureamid;
N-(6-Methyl-pyridin-2-yl)-2,6-dichlor-isonicotinsäureamid;
N-(4-Methyl-pyridin-2-yl)-2,6-dichlor-isonicotinsäureamid;
N-(5-Chlor-pyridin-2-yl)-2,6-dichlor-isonicotinsäureamid;
N-Pyridin-4-yl-2,6-dichlor-isonicotinsäureamid.

2,6-Dihaloisonicotinsäure-Derivate sind bereits teilweise bekannt. So sind in der britischen Patentschrift Nr. 923,387 2,6-Dihaloisonicotinsäurederivate, z.B. aliphatische Amide als Herbizide beschrieben. Ferner sind in der USA-Patentschrift No. 4,137,067 und in der kanadischen Patentschrift No. 1,072,443 2,6-Dichlorisonicotinsäurehydrazide zur Bekämpfung phytopathogener Mikroorganismen offenbart. Darüber hinaus sind 2,6-Dihaloisonicotinsäure-Derivate als Tuberkulostatika bekannt geworden (vgl. Acta Fac. Pharm. Brun. Bratislav. 4, 65-66 [1962]; Chem. Abstr. Vol 57, 1962, 4769b).

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mirkoorganismen mittels Blattapplikation (direkte Wirkung) oder Bodenapplikation (systemische Wirkung) als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen gegen Schädlinge durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet.

Darüber hinaus bewirkt die den Verbindungen der Formel I zueigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizoctonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden: Pilze, wie z.B. Oomyceten (z.B. Plasmopara viticola, Phytophthora infestans, Peronospora tabacina, Pseudoperonospora), Fungi imperfecti (z.B. Colletotrichum lagenarium, Piricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen aus unterschiedlichen Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen,

Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomate, Weizen, Gerste, Birne und Apfel.

Die Verbindungen der Formel I werden durch Synthese aus 2,6-Dihalogenisonicotinsäurechloriden, -anhydriden oder -azoliden als Zwischenprodukte gewonnen:

Die Verbindungen der Formel I werden hergestellt, indem man umsetzt:

a) ein Isonicotinsäurehalogenid der Formel II

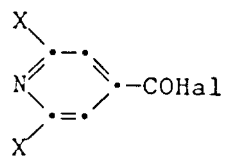

(II)

mit einem Amin der Formel III

$H_2N—Q_{1-6}$ (III)

in Gegenwart einer Base in einem inerten Lösungsmittel oder ohne Lösungsmittel; oder

b) ein Isonicotinsäureanhydrid der Formel IV

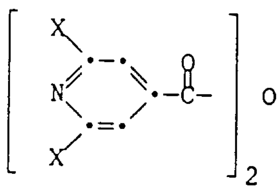

(IV)

mit einem Amin der Formel III

$H_2N—Q_{1-6}$ (III)

in Gegenwart einer Base in einem inerten Lösungsmittel oder ohne Lösungsmittel; oder

c) ein Isonicotinsäureazolid der Formel V

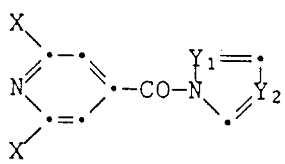

(V)

mit einem Amin der Formel III

$H_2N—Q_{1-6}$ (III)

in einem inerten Lösungsmittel unter Abscheidung eines Azols; wobei X und $Q_{1-6}$ die unter der Formel I angegebenen Bedeutungen besitzen, $Y_1$ und $Y_2$ unabhängig voneinander N oder CH darstellen wobei $Y_2$ vorzugsweise N ist, und Hal für Halogen, vorzugsweise für Chlor, steht.

Die Reaktionstemperaturen in den einzelnen Verfahrensvarianten betragen für (a) und (b) -50° bis 100°C, bevorzugt 0° bis 50°C, und für (c) 20° bis 180°C, bevorzugt 50°-130°C.

Als Basen zur Säurebindung in den Verfahrensvarianten (a) und (b) kommen organische und anorganische Basen in Betracht, z.B. tertiäre Amine wie Trialkylamine (Trimethylamin, Triethylamin, Tripropylamin usw.), Pyridin, Pyridinbasen (4-Dimethylaminopyridin, 4-Pyrrolidylaminopyridin usw.), und Alkoholate wie z.B. Kaliumtert.-Butylat, Oxide und Hydroxide, Carbonate und Hydrogencarbonate von Alkali- und Erdalkalimetallen sowie Alkaliacetate.

In den Verfahrensvarianten (a) und (b) werden als Reaktionsmedien in Anpassung an die jeweiligen Reaktionsbedingungen geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon; sowie Gemische solcher Lösungsmittel untereinander.

Umsetzungsreaktionen von Säurechloriden bzw. -anhydriden mit Aminen sind in Houben-Weyl Bd. 18, S. 655 beschrieben, während über Umamidierung in Angew. Chemie 1962, S. 413/412 berichtet wird.

Die Herstellung der Ausgangsstoffe wie Säurechloride und -anhydride ist dem Fachmann geläufig und bekannt aus der Fachliteratur (z.B. Houben-Weyl 5/3, S. 925).

Die Herstellung der als Ausgangsstoffe verwendeten Verbindungen der Formel V

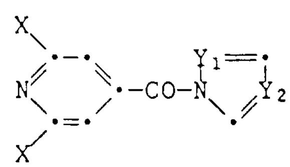

(V)

wird durch Umsetzung von Verbindungen der Formel II

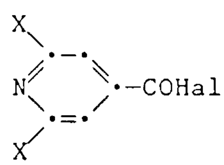

(II)

mit einem Azol der Formel VI

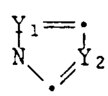

(VI)

in Gegenwart einer Base in einem inerten Lösungsmittel durchgeführt, wobei X die unter Formel I angegebenen Bedeutungen hat, Hal für Halogen, vorzugsweise für Chlor, steht und $Y_1$ und $Y_2$ unabhängig voneinander N oder CH darstellt.

Die Reaktionstemperaturen für die vorhergehend beschriebene Synthese betragen -50° bis 200°C, bevorzugt 10° bis 100°C.

Als Basen und Lösungsmittel kommen die für die Herstellung der Verbindungen der Formel I angegebenen in Frage.

Umsetzungen von Säurehalogeniden mit Azolen sind in Angew. Chemie 1962, S. 409-411 beschrieben.

Die Ausgangsstoffe der Formel V

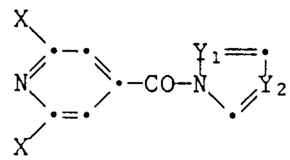

(V)

worin X in ortho/ortho'-Stellung gleiche Atome Fluor, Chlor, Brom oder Jod bedeutet und $Y_1$ und $Y_2$ unabhängig voneinander N oder CH darstellen, sind wertvolle Zwischenprodukte zur Herstellung der erfindungsgemässen Verbindungen der Formel I. Bei den Verbindungen der Formel V handelt es sich um neue Stoffe, die ebenfalls eine gegen die erwähnten Phytopathogene schützende Aktivität aufweisen. Sie stellen einen Bestandteil der vorliegenden Erfindung dar.

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der

physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation-und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungsbeispiele

Beispiel 1.1

a) Vorstufe: Herstellung von N-(2,6-Dichlorisonicotinoyl)-imidazol

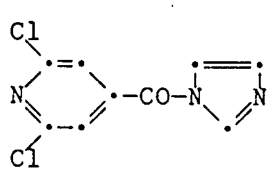

6,8 g (0,10 Mol) Imidazol und 15,3 ml (0,11 Mol) Triäthylamin werden in 100 ml Tetrahydrofuran gerührt. Anschliessend lässt man unter Kühlung bei 25°-30°C eine Lösung von 23,1 g (0,11 Mol) 2,6-Dichlorisonicotinsäurechlorid in 20 ml Tetrahydrofuran zutropfen, wobei sich Triäthylamin-Hydrochlorid abscheidet. Dieses wird abgesaugt und das Filtrat zu 26,4 g Rohprodukt eingeengt. Durch Umkristallisation aus Essigsäureethylester erhält man 20,1 g (83 % der Theorie) Reinsubstanz; Smp. 148°-151°C.

b) Endprodukt: Herstellung von N-[(2-Isopropoxy-5-chlor)-pyrimidin-4-yl]-2,6-dichlor-isonicotinsäureamid

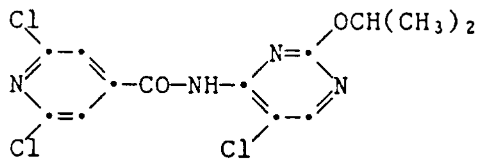

131,3 g (0,70 Mol) 2-Isopropoxy-4-amino-5-chlor-pyrimidin und 176,4 g (0,73 Mol) N-(2,6-Dichlorisonicotinoyl)-imidazol werden in 500 ml Toluol suspendiert. Man heizt auf Rückflusstemperatur und kocht 1 Stunde. Nach dem Erkalten wird das Reaktionsgemisch mit Essigsäureethylester und Wasser extrahiert, und danach die Essigsäureethylesterphase zur vollständigen Entfernung des gebildeten Imidazols zweimal mit Wasser gewaschen. Man trocknet die organische Phase über $Na_2SO_4$ und engt am Rotationsverdampfer zu 246 g Rohprodukt ein, das mittels säulenchromatographischer Auftrennung (Kieselgel; Laufmittel-Gemisch: n-Hexan/Essigsäureethylester 4:1) gereinigt wird. Man erhält 83 g (32,8 % d.Th.) Reinprodukt und 130 g Mischfraktionen, die schwer abtrennbares Edukt enthalten. Die erneute säulenchromatographische Auftrennung dieses Gemischs ergibt weitere 78 g (30,8 % d.Th.) Reinsubstanz. Die Umkristallisation der 83 g Produkt aus 400 ml Diethylether ergibt 57 g kristalline Verbindung vom Smp. 106°-108°C.

Beispiel 1.2: Herstellung von N-[(2-Dimethylamino-6-methoxy)-pyrimidin-4-yl]-2,6-dichlor-isonicotinsäureamid

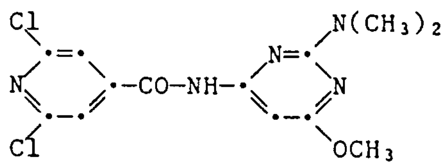

3,00 g (0,078 Mol) 2-Dimethylamino-4-amino-6-methoxypyrimidin werden in einem Gemisch aus 10 ml Pyridin und 10 ml Tetrahydrofuran gelöst. Unter Kühlung lässt man eine Lösung von 5,26 g (0,025 Mol) 2,6-Dichlorisonicotinsäurechlorid in 20 ml Tetrahydrofuran bei 20°C zutropfen, wobei sich Pyridin-Hydrochlorid abscheidet. Man rührt noch eine Stunde bei 20°C und extrahiert dann mit Essigsäureethylester und Wasser unter Zusatz von Essigsäure bei pH 6-7. Die organische Phase wird abgetrennt, über $Na_2SO_4$ getrocknet und zum Rohprodukt eingeengt, das dann über Kieselgel mit einem Gemisch Hexan/Essigsäureethylester 4:1 chromatographiert wird. Einengung der Reinfraktionen ergibt 5,21 g Substanz, die in 30 ml Acetonitril gekocht wird. Man saugt bei 20°C ab, trocknet bei 60°C im Vakuum und erhält 3,51 g (57,6 % d.Th.) Reinprodukt; Smp. 200°-202°C.

Beispiel 1.3:

a) Vorstufe: Herstellung von N-(2,6-Dichlorisonicotinoyl)-triazol

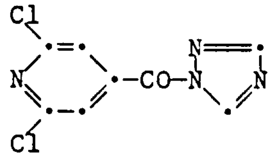

6,90 g (0,10 Mol) Triazol und 12,12 g (0,12 Mol) Triethylamin werden in 150 ml Tetrahydrofuran gelöst, mit Molekularsieb (0,4 nm, Merck) versetzt und zwei Tage bei 20°C stehen gelassen. Danach lässt man eine Lösung von 23,15 g (0,11 Mol) 2,6-Dichlorisonicotinsäurechlorid in 30 ml mit Molekularsieb getrocknetem Tetrahydrofuran langsam zutropfen. Die Reaktion verläuft exotherm; man hält die Mischung durch

Kühlung auf 15°-25°C. Das abgeschiedene Triäthylamino-Hydrochlorid wird abgesaugt und das Filtrat am Rotationsverdampfer zu 25,2 g Rohprodukt eingeengt. Die Umkristallisation aus einem Gemisch von 50 ml wasserfreiem Toluol und 30 ml wasserfreiem Cyclohexan ergibt 12,3 g (50,9 % d.Th.) N-(2,6-Dichlorisonicotinoyl)-triazol. Durch Einengen der Mutterlauge lassen sich weitere 6,5 g (27 % d.Th.) Substanz isolieren. Smp.: 110°-113°C.

b) Endprodukt: Herstellung von N-[(2-n-Propyl-6-chlor)-pyrimidin-4-yl]-2,6-dichlor-isonicotinsäureamid

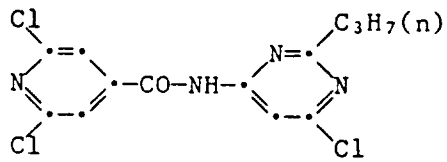

2,23 g (0,013 Mol) 2-n-Propyl-4-amino-6-chlorpyrimidin und 3,40 g (0,014 Mol) N-(2,6-Dichlorisonicotinoyl)-triazol werden in 15 ml mit Molekularsieb getrocknetes Toluol eingewogen und unter Ausschluss von Luftfeuchtigkeit 1,5 Stunden am Rückfluss gekocht. Es entsteht zu Beginn eine klare Lösung, die später trübe wird. Nach dem Erkalten wird mit Wasser und Essigsäureethylester im Scheidetrichter extrahiert um das gebildete Produkt abzutrennen. Nach dem Einengen der organischen Phase wird das Rohprodukt über eine Kieselgel-Säule mit einem Gemisch aus Hexan/Essigsäueethyleter 3:1 chromatographiert. Einengung der Reinfraktionen und Umkristallisation des Rückstands aus einem Gemisch von 3 ml Essigsäureethylester und 10 ml Hexan ergibt 3,34 g (74,3 % d.Th.) Produkt. Aus der Mutterlauge erhält man durch weitere Umkristallisation 0,90 g (20,0 % d.Th.) einer zweiten Fraktion. Beide Substanzen haben einen Schmelzpunkt von 134°-135°C.

Gemäss den beschriebenen Herstellungsweisen werden die nachfolgend aufgeführten Verbindungen erhalten.

Tabelle 1

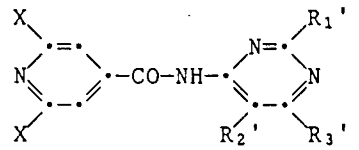

| Nr. | X | $R_1'$ | $R_2'$ | $R_3'$ | physik. Daten |
|---|---|---|---|---|---|
| 1.1 | Cl | $OCH(CH_3)_2$ | Cl | H | Smp. 106-108°C |
| 1.2 | Cl | $N(CH_3)_2$ | H | $OCH_3$ | Smp. 200-202°C |
| 1.3 | Cl | $CH_2CH_2CH_3$ | H | Cl | Smp. 134-135°C |
| 1.4 | Cl | $OCH_3$ | Cl | H | Smp. 148°C |
| 1.5 | Cl | H | $CH_3$ | Cl | Smp. 176-178°C |
| 1.6 | Cl | H | $C_2H_5$ | Cl | Smp. 145-147°C |
| 1.7 | Cl | H | $CH_2CH_2CH_3$ | Cl | Smp. 149-151°C |
| 1.8 | Cl | H | H | Cl | Smp. 171-172°C |
| 1.9 | Cl | H | H | $SCH_3$ | Smp. 160-165°C |
| 1.10 | Cl | H | H | $OCH_3$ | |
| 1.11 | Cl | H | H | $N(CH_3)_2$ | |
| 1.12 | Cl | H | H | $OCH(CH_3)_2$ | |
| 1.13 | Cl | H | H | $SC(CH_3)_3$ | |
| 1.14 | Cl | H | H | $OCH_2CH=CH_2$ | |
| 1.15 | Cl | H | H | $OCH_2C\equiv CH$ | |
| 1.16 | Br | $OCH_3$ | Cl | H | |
| 1.17 | Br | $OCH(CH_3)_2$ | Cl | H | |
| 1.18 | Cl | $CH_3$ | H | $CH_3$ | Smp. 142-144°C |
| 1.19 | Br | $CH_3$ | H | $CH_3$ | |
| 1.20 | Cl | $CH_3$ | $CH_3$ | Cl | Smp. 154-155°C |
| 1.21 | Cl | $CH_3$ | $CH_3$ | $OC_2H_5$ | |
| 1.22 | Cl | $CH_3$ | $CH_3$ | $SCH_2CH_2CH_3$ | |
| 1.23 | Cl | $CH_3$ | H | Cl | |
| 1.24 | Cl | $CH_3$ | H | $OCH(CH_3)C_2H_5$ | |
| 1.25 | Cl | $CH_3$ | H | $SC_2H_5$ | |
| 1.26 | Cl | $CH_3$ | H | $N(C_2H_5)_2$ | |
| 1.27 | Cl | $C_2H_5$ | $CH_3$ | Cl | Smp. 168-169°C |
| 1.28 | Br | $C_2H_5$ | $CH_3$ | Cl | |
| 1.29 | Cl | $C_2H_5$ | $CH_3$ | $OCH_3$ | |
| 1.30 | Cl | $C_2H_5$ | $CH_3$ | $OC_2H_5$ | |
| 1.31 | Cl | $C_2H_5$ | $CH_3$ | $OCH_2CH=CH_2$ | |
| 1.32 | Cl | $C_2H_5$ | $CH_3$ | F | |
| 1.33 | Cl | $C_2H_5$ | $CH_3$ | $SCH_3$ | |
| 1.34 | Cl | $C_2H_5$ | $CH_3$ | $N(CH_3)_2$ | |
| 1.35 | Cl | $C_2H_5$ | $C_2H_5$ | Cl | Smp. 115-116°C |
| 1.36 | Br | $C_2H_5$ | $C_2H_5$ | Cl | |
| 1.37 | Cl | $C_2H_5$ | $C_2H_5$ | $OCH_3$ | |
| 1.38 | Cl | $C_2H_5$ | $C_2H_5$ | $OCH(CH_3)_2$ | |
| 1.39 | Cl | $C_2H_5$ | $C_2H_5$ | $SCH(CH_3)_2$ | |
| 1.40 | Cl | $C_2H_5$ | $C_2H_5$ | $OCH_2CF_3$ | |
| 1.41 | Cl | $CH_2CH_2CH_3$ | $CH_3$ | Cl | Smp. 125-126°C |
| 1.42 | Cl | $CH(CH_3)_2$ | $CH_3$ | Cl | |
| 1.43 | Cl | $C(CH_3)_3$ | $CH_3$ | Cl | |

| Nr. | X | R1' | R2' | R3' | physik. Daten |
|---|---|---|---|---|---|
| 1.44 | Cl | $CH_3$ | $C_2H_5$ | Cl | |
| 1.45 | Cl | $CH_2CH_2CH_3$ | $C_2H_5$ | Cl | |
| 1.46 | Cl | $CH(CH_3)_2$ | $C_2H_5$ | Cl | |
| 1.47 | Cl | $(CH_2)_5CH_3$ | $C_2H_5$ | Cl | |
| 1.48 | Cl | $C(CH_3)_3$ | $C_2H_5$ | Cl | |
| 1.49 | Cl | $CH_3$ | $CH_2CH_2CH_3$ | Cl | |
| 1.50 | Cl | $CH_3$ | $CH_2CH_2CH_3$ | $OCH_3$ | |
| 1.51 | Cl | $CH_3$ | $C_2H_5$ | $SCH_3$ | |
| 1.52 | Cl | $N(CH_3)_2$ | $CH_3$ | Cl | |
| 1.53 | Cl | $N(CH_3)_2$ | H | Cl | Smp. 195-197°C |
| 1.54 | Cl | $N(CH_3)_2$ | $CH_3$ | $SCH_3$ | |
| 1.55 | Cl | $C_2H_5$ | H | Cl | Smp. 132-134°C |
| 1.56 | Cl | $CH(CH_3)_2$ | H | Cl | Smp. 137-138°C |
| 1.57 | Cl | $CH(CH_3)_2$ | H | $CH_3$ | Smp. 117-119°C |
| 1.58 | Cl | $CH(CH_3)_2$ | H | $OC_2H_5$ | Smp. 119-120°C |
| 1.59 | Cl | $CH(CH_3)_2$ | H | $OCH_2CH(CH_3)_2$ | Smp. 110-111°C |
| 1.60 | Cl | $CH(CH_3)_2$ | H | $SCH(CH_3)_2$ | Smp. 144-145°C |
| 1.61 | Br | $CH(CH_3)_2$ | H | $SCH_3$ | |
| 1.62 | Br | $CH(CH_3)_2$ | H | $OC_2H_5$ | |
| 1.63 | Cl | $CH(CH_3)_2$ | H | $SC_2H_5$ | Smp. 169-171°C |
| 1.64 | Cl | $CH(CH_3)_2$ | H | $OCH(CH_3)C_2H_5$ | $n_D^{50}$ 1,552 |
| 1.65 | Cl | $CH(CH_3)_2$ | H | $N(C_2H_5)_2$ | |
| 1.66 | Cl | $CH(CH_3)_2$ | H | 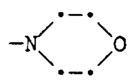 | |
| 1.67 | Cl | $CH(CH_3)_2$ | H | 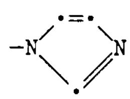 | Smp. 229-231°C |
| 1.68 | Cl | H | H | 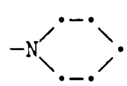 | |
| 1.69 | Cl | H | H | $SC_2H_5$ | |
| 1.70 | Cl | H | H | 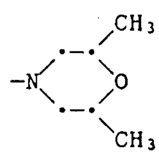 | |
| 1.71 | Cl | cyclo-$C_3H_5$ | H | F | Smp. 98-101°C |
| 1.72 | Br | cyclo-$C_3H_5$ | H | F | |
| 1.73 | Cl | cyclo-$C_3H_5$ | H | Cl | Smp. 150°C (Zers.) |
| 1.74 | Cl | cyclo-$C_3H_5$ | $CH_3$ | Cl | Smp. 156-158°C |
| 1.75 | Cl | cyclo-$C_3H_5$ | $C_2H_5$ | Cl | Smp. 143-144°C |
| 1.76 | Cl | $OCH(CH_3)_2$ | Br | H | |
| 1.77 | Cl | $OCH(CH_3)_2$ | F | H | |
| 1.78 | Cl | $OCH(CH_3)_2$ | J | H | |
| 1.79 | Cl | Cl | F | H | Smp. 172-174°C |
| 1.80 | Cl | Cl | Cl | H | Smp. 244-246°C |
| 1.81 | Cl | $C_6H_5$ | H | Cl | |
| 1.82 | Cl | $C_6H_5$ | H | $SCH(CH_3)C_2H_5$ | |
| 1.83 | Cl | $C_6H_5$ | H | $OCH_3$ | |

| Nr. | X | R₁' | R₂' | R₃' | physik. Daten |
|---|---|---|---|---|---|
| 1.84 | Cl | $C_6H_5$ | H | $SCH_2CH_2CH_3$ | |
| 1.85 | Cl | Cl | Cl | Cl | Smp. 184-186°C |
| 1.86 | Cl | H | H | H | |
| 1.87 | Cl | $OCH_3$ | Cl | $CH_3$ | |
| 1.88 | Cl | $OCH_3$ | Cl | $C_2H_5$ | |
| 1.89 | Cl | $OCH_3$ | F | H | Smp. 132-134°C |
| 1.90 | Cl | $C_6H_5$ | H | 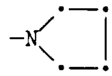 | |
| 1.91 | Cl | H | H | 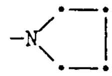 | |
| 1.92 | Cl | Cl | H | $CH_2CH_2CH_3$ | Smp. 93-94°C |
| 1.93 | Cl | Cl | H | $C_2H_5$ | |
| 1.94 | Cl | Cl | H | $CH_3$ | Smp. 200-204°C |
| 1.95 | Br | Cl | Cl | $CH_3$ | |
| 1.96 | Cl | $OC_2H_5$ | Cl | H | Smp. 140-141°C |
| 1.97 | Cl | $OC_2H_5$ | F | H | |
| 1.98 | Cl | $OCH(CH_3)_2$ | Cl | $CH_3$ | |
| 1.99 | Br | $OCH(CH_3)_2$ | Cl | $C_2H_5$ | |
| 1.100 | Cl | $OCH_2CH_2CH_3$ | Cl | H | Smp. 105-107°C |
| 1.101 | Cl | $SCH_3$ | Cl | H | Smp. 124-125°C |
| 1.102 | Cl | $SCH_3$ | Cl | $CH_3$ | |
| 1.103 | Cl | $OCH_2CF_3$ | Cl | H | Smp. 50-55°C |
| 1.104 | Cl | $OCH_2CH(CH_3)_2$ | Cl | H | |
| 1.105 | Cl | $SC_2H_5$ | Cl | H | |
| 1.106 | Cl | $OCH(CH_3)_2$ | Cl | $CH_2CH_2CH_3$ | |
| 1.107 | Cl | $OC(CH_3)_3$ | Cl | H | |
| 1.108 | Cl | $OCH(CH_3)C_2H_5$ | Cl | H | |
| 1.109 | Cl | $SCH_3$ | $CH_3$ | Cl | Smp. 182-187°C |
| 1.110 | Br | $SCH_3$ | $CH_3$ | $OC_2H_5$ | |
| 1.111 | Cl | $SCH_3$ | $CH_3$ | $OCH_3$ | |
| 1.112 | Cl | $SCH_3$ | H | Cl | Smp. 84-89°C |
| 1.113 | Cl | $SCH_3$ | H | $OCH_3$ | Smp. 227-229°C |
| 1.114 | Cl | $CF_3$ | H | Cl | Oel |
| 1.115 | Cl | H | $NO_2$ | $OCH_3$ | |
| 1.116 | Cl | H | $NO_2$ | $SCH_2CH=CH_2$ | |
| 1.117 | Cl | H | $NO_2$ | $SC(CH_3)_3$ | |
| 1.118 | Cl | $SCH(CH_3)_2$ | Cl | H | |
| 1.119 | Cl | $N(CH_3)_2$ | Cl | H | |
| 1.120 | Cl | $CH_2CH_2OCH_3$ | H | Cl | Smp. 176-178°C |
| 1.121 | Cl | $OCH_3$ | H | H | |
| 1.122 | Cl | $OCH(CH_3)_2$ | H | H | |
| 1.123 | Cl | $OCH_3$ | Br | H | |
| 1.124 | Cl | $OCH_3$ | $NO_2$ | H | |
| 1.125 | Cl | $OC_6H_5$ | Cl | H | |
| 1.126 | Cl | $SC_6H_5$ | Cl | H | |
| 1.127 | Cl | 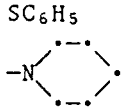 | Cl | H | |

| Nr. | X | $R_1$' | $R_2$' | $R_3$' | physik. Daten |
|---|---|---|---|---|---|
| 1.128 | Cl | 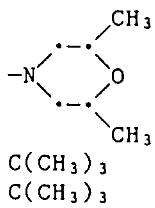 | Cl | H | |
| 1.129 | Cl | $C(CH_3)_3$ | H | Cl | Smp. 131-133°C |
| 1.130 | Cl | $C(CH_3)_3$ | H | $SC_6H_5$ | |
| 1.131 | Cl | H | H | $SC_6H_5$ | |
| 1.132 | Cl | H | H | $OC_6H_5$ | |
| 1.133 | Cl | $OCH_2CH=CH_2$ | Cl | H | |
| 1.134 | Cl | $OCH_2C{\equiv}CH$ | Cl | H | |
| 1.135 | Cl | $SCH_2CH=CH_2$ | Cl | H | |
| 1.136 | Cl | Cl | H | $C_6H_5$ | |
| 1.137 | Cl | $OCH_3$ | H | $C_6H_5$ | |
| 1.138 | Cl | $OC(CH_3)_3$ | H | $C_6H_5$ | |
| 1.139 | Br | $C_2H_5$ | $CH_3$ | Cl | Smp. 145-146°C |
| 1.140 | Br | $OCH(CH_3)_2$ | Cl | H | |
| 1.141 | F | $OCH(CH_3)_2$ | Cl | H | |
| 1.142 | J | $OCH(CH_3)_2$ | Cl | H | |
| 1.143 | Cl | $OCH_3$ | J | H | |
| 1.144 | Cl | $OCH_3$ | Br | H | |
| 1.145 | Cl | $CH_2CH_2CH_3$ | H | 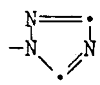 | Smp. 234-235°C |
| 1.146 | Br | $OCH_3$ | Cl | H | |
| 1.147 | F | $OCH_3$ | Cl | H | |
| 1.148 | J | $OCH_3$ | Cl | H | |
| 1.149 | Cl | $CH_3$ | H | $CF_3$ | Smp. 149-150°C |
| 1.150 | Cl | $CH_3$ | $COOC_2H_5$ | $CH_3$ | |
| 1.151 | Cl | $CH_2C_6H_5$ | $COOCH_3$ | H | |
| 1.152 | Cl | $CH_2C_6H_5$ | H | CN | |
| 1.153 | Cl | $CH_3$ | CN | H | |
| 1.154 | Cl | Cl | CN | $SCH_3$ | |
| 1.155 | Cl | $CH_3$ | J | $CH_3$ | |
| 1.156 | Cl | $OCH_3$ | H | $OCH_3$ | Smp. 162-164°C |
| 1.157 | Cl | $N(CH_3)_2$ | $NO_2$ | H | |
| 1.158 | Cl | H | $NO_2$ | $N(CH_3)_2$ | |
| 1.159 | Cl | Cl | $CH_2C_6H_5$ | H | |
| 1.160 | Cl | Cl | $CH_2C_6H_5$ | Cl | |
| 1.161 | Cl | H | Br | H | |
| 1.162 | Cl | $C_2H_5$ | H | Br | |
| 1.163 | Cl | $CH_3$ | $CH_2Br$ | H | |
| 1.164 | Cl | Cl | $CH_2Cl$ | H | |
| 1.165 | Cl | $CH_3$ | $(CH_2)_5CH_3$ | Cl | |
| 1.166 | Cl | $CH_3$ | $(CH_2)_3CH_3$ | Cl | Smp. 135-137°C |
| 1.167 | Cl | cyclo-$C_3H_5$ | $CH_3$ | Cl | Smp. 198-199°C |
| 1.168 | Cl | cyclo-$C_3H_5$ | $C_2H_5$ | Cl | Smp. 143-144°C |
| 1.169 | Cl | H | $CH_2CH=CH_2$ | Cl | Smp. 117-118°C |
| 1.170 | Cl | $CH_3$ | $C_2H_5$ | Cl | Smp. 133-135°C |
| 1.171 | Cl | $CH_3$ | $CH_2CH=CH_2$ | Cl | Smp. 123-124°C |
| 1.172 | Cl | $CH_3$ | $C_2H_5$ | H | Smp. 131-133°C |

| Nr. | X | $R_1'$ | $R_2'$ | $R_3'$ | physik. Daten |
|---|---|---|---|---|---|
| 1.173 | Cl | $CH_3$ | $CH_2-C_6H_5$ | Cl | Smp. 138-139°C |
| 1.174 | Cl | H | $CH_2-C_6H_5$ | Cl | Smp. 128-129°C |
| 1.175 | Cl | $CH_3$ | $CH_3$ | Cl | Smp. 154-155°C |
| 1.176 | Cl | $C_2H_5$ | $CH_3$ | F | Smp. 140-142°C |
| 1.177 | Cl | $CH_3$ | $C_3H_7(n)$ | Cl | Smp. 185-187°C |
| 1.178 | Cl | H | $(CH_2)_3CH_3$ | Cl | Smp. 103-105°C |
| 1.179 | Cl | $CH(CH_3)_2$ | $CH_3$ | Cl | Smp. 131-133°C |
| 1.180 | Cl | H | $C_2H_5$ | F | Smp. 136-138°C |
| 1.181 | Cl | $C_2H_5$ | $C_2H_5$ | F | Smp. 102-104°C |
| 1.182 | Cl | $C_2H_5$ | $C_2H_5$ | H | Smp. 98-99°C |
| 1.183 | Br | H | $C_2H_5$ | Cl | Smp. 164-167°C |

Tabelle 2

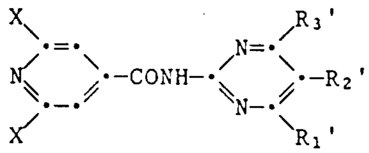

| Nr. | X | R1' | R2' | R3' | physik. Daten |
|---|---|---|---|---|---|
| 2.1 | Cl | H | H | H | Smp. 214-216°C |
| 2.2 | F | H | H | H | |
| 2.3 | Cl | Cl | H | Cl | Smp. 198-199°C |
| 2.4 | Cl | Cl | H | $OCH_3$ | |
| 2.5 | Cl | Cl | H | $OC_2H_5$ | |
| 2.6 | Cl | Cl | H | $OCH_2CH{=}CH_2$ | Smp. 160°C |
| 2.7 | Cl | Cl | H | $OCH_2C{\equiv}CH$ | Smp. 148-149°C |
| 2.8 | Cl | Cl | H | $SCH_3$ | |
| 2.9 | Cl | Cl | H | $SC_6H_5$ | |
| 2.10 | Cl | H | H | $OC_3H_7(n)$ | |
| 2.11 | Cl | H | H | $SC_2H_5$ | |
| 2.12 | Cl | H | Cl | Cl | |
| 2.13 | Cl | H | Cl | $OC_2H_5$ | Smp. 162-165°C |
| 2.14 | F | H | Cl | $OC_2H_5$ | |
| 2.15 | Cl | H | Br | $OCH_3$ | |
| 2.16 | Cl | H | Br | Cl | |
| 2.17 | Cl | $OCH_3$ | $NO_2$ | $OCH_3$ | |
| 2.18 | Cl | $CH_3$ | H | $CH_3$ | Smp. 165-168°C |
| 2.19 | F | $CH_3$ | H | $CH_3$ | |
| 2.20 | Br | $CH_3$ | H | $CH_3$ | |
| 2.21 | Cl | $CH_3$ | H | Cl | Smp. 178-182°C |
| 2.22 | Cl | $CH_3$ | H | $N(CH_3)_2$ | |
| 2.23 | Cl | $CH_3$ | H | $SCH(CH_3)_2$ | |
| 2.24 | Cl | $CH_3$ | H | $SCH_2C_6H_5$ | |
| 2.25 | Cl | $CH_3$ | H | $OC_6H_5$ | |
| 2.26 | Cl | $CH_3$ | H | 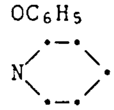 | |
| 2.27 | Cl | $CH_3$ | H | $OCHF_2$ | Smp. 160-162°C |
| 2.28 | F | $CH_3$ | H | $OCHF_2$ | |
| 2.29 | Cl | $CH_3$ | H | $C_2H_5$ | |
| 2.30 | Cl | $CH_3$ | H | $C_3H_7(n)$ | |
| 2.31 | Cl | $CH_3$ | H | cyclo-$C_3H_5$ | Smp. 145-147°C |
| 2.32 | Cl | $C_2H_5$ | H | Cl | |
| 2.33 | Br | $CH(CH_3)_2$ | H | Cl | |
| 2.34 | J | $CH(CH_3)_2$ | H | Cl | |
| 2.35 | F | $CH(CH_3)_2$ | H | Cl | |
| 2.36 | Cl | $CH(CH_3)_2$ | H | Cl | Smp. 174-176°C |
| 2.37 | Cl | Cl | Cl | Cl | |
| 2.38 | Cl | Cl | Br | Cl | |
| 2.39 | Cl | $CH_2CH_2CH_3$ | H | Cl | Smp. 135-137°C |
| 2.40 | Cl | $CH_2CH_2CH_3$ | H | $OCH_3$ | |

| Nr. | X | $R_1'$ | $R_2'$ | $R_3'$ | physik. Daten |
|---|---|---|---|---|---|
| 2.41 | Cl | $CH_2CH_2CH_3$ | H | 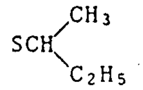 | |
| 2.42 | Cl | $CH_2CH_2CH_3$ | H | $OCH_2CF_3$ | |
| 2.43 | Cl | Cl | H | $OC_6H_5$ | |
| 2.44 | Cl | $CH(CH_3)_2$ | H | $OCH_3$ | |
| 2.45 | Br | $CH(CH_3)_2$ | H | $OCH(CH_3)_2$ | |
| 2.46 | Cl | $CH(CH_3)_2$ | H | $OCH(CH_3)_2$ | |
| 2.47 | Cl | $CH(CH_3)_2$ | H | $SC_2H_5$ | |
| 2.48 | Cl | $CH(CH_3)_2$ | H | 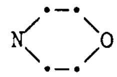 | |
| 2.49 | Cl | $CH_2COOC_2H_5$ | H | $OC_2H_5$ | Smp. 111-113°C |
| 2.50 | Cl | $OCH_3$ | H | $OCH_3$ | |
| 2.51 | Cl | $OCH(CH_3)_2$ | H | $OCH(CH_3)_2$ | |
| 2.52 | Cl | $SC_2H_5$ | H | $SC_2H_5$ | |
| 2.53 | Cl | $SC_6H_5$ | H | $SC_6H_5$ | |
| 2.54 | Cl | $OC_2H_5$ | H | $SC_3H_7(n)$ | |
| 2.55 | Cl | $N(C_2H_5)_2$ | H | $SCH_2C_6H_5$ | |
| 2.56 | Cl | $CH_2Cl$ | H | Cl | Smp. 135-138°C |
| 2.57 | Cl | $CH_3$ | $CH_3$ | Cl | |
| 2.58 | Cl | $CH_3$ | $C_2H_5$ | Cl | |
| 2.59 | Cl | $CH_3$ | $C_3H_7$ | Cl | |
| 2.60 | Cl | $CH_3$ | $CH_3$ | $OC_3H_7(n)$ | |
| 2.61 | Cl | $CH_3$ | $C_2H_5$ | $SC_2H_5$ | |
| 2.62 | Cl | $CH_3$ | $CH_3$ | $CH_3$ | |
| 2.63 | Cl | $CH_3$ | $C_2H_5$ | $CH_3$ | |
| 2.64 | Cl | $CH_3$ | $C_3H_7(n)$ | $CH_3$ | |
| 2.65 | Cl | $CH_3$ | $CH(CH_3)_2$ | $CH_3$ | |
| 2.66 | Cl | $CH_3$ | Cl | $CH_3$ | |
| 2.67 | Cl | $CH_3$ | $OCH_3$ | $CH_3$ | |
| 2.68 | Cl | $CH_3$ | $SCH_3$ | $CH_3$ | |
| 2.69 | Cl | $CH_3$ | $SCH(CH_3)_2$ | $CH_3$ | |
| 2.70 | Cl | $CH_3$ | F | $CH_3$ | |
| 2.71 | Cl | $CH_3$ | $OC_6H_5$ | $CH_3$ | |
| 2.72 | Cl | $CH_3$ | $N(C_2H_5)_2$ | $CH_3$ | |
| 2.73 | Cl | $CH_3$ | 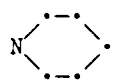 | $CH_3$ | |
| 2.74 | Cl | $CH_3$ | 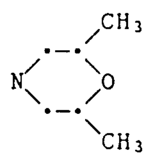 | $CH_3$ | |
| 2.75 | Cl | $CH_3$ | $CH_3$ | H | |
| 2.76 | Cl | $CH_3$ | $C_2H_5$ | H | |
| 2.77 | Cl | $C_2H_5$ | $CH_3$ | H | |
| 2.78 | Cl | $CH(CH_3)_2$ | H | H | |
| 2.79 | Cl | $C_3H_7(n)$ | H | H | |
| 2.80 | Cl | $CH_3$ | H | H | |
| 2.81 | Cl | Cl | $CH_3$ | Cl | |

| Nr. | X | $R_1'$ | $R_2'$ | $R_3'$ | physik. Daten |
|---|---|---|---|---|---|
| 2.82 | Cl | F | $CH_3$ | F | |
| 2.83 | Cl | Cl | Cl | $OCH_3$ | |
| 2.84 | Cl | Cl | Cl | $SC_2H_5$ | |
| 2.85 | Cl | $CH_3$ | H | F | |
| 2.86 | Cl | $CH_2CH_2CH_3$ | H | F | |
| 2.87 | Cl | $CH(CH_3)_2$ | H | F | |
| 2.88 | Cl | $CH_3$ | H | $OCH_2CF_3$ | |
| 2.89 | Cl | Cl | $SCH_3$ | Cl | |
| 2.90 | Cl | $CH(OC_2H_5)_2$ | H | Cyclo-$C_3H_5$ | Smp. 100-101°C |
| 2.91 | Cl | $C_2H_5$ | H | $C_2H_5$ | Smp. 117-119°C |
| 2.92 | Cl | $CH_2OCH_3$ | H | Cyclo-$C_3H_5$ | Smp. 115-118°C |
| 2.93 | Cl | Cyclo-$C_3H_5$ | H | Cyclo-$C_3H_5$ | Smp. 138-140°C |
| 2.94 | Cl | $C_2H_5$ | H | $CH_2OCH_3$ | Smp. 89-91°C |
| 2.95 | Cl | $CH_3$ | H | 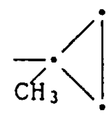 | Smp. 159-161°C |

Tabelle 3

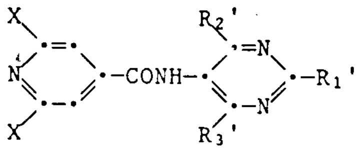

| Nr. | X | $R_1$' | $R_2$' | $R_3$' | physik. Daten |
|---|---|---|---|---|---|
| 3.1 | Cl | H | H | H | Smp. 161-162°C |
| 3.2 | Cl | H | Cl | Cl | Smp. 224-225°C |
| 3.3 | Cl | H | Cl | $OCH_3$ | |
| 3.4 | Cl | H | Cl | $OC_2H_5$ | |
| 3.5 | Cl | H | Cl | $OCH_2CF_3$ | Smp. 197-198°C |
| 3.6 | Cl | H | Cl | $OCH_2CH{=}CH_2$ | |
| 3.7 | Cl | H | Cl | $SCH_3$ | Smp. 201-203°C |
| 3.8 | Cl | H | Cl | $SC_6H_5$ | |
| 3.9 | F | H | Cl | $OCH_3$ | |
| 3.10 | Br | H | Cl | $OCH_3$ | |
| 3.11 | Cl | H | $OCH_3$ | $OCH_3$ | |
| 3.12 | Cl | H | $SC_3H_7(n)$ | $SC_3H_7(n)$ | |
| 3.13 | Cl | H | H | Cl | |
| 3.14 | Cl | H | H | $OCH_3$ | |
| 3.15 | Cl | H | $SCH_3$ | $SCH_3$ | |
| 3.16 | Cl | H | $OCH_2CF_3$ | $OCH_2CF_3$ | |
| 3.17 | Cl | $CH_3$ | Cl | Cl | Smp. 230-231°C |
| 3.18 | Cl | $CH_3$ | Cl | $OCH(CH_3)_2$ | |
| 3.19 | Cl | $CH_3$ | Cl | $OCH_2CF_3$ | Smp. 197-198°C |
| 3.20 | Br | $CH_3$ | Cl | $OCH_2CF_3$ | |
| 3.21 | Cl | $CH_3$ | H | $OC_2H_5$ | Smp. 215-216°C |
| 3.22 | Cl | H | Cl | $OCH_2C_6H_5$ | |
| 3.23 | Cl | H | Cl | $OC_6H_5$ | |
| 3.24 | Cl | H | Cl | $N(C_3H_7)_2$ | |
| 3.25 | Cl | H | Cl | H | |
| 3.26 | Cl | $CH_3$ | Cl | $OC_2H_5$ | Smp. 211-212°C |
| 3.27 | Cl | $CH_3$ | 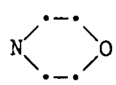 | $OC_2H_5$ | |
| 3.28 | Cl | $C_2H_5$ | Cl | Cl | |
| 3.29 | Cl | $C_2H_5$ | H | H | |
| 3.30 | Cl | $C_2H_5$ | Cl | 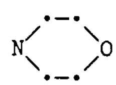 | |
| 3.31 | Cl | $C_2H_5$ | Cl | $SCH(CH_3)_2$ | |
| 3.32 | Cl | $C_2H_5$ | Cl | $N(C_4H_9\text{-}n)_2$ | |
| 3.33 | Cl | $C_2H_5$ | Cl | $OCH_2CF_3$ | Smp. 200-202°C |
| 3.34 | Cl | $CH(CH_3)_2$ | H | H | Smp. 176-177°C |
| 3.35 | Cl | $CH(CH_3)_2$ | Cl | Cl | Smp. 212-214°C |
| 3.36 | Cl | $CH(CH_3)_2$ | Cl | $OCH_3$ | |
| 3.37 | Cl | $CH(CH_3)_2$ | Cl | $OC_2H_5$ | |
| 3.38 | Cl | $CH(CH_3)_2$ | Cl | $OCH_2CF_3$ | Smp. 139-141°C |
| 3.39 | Cl | $CH(CH_3)_2$ | Cl | $OC(CH_3)_3$ | |
| 3.40 | Cl | $CH(CH_3)_2$ | Cl | $SCH_3$ | Smp. 208-209°C |

| Nr. | X | $R_1$' | $R_2$' | $R_3$' | physik. Daten |
|---|---|---|---|---|---|
| 3.41 | Cl | $CH(CH_3)_2$ | Cl | H | |
| 3.42 | Cl | $CH(CH_3)_2$ | Cl | $SCH(CH_3)C_2H_5$ | |
| 3.43 | Cl | $C(CH_3)_3$ | Cl | Cl | Smp. 203-205°C |
| 3.44 | Cl | $C(CH_3)_3$ | Cl | H | |
| 3.45 | Cl | $C(CH_3)_3$ | H | H | |
| 3.46 | Cl | $C(CH_3)_3$ | Cl | $OCH_3$ | Smp. 188-191°C |
| 3.47 | Cl | $C(CH_3)_3$ | Cl | $SCH_3$ | |
| 3.48 | Cl | $CH_3$ | Cl | H | |
| 3.49 | Cl | cyclo-$C_3H_5$ | Cl | Cl | Smp. 228-230°C |
| 3.50 | Cl | cyclo-$C_3C_5$ | H | H | |
| 3.51 | Cl | cyclo-$C_3H_5$ | Cl | $N(CH_3)_2$ | |
| 3.52 | Cl | $N(CH_3)_2$ | Cl | Cl | |
| 3.53 | Cl | $N(CH_3)_2$ | F | F | |
| 3.54 | Cl | $CH_3$ | F | F | |
| 3.55 | Cl | $C_2H_5$ | F | F | |
| 3.56 | Cl | $CCl_3$ | F | F | |
| 3.57 | Cl | $CF_3$ | Cl | Cl | |
| 3.58 | Cl | $CCl_3$ | Cl | Cl | |
| 3.59 | Cl | $N(CH_3)_2$ | Cl | $OCH_3$ | |
| 3.60 | Cl | $N(CH_3)_2$ | Cl | $SCH_3$ | |
| 3.61 | Cl | $N(CH_3)_2$ | Cl | $OCH(CH_3)C_3H_7$ | |
| 3.62 | Cl | $N(CH_3)_2$ | Cl | $OCH_2C{\equiv}CH$ | |
| 3.63 | Cl | $N(CH_3)_2$ | Cl | $CH_3$ | |
| 3.64 | Cl | $N(CH_3)_2$ | $OCH_3$ | $CH_3$ | |
| 3.65 | Cl | $N(CH_3)_2$ | $SCH_3$ | $CH_3$ | |
| 3.66 | Cl | $N(CH_3)_2$ | F | $CH_3$ | |
| 3.67 | Cl | Cl | Cl | H | Smp. 151-152°C |
| 3.68 | Cl | Cl | $OCH_3$ | H | Smp. 221-224°C |
| 3.69 | Cl | Cl | $OC_2H_5$ | H | Smp. 184-185°C |
| 3.70 | Cl | Cl | $SCH_3$ | H | |
| 3.71 | Cl | Cl | $OC_3H_7$-n | H | Smp. 135-136°C |
| 3.72 | Cl | H | $OC_2H_5$ | H | |
| 3.73 | Cl | Cl | $SC(CH_3)_3$ | H | |
| 3.74 | Cl | Cl | $OCH_2CF_3$ | H | Smp. 128-130°C |
| 3.75 | Cl | $SC_6H_5$ | $OCH_2CF_3$ | H | |
| 3.76 | Cl | $SCH_3$ | $OC_2H_5$ | H | |
| 3.77 | Cl | Cl | $OCH_2CH_2OCH_3$ | H | Smp. 138-140°C |
| 3.78 | Cl | F | F | H | |
| 3.79 | Cl | F | $OCH_3$ | H | |
| 3.80 | Cl | F | $SC_2H_5$ | H | |
| 3.81 | Cl | Cl | $OCH_2C{\equiv}CH$ | H | |
| 3.82 | Cl | $SCH_3$ | Cl | Cl | Smp. 232-234°C |
| 3.83 | Cl | $SCH_3$ | Cl | $OCH_3$ | |
| 3.84 | Cl | $SCH_3$ | Cl | $SCH_3$ | |
| 3.85 | Cl | $SCH_3$ | Cl | 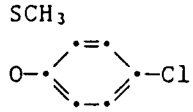 | |
| 3.86 | Cl | $SCH_3$ | Cl | 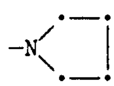 | |

| Nr. | X | $R_1$' | $R_2$' | $R_3$' | physik. Daten |
|---|---|---|---|---|---|
| 3.87 | Cl | $SCH_3$ | Cl | $OCH(CH_3)_2$ | |
| 3.88 | Cl | $SCH_3$ | Cl | $OCH_2CCl_2CF_3$ | |
| 3.89 | Cl | $SCH_3$ | Cl | $N(CH_3)C_3H_7$ | |
| 3.90 | Cl | $SCH_3$ | $SC_2H_5$ | $SC_2H_5$ | |
| 3.91 | Cl | $SCH_3$ | $SC_2H_5$ | $OCH_3$ | |
| 3.92 | Cl | $SCH_3$ | $SC_2H_5$ | $OCH(CH_3)$ | |
| 3.93 | Cl | Cl | $CH_3$ | Cl | |
| 3.94 | Cl | Cl | $CH_3$ | $OCH_3$ | Smp. 216-218°C |
| 3.95 | Cl | Cl | $CH_3$ | $SCH_3$ | |
| 3.96 | Cl | Cl | $CH_3$ | $N(CH_3)_2$ | |
| 3.97 | Cl | $SC_2H_5$ | $CH_3$ | $SC_2H_5$ | |
| 3.98 | Cl | $SC_6H_5$ | $CH_3$ | $SC_6H_5$ | |
| 3.99 | Cl | F | $CH_3$ | F | |

Tabelle 4

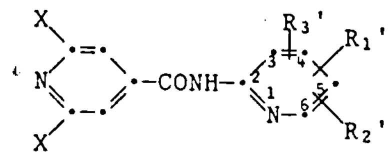

| Nr. | X | $R_1$' | $R_2$' | $R_3$' | physik. Daten |
|---|---|---|---|---|---|
| 4.1 | Cl | H | H | H | Smp. 170-173°C |
| 4.2 | Cl | 6-$CH_3$ | H | H | Smp. 89-92°C |
| 4.3 | Cl | 4-$CH_3$ | H | H | Smp. 170-172°C |
| 4.4 | Cl | 5-Cl | H | H | Smp. 166-168°C |
| 4.5 | Br | H | H | H | |
| 4.6 | Br | 5-Cl | H | H | |
| 4.7 | Br | 4-$CH_3$ | H | H | |
| 4.8 | Br | 6-$CH_3$ | H | H | |
| 4.9 | F | H | H | H | |
| 4.10 | Cl | 5-$CH_3$ | H | H | |
| 4.11 | Cl | 5-Br | H | H | |
| 4.12 | Cl | 3-Br | 5-Br | H | |
| 4.13 | Cl | 3-Cl | 5-Cl | H | |
| 4.14 | Cl | 4-$CH_3$ | 6-$CH_3$ | H | |
| 4.15 | Cl | 3-$NO_2$ | H | H | |
| 4.16 | Cl | 5-$NO_2$ | H | H | |
| 4.17 | Cl | 3-$C_4H_9$(tert.) | H | H | |
| 4.18 | Cl | 4-$C_2H_5$ | H | H | |
| 4.19 | Cl | 3-$CH_3$ | 4-$CH_3$ | H | |
| 4.20 | Cl | 3-$CH_3$ | 5-$CH_3$ | H | |
| 4.21 | Cl | 3-$C_2H_5$ | 6-$CH_3$ | H | |
| 4.22 | Cl | 6-Br | H | H | |
| 4.23 | Cl | 3-Cl | 5-$CF_3$ | H | Smp. 178-179°C |
| 4.24 | Cl | 3-$CH_3$ | 5-Br | H | |
| 4.25 | Cl | 4-$CH_3$ | 6-Br | H | |
| 4.26 | Cl | 4-Cl | 6-$CH_3$ | H | |
| 4.27 | Cl | 3-$NO_2$ | 6-$CH_3$ | 6Me | |
| 4.28 | Cl | 3-$NO_2$ | 4-Cl | H | |
| 4.29 | Cl | 3-$NO_2$ | 5-Cl | H | |
| 4.30 | Cl | 3-$OCH_3$ | H | H | |
| 4.31 | Cl | 3-$OC_2H_5$ | H | H | |
| 4.32 | Cl | 6-$OC_2H_5$ | H | H | |
| 4.33 | Br | 3-$NO_2$ | H | H | |
| 4.34 | Br | 5-$NO_2$ | H | H | |
| 4.35 | Br | 4-$CH_3$ | 6-$CH_3$ | H | |
| 4.36 | Cl | 6-$CH_3$ | H | H | |
| 4.37 | Br | 6-$CH_3$ | H | H | |
| 4.38 | Cl | 5-$CF_3$ | H | H | |
| 4.39 | Cl | 3-Br | 5-Cl | H | Smp. 163-164°C |
| 4.40 | Cl | 3-$COOC_2H_5$ | 5-Br | H | Smp. 219-220°C |

Tabelle 5

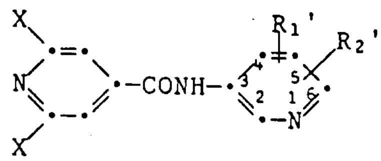

| Nr. | X | $R_1$' | $R_2$' | physik. Daten |
|---|---|---|---|---|
| 5.1 | Cl | H | H | Smp. 155-157°C |
| 5.2 | Cl | 2-Cl | H | |
| 5.3 | Cl | 2-Cl | 6-Cl | |
| 5.4 | Cl | 2-$OCH_3$ | 6-$OCH_3$ | |
| 5.5 | Cl | 2-$CH_3$ | H | |
| 5.6 | Cl | 4-$CH_3$ | H | |
| 5.7 | Cl | 5-$CH_3$ | H | |
| 5.8 | Cl | 6-$CH_3$ | H | |
| 5.9 | Cl | 2-F | H | |
| 5.10 | Cl | 5-Br | H | |
| 5.11 | Cl | 4-Cl | 6-Cl | |
| 5.12 | Cl | 4-$NO_2$ | H | |
| 5.13 | Br | H | H | |
| 5.14 | Br | 2-Cl | H | |
| 5.15 | F | H | H | |
| 5.16 | J | H | H | |

Tabelle 6

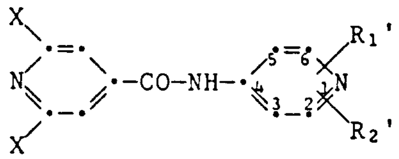

| Nr. | X | $R_1'$ | $R_2'$ | physik. Daten |
|---|---|---|---|---|
| 6.1 | Cl | H | H | Smp. 227-228°C |
| 6.2 | Cl | $2-CH_3$ | H | |
| 6.3 | Cl | $3-CH_3$ | H | |
| 6.4 | Cl | $3-C_3H_7$(iso) | H | |
| 6.5 | Cl | $2-C_4H_9$(tert.) | H | |
| 6.6 | Cl | 2-Cl | H | |
| 6.7 | Cl | 2-Br | H | |
| 6.8 | Cl | 2-J | H | |
| 6.9 | Cl | 3-Cl | H | |
| 6.10 | Cl | 3-Br | H | |
| 6.11 | Cl | 3-F | H | |
| 6.12 | Cl | $2-OCH_3$ | H | |
| 6.13 | Cl | $2-CH_3$ | 6-Cl | |
| 6.14 | Cl | $2-CH_3$ | $5-NO_2$ | |
| 6.15 | Cl | 2-Cl | $3-NO_2$ | |
| 6.16 | Cl | 2-Cl | $4-NO_2$ | |
| 6.17 | Cl | 3-Cl | $5-NO_2$ | |
| 6.18 | Br | H | H | |
| 6.19 | Br | $2-CH_3$ | H | |
| 6.20 | Br | 2-Cl | H | |
| 6.21 | Br | 3-F | H | |

Tabelle 7

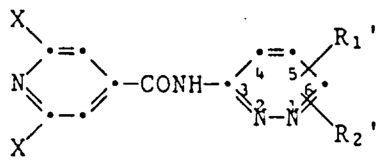

| Nr. | X | $R_1$' | $R_2$' | physik. Daten |
|---|---|---|---|---|
| 7.1 | Cl | H | H | Smp. >250°C |
| 7.2 | Cl | 6-Cl | H | |
| 7.3 | Cl | 4-$CH_3$ | 6-Cl | |
| 7.4 | Cl | 5-$CH_3$ | H | |
| 7.5 | Cl | 5-$CH_3$ | H | |
| 7.6 | Br | H | H | |
| 7.7 | Br | 6-Cl | H | |
| 7.8 | Br | 6-$CH_3$ | H | |
| 7.9 | Cl | 6-$CH_3$ | H | |
| 7.10 | Cl | 6-$SCH_3$ | H | |
| 7.11 | Cl | 6-$OCH_3$ | H | |
| 7.12 | Cl | 6-$SC_2H_5$ | H | |
| 7.13 | F | H | H | |
| 7.14 | F | 6-Cl | H | |
| 7.15 | J | H | H | |

Tabelle 8

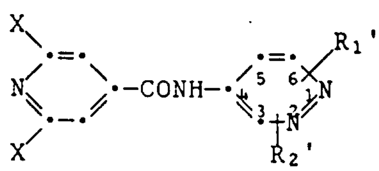

| Nr. | X | $R_1$' | $R_2$' | physik. Daten |
|---|---|---|---|---|
| 8.1 | Cl | H | H | |
| 8.2 | Br | H | H | |
| 8.3 | F | H | H | |
| 8.4 | Cl | 3-Cl | 5-Cl | |
| 8.5 | Cl | 3-Cl | 6-Cl | |
| 8.6 | Cl | 3-$CH_3$ | H | |
| 8.7 | Cl | 3-Cl | H | |
| 8.8 | Cl | 3-$OCH_3$ | 6-$CH_3$ | |
| 8.9 | Cl | 3-Cl | 6-$CH_3$ | |
| 8.10 | Cl | 3-$OCH_3$ | 6-$OCH_3$ | |
| 8.11 | Cl | 6-$CH_3$ | H | |
| 8.12 | Cl | 6-Cl | H | |
| 8.13 | Br | 3-Cl | 5-Cl | |
| 8.14 | Br | 3-Cl | 6-Cl | |
| 8.15 | Br | 3-$OCH_3$ | 6-$OCH_3$ | |

Tabelle 9

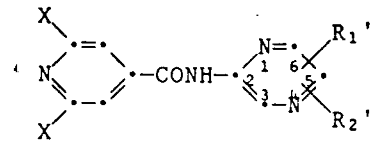

| Nr. | X | $R_1$' | $R_2$' | physik. Daten |
|---|---|---|---|---|
| 9.1 | Cl | H | H | |
| 9.2 | Br | H | H | |
| 9.3 | Cl | $3-CH_3$ | H | |
| 9.4 | Cl | $5-CH_3$ | H | |
| 9.5 | Cl | $3-C_2H_5$ | H | |
| 9.6 | Cl | $3-C_2H_5$ | $5-CH_3$ | |
| 9.7 | Cl | $3-CH_3$ | $6-CH_3$ | |
| 9.8 | Cl | $6-C_3H_9$(sec.) | H | |
| 9.9 | Cl | $5-CH_3$ | $6-CH_3$ | |
| 9.10 | Br | $3-CH_3$ | H | |
| 9.11 | Cl | 3-Br | H | |
| 9.12 | Cl | 5-Br | H | |
| 9.13 | Cl | 3-Cl | 5-Cl | |
| 9.14 | Cl | 5-Br | 3-Cl | |
| 9.15 | Cl | 3-Cl | H | |
| 9.16 | Cl | 5-Cl | H | |
| 9.17 | Cl | 3-Cl | $5-CH_3$ | |
| 9.18 | F | H | H | |
| 9.19 | J | H | H | |

Tabelle 10

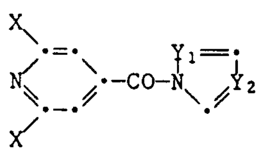

| Nr. | X | $Y_1$ | $Y_2$ | physik. Daten |
|---|---|---|---|---|
| 10.1 | Cl | N | N | Smp. 110-113°C |
| 10.2 | Cl | CH | N | Smp. 148-151°C |
| 10.3 | Br | N | N | |
| 10.4 | Br | CH | N | |
| 10.5 | J | N | N | |
| 10.6 | F | N | N | |
| 10.7 | F | CH | N | |
| 10.8 | Cl | CH | CH | |
| 10.9 | Br | CH | CH | |
| 10.10 | Cl | N | CH | |
| 10.11 | Br | N | CH | |

2. Formulierungsbeispiele für flüssige Wirkstoffe der Formel I (% = Gewichtsprozent)

2.1 Emulsions-Konzentrate

| | a) | b) | c) |
|---|---|---|---|
| Wirkstoff aus den Tabellen | 25 % | 40 % | 50 % |
| Ca-Dodecylbenzolsulfonat | 5 % | 8 | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

2.2 Lösungen

| | a) | b) | c) | d) |
|---|---|---|---|---|
| Wirkstoff aus den Tabellen | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 ° C) | - | - | 94 % | - |
| (MG = Molekulargewicht) | | | | |

Die Lösungen sind zur Anwendung in Form kleinster Tropfen geeignet.

2.3 Granulate

| | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Stäubemittel

| | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

2.5 Spritzpulver

| | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

2.6 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.7 Stäubemittel

| | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

2.8 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.9 Umhüllungs-Granulat

| | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Lingninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffe hergestellten Spritzbrühe besprüht (Konzentration: 200 ppm).

Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C bis 23 °C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen).

Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C weitergeführt.

Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchigkeit und bei 22 ° bis 23 °C weitergeführt.

Die Beurteilung der Schutzwirkung erfolgt aufgrund des Pilzbefalls 7-8 Tage nach der Infektion.

Verbindungen aus den Tabellen 1 bis 10 zeigten in den Tests (a) und (b) gute Wirkung. So reduzierten z.B. die Verbindungen 1.1, 1.2, 1.5, 1.6, 1.9, 1.18, 1.35, 1.55, 1.56, 1.60, 1.63, 1.64, 1.71, 1.73, 1.80, 1.85, 1.96, 1.100, 1.114, 1.149, 1.156, 2.6, 2.7, 2.18, 2.27, 2.31, 2.36, 2.39, 2.49, 2.56, 3.1, 3.7, 3.19, 3.33, 3.40,

3.77, 3.94, 4.1, 4.2, 4.3, 6.1, 7.1, 10.1 und 10.2 den Pilzbefall auf 0 bis 20 %. Unbehandelte jedoch infizierte Kontrollpflanzen wiesen dagegen einen Colletotrichum-Befall von 100 % auf.

Beispiel 3.2: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

b) Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

Verbindungen aus den Tabellen 1 bis 10 zeigten gegen Puccinia-Pilze eine gute Wirkung. So reduzierten z.B. die Verbindungen 1.1, 1.3, 1.4, 1.9, 1.56, 1.60, 1.63, 1.71, 1.79, 1.80, 1.85, 1.92, 1.100, 1.112, 2.1, 2.39, 2.49, 3.7, 3.94, 4.1, 6.1, 7.1 und 10.2 den Pilzbefall auf 0 bis 20 %. Unbehandelte jedoch infizierte Kontrollpflanzen wiesen dagegen einen Puccinia-Befall von 100 % auf.

Beispiel 3.3: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 9O-100 % relativer Luftfeuchtigkeit und 20 °C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1 bis 10 zeigten gegen den Phytophthora-Pilz eine gute Schutzwirkung. So reduzierten z.B. die Verbindungen 1.1, 1.4, 1.6, 1.7, 1.35, 1.74, 1.75, 1.89, 1.96, 2.6, 2.39, 2.49, 3.38, 3.74, 3.77, 5.1 und 7.1 den Pilzbefall auf 0 bis 20 %. Unbehandelte jedoch infizierte Kontrollpflanzen wiesen dagegen einen 100 %igen Phytophthora-Befall auf.

Beispiel 3.4: Wirkung gegen Cercospora arachidicola auf Erdnusspflanzen

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, jedoch infizierten Kontrollpflanzen (Anzahl und Grösse der Flecken = 100 %), zeigten Erdnusspflanzen, die mit Wirkstoffen aus den Tabellen 1 bis 10 behandelt wurden, einen reduzierten Cercospora-Befall (10-20 %), so z.B. die Verbindungen, 1.3, 1.41, 1.57, 1.112, 1.114, 2.31 und 2.39.

Beispiel 3.5: Wirkung gegen Plasmopara viticola auf Reben

a) Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 %

relativer Luftfeuchtigkeit und 20 °C wird der Pilzbefall beurteilt.

b) Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C werden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen 1 bis 10 zeigten gegen Plasmopara viticola eine gute Schutzwirkung (Befall 0-20 %), so z.B. 1.5, 1.6, 1.18 und 1.56. Unbehandelte jedoch infizierte Kontrollpflanzen wiesen dagegen einen 100%igen Plasmopara-Befall auf.

Beispiel 3.6: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt.

b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24 °C wird der Pilzbefall beurteilt.

Reispflanzen, die mit einer Spritzbrühe behandelt wurden, die als Aktivsubstanz eine Verbindung aus den Tabellen 1 bis 10 enthielt, zeigten im Vergleich zu unbehandelten Kontrollpflanzen (100 % Befall) nur geringen Pilzbefall. So reduzierten z.B. im Test (a) die Verbindungen 1.2, 1.7, 1.9, 1.20, 1.27, 1.35, 1.55, 1.57, 1.58, 1.63, 1.79, 1.80, 1.83, 1.85, 1.92, 1.94, 1.129, 2.18, 2.36, 2.39, 2.49, 3.2, 3.33, 3.35, 3.38, 3.40, 3.49, 3.67, 3.71, 3.74, 3.94 und im Test (b) die Verbindungen 1.2, 1.35, 1.55, 1.58, 1.60, 1.63, 1.92, 1.100, 1.120, 1.129, 1.156 und 3.17 den Pilzbefall auf 0 bis 20 %.

Beispiel 3.7: Wirkung gegen Pseudomonas tomato an Tomatenpflanzen

a) Tomatenpflanzen werden nach 3-wöchiger Anzucht mit einer aus Spritzpulver des Wirkstoffes hergestellten Spritzbrühe durch Blattapplikation behandelt (Konzentration: 200 ppm). Nach 3,5 Wochen werden die Pflanzen mit einer Bakteriensuspension ($10^8$ Bakterien/ml) inokuliert und für 6 Tage bei hoher Luftfeuchtigkeit und bei einer Temperatur von 25 °C inkubiert. Die Beurteilung der Schutzwirkung erfolgt aufgrund des Bakterienbefalls 7-8 Tage nach Inokulation.

b) Tomatenpflanzen werden nach 3-wöchiger Anzucht mit einer aus Spritzpulver des Wirkstoffes hergestellten Spritzbrühe durch Bodenapplikation behandelt (Konzentration: 60 ppm bezogen auf das Bodenvolumen). Nach 3,5 Wochen werden die Pflanzen mit Bakteriensuspension ($10^8$ Bakterien/ml) inokuliert und für 6 Tage bei hoher Luftfeuchtigkeit und bei einer Temperatur von 25 °C inkubiert.

Die Beurteilung der Schutzwirkung erfolgt aufgrund des Bakterienbefalls 7-8 Tage nach Inokulation.

Verbindungen aus den Tabellen 1 bis 10 zeigten gegen Pseudomonas eine gute Schutzwirkung. So verhinderten im Test (a) die Verbindungen 1.1, 1.6, 1.60, 1.63, 1.73, 1.94, 1.100, 1.112, 1.114, 2.1, 2.27, 2.31, 3.33 und im Test (b) die Verbindungen 1.1, 1.4, 1.6, 1.7, 1.8, 1.35, 1.57, 1.58, 1.60, 1.63, 1.73, 1.74, 1.75, 1.79, 1.85, 1.89, 1.92, 1.94, 1.100, 2.7, 2.31, 2.39, 2.56, 3.1, 3.33 und 3.94 den Bakterienbefall auf 0 bis 20 %. Unbehandelte jedoch infizierte Kontrollpflanzen wiesen dagegen einen 100%-igen Pseudomonas-Befall auf.

Beispiel 3.8: Wirkung gegen den Tabakmosaik-Virus auf Tabak

8 Wochen alte Tabakpflanzen werden mit einer formulierten Lösung des Wirkstoffes besprüht (Konzentration: 200 ppm) oder injiziert (Konzentration: 200 ppm). Nach 4 Tagen werden die Pflanzen mit einer Suspension des Tabakmosaik-Virus (0.5 µg/ml + Carborundum) mechanisch inokuliert und bei einer Temperatur von 20° - 22 °C inkubiert.

Die Beurteilung der Schutzwirkung erfolgt aufgrund der Anzahl und Grösse der Lokalläsionen 7 Tage nach der Inokulation.

Verbindungen aus den Tabellen 1 bis 10 zeigten eine gute Schutzwirkung gegen den Tabakmosaik-Virus. Infizierte jedoch unbehandelte Kontrollpflanzen wiesen dagegen Läsionen von 100 % auf.

Beispiel 3.9: Wirkung gegen Pseudomonas lachrymans auf Cucumis sativus L.

a) Gurkenpflanzen werden nach 2-wöchiger Anzucht mit einer aus Spritzpulver des Wirkstoffes hergestellten Spritzbrühe besprüht (Konzentration: 20 ppm).

Nach 1 Woche werden die Pflanzen mit einer Bakteriensuspension ($10^8$ Bakterien/ml) infiziert und für 7 Tage bei hoher Luftfeuchtigkeit und einer Temperatur von 23°C inkubiert.

Die Beurteilung der Schutzwirkung erfolgt aufgrund des Bakterienbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentrationen: 60, 20, 6, 2 ppm bezogen auf das Bodenvolumen).

Nach 1 Woche werden die Pflanzen mit einer Bakteriensuspension ($10^8$ Bakterien/ml) infiziert und für 7 Tage bei hoher Luftfeuchtigkeit und einer Temperatur von 23°C inkubiert.

Die Beurteilung der Schutzwirkung erfolgt aufgrund des Bakterienbefalls 7 - 8 Tage nach der Infektion.

Verbindungen aus den Tabellen 1 bis 10 zeigten eine gute Schutzwirkung gegen Pseudomonasbefall. So wurde der Bakterienbefall in Test (a) durch die Verbindungen 1.18, 1.35, 1.85, 1.103, 2.7, 2.30, 2.31, 4.1, 6.1 und im Test (b) durch die Verbindungen 1.18, 2.7, 2.31, 2.36 und 2.85 auf 0-20 % reduziert. Unbehandelte jedoch infizierte Kontrollpflanzen wiesen dagegen einen 100 %igen Pseudomonas-Befall auf.

Beispiel 3.10: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocken dieses Spritzbelags werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 bis 10 zeigten eine gute Schutzwirkung gegen Xanthomonas oryzae. So reduzierten z.B. im Test (a) die Verbindungen 1.3, 1.4, 1.5, 1.8, 1.9, 1.56, 1.58, 1.59, 1.60, 1.63, 1.64, 1.92, 1.100, 1.114, 1.120, 1.129, 2.1, 2.27, 2.31, 3.19, 3.33 und 3.34 und im Test (b) die Verbindungen 1.3, 1.6, 1.8, 1.9, 1.55, 1.56, 1.57, 1.58, 1.59, 1.60, 1.63, 1.85, 1.92, 1.94, 1.100, 1.129, 1.149, 2.1, 2.27, 2.39, 2.49 und 3.17 den Bakterienbefall auf 0 bis 20 %. Unbehandelte jedoch infizierte Kontrollpflanzen wiesen dagegen einen Krankheitsbefall von 100 % auf.

Beispiel 3.11: Wirkung gegen Xanthomonas vesicatoria auf Paprika (Capsicum annuum)

a) Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelages werden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der residualen Wirksamkeit der Prüfsubstanz.

b) Paprikapflanzen der Sorte "California Wonder" werden nach 3-wöchiger Anzucht im Gewächshaus mit einer Suspension der Prüfsubstanz gegossen (0,006 % Aktivsubstanz bezogen auf das Erdvolumen).

Drei Tage nach dieser Behandlung werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der systemischen Wirksamkeit der Prüfsubstanz.

Verbindungen aus den Tabellen 1 bis 10 zeigten eine gute Schutzwirkung gegen Xanthomonas vesicatoria. So reduzierten z.B. im Test (a) die Verbindungen 1.1, 1.57, 1.58, 1.85, 1.89, 1.112, 2.21, 2.31, 3.5, 3.19, 3.27, 3.7 und 3.9 und im Test (b) die Verbindungen 1.1, 1.4, 1.6, 1.7, 1.9, 1.35, 1.41, 1.74, 1.75, 1.79, 1.85, 1.89, 1.103, 1.112, 1.129, 2.21, 2.31, 2.56, 3.1, 3.34, 3.7 und 3.9 den Bakterienbefall auf 0 bis 20 %. Unbehandelte jedoch infizierte Kontrollpflanzen wiesen dagegen einen Krankheitsbefall von 100 % auf.

Beispiel 3.12: Beizwirkung gegen Fusarium nivale an Roggen

Natürlich mit Fusarium nivale infizierter Roggen der Sorte Tetrahell wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Der infizierte und behandelte Roggen wird im Oktober im Freiland mittels einer Sämaschine auf Parzellen von 3 m Länge und 6 Saatreihen ausgesät, mit 3 Wiederholungen pro Versuchsprodukt.

Bis zur Befallsauswertung wird die Versuchspflanzung unter normalen Feldbedingungen kultiviert (vorzugsweise in einer Region mit geschlossener Schneedecke während der Wintermonate).

Zur Ermittlung der Wirkstoffaktivität wird im Frühjahr unmittelbar nach der Schneeschmelze, der prozentuale Anteil mit Fusarium befallener Pflanzen ausgezählt.

Verbindungen aus den Tabellen 1 bis 10 zeigten eine gute Schutzwirkung gegen Fusarium. Unbehandelte jedoch infizierte Kontrollpflanzen wiesen einen Krankheitsbefall von 100 % auf.

Beispiel 3.13: Beizwirkung gegen Helminthosporium gramineum an Gerste

Natürlich mit Helminthosporium gramineum infizierte Wintergerste der Sorte "Cl" wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Die infizierte und behandelte Gerste wird im Oktober im Freiland mittels einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil mit Helminthospora befallener Halme ausgezählt.

Verbindungen aus den Tabellen 1 bis 10 zeigten eine gute Schutzwirkung gegen Helminthosporium. Unbehandelte jedoch infizierte Kontrollpflanzen wiesen einen Krankheitsbefall von 100 % auf.

Beispiel 3.14: Beizwirkung gegen Ustilago nuda an Gerste

Natürlich mit Ustilago nuda infizierte Wintergerste der Sorte "RM1" wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Die infizierte und behandelte Gerste wird im Oktober im Freiland mittels einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität wird während der Blüte der prozentuale Anteil Ustilago befallener Aehren ausgezählt.

Verbindungen aus den Tabellen 1 bis 10 zeigten eine gute Schutzwirkung gegen Ustilago. Unbehandelte jedoch infizierte Kontrollpflanzen wiesen einen Krankheitsbefall von 100 % auf.

Beispiel 3.15: Beizwirkung gegen Colletotrichum lagenarium an Cucumis sativus L.

Gurkensamen werden mit einer Lösung des Wirkstoffes gebeizt (Konzentration: 180 g/100 kg Samen). Die Samen werden ausgesät. Nach 4 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 ° bis 23 °C weitergeführt. Die Beurteilung der Schutzwirkung erfolgt aufgrund des Pilzbefalls 7 - 8 Tage nach der Infektion.

Verbindungen aus den Tabellen 1 bis 10 zeigten eine gute Schutzwirkung gegen Colletotrichum. Infizierte Kontrollpflanzen, deren Samen nicht behandelt wurden, wiesen einen Pilzbefall von 100 % auf.

Beispiel 3.16: Residual-protektive Wirkung gegen Venturia inaequalis auf Apfeltrieben

Apfelstecklinge mit 10 - 20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90 - 100 % relativer Luftfeuchtigkeit inkubiert und während 10 weiteren Tagen in einem Gewächshaus bei 20 - 24 °C aufgestellt. Der Schorfbefall wird 15 Tage nach der Infektion beurteilt.

Verbindungen aus den Tabellen 1 bis 10 zeigten gute Schutzwirkung gegen Venturia. So reduzierte z.B. die Verbindung 1.114 den Schorfbefall auf 5 bis 20 %. Unbehandelte jedoch infizierte Triebe wiesen dagegen einen 100 %igen Venturia-Befall auf.

Beispiel 3.17: Wirkung gegen Erysiphe graminis auf Gerste

a) Ca. 8 cm hohe Gerstenpflanzen werden mit einer aus Spritzpulver des Wirkstoffes hergestellten Spritzbrühe (0,02 % Aktivsubstanz) besprüht. Nach 3 - 4 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

b) Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt.

Nach 48 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

Verbindungen aus den Tabellen 1 bis 10, z.B. die Verbindungen 1.1, 1.2, 1.5, 1.7, 1.9, 1.18, 1.20, 1.27, 1.35, 1.41, 1.53, 1.56, 1.63, 1.64, 1.67, 1.79, 1.80, 1.85, 1.89, 1.94, 1.100, 1.103, 1.120, 1.149, 2.18, 2.36, 3.71, 4.1, 4.2, 10.1 und 10.2 reduzierten den Pilzbefall auf weniger als 20 %, während unbehandelte aber infizierte Kontrollpflanzen zu 100 % befallen waren.

**Patentansprüche**

**Patentansprüche für folgende Vertragsstaaten : AT, BE, CH, DE, FR, GB, IT, LI, NL**

**1.** Verbindungen der Formel I

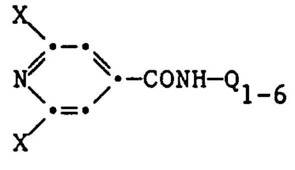

(I),

in welcher bedeuten:

X in ortho/ortho'-Stellung gleiche Atome Fluor, Chlor, Brom oder Jod;

$Q_1$ unsubstituiertes oder durch $R_1$, $R_2$ und $R_3$ substituiertes Pyrimidin-4-yl;

$Q_2$ unsubstituiertes oder durch $R_1$, $R_2$ und $R_3$ substituiertes Pyrimidin-2-yl;

$Q_3$ unsubstituiertes oder durch $R_1$, $R_2$ und $R_3$ substituiertes Pyrimidin-5-yl;

$Q_4$ unsubstituiertes oder durch $R_1$, $R_2$ und $R_3$ substituiertes Pyridin-2-yl, Pyridin-3-yl oder Pyridin-4-yl;

$Q_5$ unsubstituiertes oder durch $R_1$, $R_2$ und $R_3$ substituiertes Pyridazin-3-yl oder Pyridazin-4-yl;

$Q_6$ unsubstituiertes oder durch $R_1$, $R_2$ und $R_3$ substituiertes Pyrazin-2-yl;

$R_1$, $R_2$, $R_3$ Wasserstoff, Halogen, $C_1$-$C_6$-Alkyl, $C_1$-$C_6$-Haloalkyl mit 1 bis 3 Halogenatomen, $C_1$-$C_6$-Alkoxy, $C_2$-$C_6$-Alkoxyalkyl, $C_1$-$C_6$-Thioalkyl, $C_1$-$C_6$-Haloalkoxy mit 1 bis 5 Halogenatomen, $C_3$-$C_6$-Alkenyloxy, $C_3$-$C_6$-Alkinyloxy, $C_3$-$C_6$-Thioalkenyl, $C_3$-$C_6$-Cycloalkyl, mit Methyl substituiertes $C_3$-$C_6$-Cycloalkyl, Nitro, Cyano, die Reste $CH(OR_4)_2$ oder $COOR_4$, wobei $R_4$ $C_1$-$C_4$-Alkyl darstellt; ferner Phenyl, Phenoxy, Thiophenyl, Benzyl, Benzyloxy oder Thiobenzyl; oder mindestens einmal mit $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Phenyl, Phenoxy, Thiophenyl, Benzyl, Benzyloxy oder Thiobenzyl; sowie $N(R_5)R_6$, wobei

$R_5$ und $R_6$ unabhängig voneinander $C_1$-$C_6$-Alkyl darstellt; darüber hinaus Piperidinyl, Pyrrolidinyl, Morpholinyl, Imidazolyl oder Triazolyl, oder durch $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Piperidinyl, Pyrrolidinyl, Morpholinyl, Imidazolyl oder Triazolyl.

**2.** Verbindungen der Formel I, gemäss Anspruch 1,

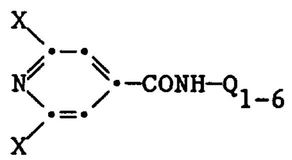

(I),

in welcher bedeuten:
X in ortho/ortho'-Stellung gleiche Atome Fluor, Chlor, Brom oder Jod;
$Q_1$ unsubstituiertes oder durch $R_1$, $R_2$ und $R_3$ substituiertes Pyrimidin-4-yl;
$Q_2$ unsubstituiertes oder durch $R_1$, $R_2$ und $R_3$ substituiertes Pyrimidin-2-yl;
$Q_3$ unsubstituiertes oder durch $R_1$, $R_2$ und $R_3$ substituiertes Pyrimidin-5-yl;
$Q_4$ unsubstituiertes oder durch $R_1$, $R_2$ und $R_3$ substituiertes Pyridin-2-yl, Pyridin-3-yl oder Pyridin-4-yl;
$Q_5$ unsubstituiertes oder durch $R_1$, $R_2$ und $R_3$ substituiertes Pyridazin-3-yl oder Pyridazin-4-yl;
$Q_6$ unsubstituiertes oder durch $R_1$, $R_2$ und $R_3$ substituiertes Pyrazin-2-yl;
$R_1$, $R_2$, $R_3$ Wasserstoff, Halogen, $C_1$-$C_6$-Alkyl, $C_1$-$C_6$-Haloalkyl mit 1 bis 3 Halogenatomen, $C_1$-$C_6$-Alkoxy, $C_2$-$C_6$-Alkoxyalkyl, $C_1$-$C_6$-Thioalkyl, $C_1$-$C_6$-Haloalkoxy mit 1 bis 5 Halogenatomen, $C_3$-$C_6$-Alkenyloxy, $C_3$-$C_6$-Alkinyloxy, $C_3$-$C_6$-Thioalkenyl, $C_3$-$C_6$-Cycloalkyl, Nitro, Cyano oder $COOR_4$, wobei $R_4$ $C_1$-$C_4$-Alkyl darstellt; ferner Phenyl, Phenoxy, Thiophenyl, Benzyl, Benzyloxy oder Thiobenzyl; oder mindestens einmal mit $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Phenyl, Phenoxy, Thiophenyl, Benzyl, Benzyloxy oder Thiobenzyl; sowie $N(R_5)R_6$, wobei $R_5$ und $R_6$ unabhängig voneinander $C_1$-$C_6$-Alkyl darstellt; darüber hinaus Piperidinyl, Pyrrolidinyl, Morpholinyl, Imidazolyl oder Triazolyl, oder durch $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Piperidinyl, Pyrrolidinyl, Morpholinyl, Imidazolyl oder Triazolyl.

**3.** Verbindungen der Formel I gemäss Anspruch 2, in denen $Q_1$ durch $R_1$, $R_2$ und $R_3$ substituiertes Pyrimidin-4-yl darstellt und X, $R_1$, $R_2$ und $R_3$ die unter Formel I angegebenen Bedeutungen besitzen.

**4.** Verbindungen der Formel I gemäss Anspruch 2, in denen $Q_2$ durch $R_1$, $R_2$ und $R_3$ substituiertes Pyrimidin-2-yl darstellt und X, $R_1$, $R_2$ und $R_3$ die unter Formel I angegebenen Bedeutungen besitzen.

**5.** Verbindungen der Formel I gemäss Anspruch 2, in denen $Q_3$ durch $R_1$, $R_2$ und $R_3$ substituiertes Pyrimidin-5-yl darstellt und X, $R_1$, $R_2$ und $R_3$ die unter Formel I angegebenen Bedeutungen besitzen.

**6.** Verbindungen der Formel I gemäss Anspruch 2, in denen $Q_4$ durch $R_1$, $R_2$ und $R_3$ substituiertes Pyridin-2-yl, Pyridin-3-yl oder Pyridin-4-yl darstellt und X, $R_1$, $R_2$ und $R_3$ die unter Formel I angegebenen Bedeutungen besitzen.

**7.** Verbindungen der Formel I gemäss Anspruch 2, in denen $Q_5$ durch $R_1$, $R_2$ und $R_3$ substituiertes Pyridazin-3-yl oder Pyridazin-4-yl darstellt und X, $R_1$, $R_2$ und $R_3$ die unter Formel I angegebenen Bedeutungen besitzen.

**8.** Verbindungen der Formel I gemäss Anspruch 2, in denen $Q_6$ durch $R_1$, $R_2$ und $R_3$ substituiertes Pyrazin-2-yl darstellt und X, $R_1$, $R_2$ und $R_3$ die unter Formel I angegebenen Bedeutungen besitzen.

**9.** Verbindungen der Formel I gemäss Anspruch 3, in denen X in ortho/ortho'-Stellung gleiche Atome Fluor, Chlor, Brom oder Jod darstellt, $Q_1$ Pyrimidin-4-yl bedeutet und $R_1$, $R_2$ und $R_3$ als Substituenten in 2-, 5- oder 6-Stellung stehen für Wasserstoff, Halogen, $C_1$-$C_6$-Alkyl, $C_1$-$C_3$-Haloalkyl mit 1 bis 3 Halogenatomen, $C_1$-$C_4$-Alkoxy, $C_2$-$C_4$-Alkoxyalkyl, $C_1$-$C_4$-Thioalkyl, $C_1$-$C_4$-Haloalkoxy mit 1 bis 3 Halogenatomen, $C_3$-$C_4$-Alkenyloxy, $C_3$-$C_4$-Alkinyloxy, $C_3$-$C_4$-Thioalkenyl, $C_3$-$C_5$-Cycloalkyl, Nitro, Cyano

oder $COOR_4$, wobei $R_4$ $C_1$-$C_4$-Alkyl darstellt, stehen ferner für Phenyl, Phenoxy, Thiophenyl, Benzyl oder mit 1 bis 3 Halogenatomen substituiertes Phenyl oder Phenyloxy; darüber hinaus für $N(R_5)R_6$, wobei $R_5$ und $R_6$ unabhängig voneinander $C_1$-$C_4$-Alkyl darstellen; sowie für Piperidinyl, Pyrrolidinyl, Morpholinyl, Imidazolyl oder Triazolyl, oder durch $C_1$-$C_3$-Alkyl oder Halogen substituiertes Piperidinyl, Pyrrolidinyl, Morpholinyl, Imidazolyl oder Triazolyl.

**10.** Verbindungen der Formel I gemäss Anspruch 3, in denen X Fluor, Chlor oder Brom darstellt, $Q_1$ Pyrimidin-4-yl bedeutet und $R_1$, $R_2$ und $R_3$ in 2-, 5-oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Thioallylen, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino, Piperidinyl, Pyrrolidinyl, Morpholinyl, Imidazolyl oder 1,2,4-Triazolyl.

**11.** Verbindungen der Formel I gemäss Anspruch 10, in denen X Chlor darstellt, $Q_1$ Pyrimidin-4-yl bedeutet und $R_1$, $R_2$ und $R_3$ in 2-, 5-oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**12.** Verbindungen der Formel I gemäss Anspruch 4, in denen X Fluor, Chlor, Brom oder Jod darstellen, $Q_2$ Pyrimidin-2-yl bedeutet und $R_1$, $R_2$ und $R_3$ in 4-, 5- oder 6-Stellung stehen für Wasserstoff, Halogen, $C_1$-$C_4$-Alkyl, $C_1$-$C_2$-Haloalkyl mit 1 bis 3 Halogenatomen, $C_1$-$C_3$-Alkoxy, $C_1$-$C_4$-Thioalkyl, $C_1$-$C_2$-Haloalkoxy mit 1 bis 3 Halogenatomen, $C_3$-$C_4$-Alkenyloxy, $C_3$-$C_4$-Alkinyloxy, $C_3$-$C_5$-Cycloalkyl, Phenoxy, Thiophenyl, Thiobenzyl; ferner $N(R_5)R_6$, wobei $R_5$ und $R_6$ unabhängig voneinander $C_1$-$C_2$-Alkyl darstellen; sowie Piperidinyl oder Morpholinyl oder durch $C_1$-$C_3$-Alkyl oder Chlor substituiertes Morpholinyl.

**13.** Verbindungen der Formel I gemäss Anspruch 12, in denen X Fluor, Chlor oder Brom darstellt, $Q_2$ Pyrimidin-2-yl bedeutet und $R_1$, $R_2$ und $R_3$ in 4-, 5- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**14.** Verbindungen der Formel I gemäss Anspruch 13, in denen X Chlor darstellt, $Q_2$ Pyrimidin-2-yl bedeutet und $R_1$, $R_2$ und $R_3$ in 4-, 5- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**15.** Verbindungen der Formel I gemäss-Anspruch 5, in denen X Fluor, Chlor, Brom oder Jod darstellt, $Q_3$ Pyrimidin-5-yl bedeutet und $R_1$, $R_2$ und $R_3$ stehen für Wasserstoff, Halogen, $C_1$-$C_4$-Alkyl, $C_1$-$C_2$-Haloalkyl mit 1 bis 3 Halogenatomen, $C_1$-$C_5$-Alkoxy, $C_2$-$C_4$-Alkoxyalkyl, $C_1$-$C_4$-Thioalkyl, $C_1$-$C_3$-Haloalkoxy mit 1 bis 4 Halogenatomen, $C_3$-$C_4$-Alkenyloxy, $C_3$-$C_4$-Alkinyloxy, $C_3$-$C_5$-Cycloalkyl, Phenoxy, Thiophenyl oder Benzyloxy, mit Halogen substituiertes Phenoxy, Thiophenyl oder Benzyloxy; ferner für $N(R_5)R_6$, wobei $R_5$ und $R_6$ unabhängig voneinander $C_1$-$C_2$-Alkyl darstellen; sowie für Pyrrolidinyl oder Morpholinyl.

**16.** Verbindungen der Formel I gemäss Anspruch 15, in denen X Fluor, Chlor oder Brom darstellt, $Q_3$ Pyrimidin-5-yl bedeutet und $R_1$, $R_2$ und $R_3$ in 2-, 4- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**17.** Verbindungen der Formel I gemäss Anspruch 16, in denen X Chlor darstellt, $Q_3$ Pyrimidin-5-yl bedeutet und $R_1$, $R_2$ und $R_3$ in 2-, 4- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor- oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**18.** Eine Verbindung, gemäss Anspruch 1, aus der Gruppe:
N-(2-Cyclopropyl-5-ethyl-6-chlor-pyrimidin-4-yl)-2,6-dichlor-isonicotinsäureamid;
N-(2-Cyclopropyl-5-methyl-6-chlor-pyrimidin-4-yl)-2,6-dichlor-isonicotinsäureamid;
N-(2,5-Dimethyl-6-chlor-pyrimidin-4-yl)-2,6-dichlor-isonicotinsäureamid;
N-(2,5-Diethyl-6-chlor-pyrimidin-4-yl)-2,6-dichlor-isonicotinsäureamid;
N-(2-Clor-6-thiomethyl-pyrimidin-4-yl)-2,6-dichlor-isonicotinsäureamid;
N-(2-Methoxy-5-chlor-pyrimidin-4-yl)-2,6-dichlor-isonicotinsäureamid;
N-(2,5-Dichlor-pyrimidin-4-yl)-2,6-dichlor-isonicotinsäureamid;
N-(2,6-Dimethyl-pyrimidin-4-yl)-2,6-dichlor-isonicotinsäureamid;
N-(5-Methyl-6-chlor-pyrimidin-4-yl)-2,6-dichlor-isonicotinsäureamid;
N-(5-n-Propyl-6-chlor-pyrimidin-4-yl)-2,6-dichlor-isonicotinsäureamid;
N-(5-Ethyl-6-chlor-pyrimidin-4-yl)-2,6-dichlor-isonicotinsäureamid;
N-(2,5,6-Trichlor-pyrimidin-4-yl)-2,6-dichlor-isonicotinsäureamid;
N-(2-Dimethylamino-6-methoxy-pyrimidin-4-yl)-2,6-dichlor-isonicotinsäureamid;
N-(2-Ethyl-5-methyl-6-chlor-pyrimidin-4-yl)-2,6-dichlor-isonicotinsäureamid;
N-(2-Trifluormethoxy-5-chlor-pyrimidin-4-yl)-2,6-dichlor-isonicotinsäureamid;
N-(2-Methoxy-5-fluor-pyrimidin-4-yl)-2,6-dichlor-isonicotinsäureamid;
N-(2-Chlor-5-fluor-pyrimidin-4-yl)-2,6-dichlor-isonicotinsäureamid;
N-(2-Isopropoxy-5-chlor-pyrimidin-4-yl)-2,6-dichlor-isonicotinsäureamid;
N-(2-Isopropyl-6-chlor-pyrimidin-4-yl)-2,6-dichlor-isonicotinsäureamid;
N-(2-Chlor-6-n-propyl-pyrimidin-4-yl)-2,6-dichlor-isonicotinsäureamid;
N-(2-n-Propoxy-5-chlor-pyrimidin-4-yl)-2,6-dichlor-isonicotinsäureamid;
N-(2-Isopropyl-6-ethylthio-pyrimidin-4-yl)-2,6-dichlor-isonicotinsäureamid;
N-(2-Isopropyl-6-isopropylthio-pyrimidin-4-yl)-2,6-dichlor-isonicotinsäureamid;
N-(2-Chlor-6-methyl-pyrimidin-4-yl)-2,6-dichlor-isonicotinsäureamid;
N-Pyridin-2-yl-2,6-dichlor-isonicotinsäureamid;
N-(6-Methyl-pyridin-2-yl)-2,6-dichlor-isonicotinsäureamid;
N-(4-Methyl-pyridin-2-yl)-2,6-dichlor-isonicotinsäureamid;
N-(5-Chlor-pyridin-2-yl)-2,6-dichlor-isonicotinsäureamid;
N-Pyridin-4-yl-2,6-dichlor-isonicotinsäureamid.

**19.** Verbindungen der Formel V

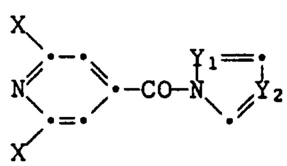

(V)

in welcher
X in ortho/ortho'-Stellung gleiche Atome Fluor, Chlor, Brom oder Jod bedeutet und $Y_1$ und $Y_2$ unabhängig voneinander N oder CH darstellen.

**20.** Verfahren zur Herstellung der Verbindungen der Formel 1 in Anspruch 1, dadurch gekennzeichnet, dass man umsetzt:

a) ein Isonicotinsäurehalogenid der Formel II

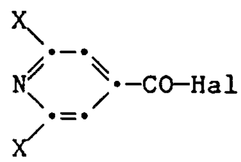

(II)

mit einem Amin der Formel III

$H_2N—Q_{1-6}$ (III)

in Gegenwart einer Base in einem inerten Lösungsmittel oder ohne Lösungsmittel; oder
b) ein Isonicotinsäureanhydrid der Formel IV

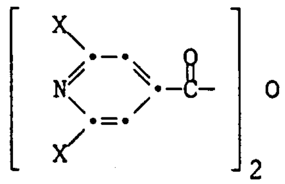

(IV)

mit einem Amin der Formel III

$H_2N—Q_{1-6}$ (III)

in Gegenwart einer Base in einem inerten Lösungsmittel oder ohne Lösungsmittel; oder
c) ein Isonicotinsäureazolid der Formel V

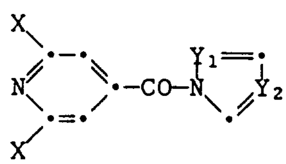

(V)

mit einem Amin der Formel III

$H_2N—Q_{1-6}$ (III)

in einem inerten Lösungsmittel unter Abscheidung eines Azols; wobei X und $Q_{1-6}$ die unter der Formel I angegebenen Bedeutungen besitzen, $Y_1$ und $Y_2$ unabhängig voneinander N oder CH darstellen, wobei $Y_2$ vorzugsweise N ist, und Hal für Halogen, vorzugsweise für Chlor, steht.

**21.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung der Formel I gemäss einem der Ansprüche 1-18 enthält.

**22.** Verwendung von Verbindungen der Formel I gemäss einem der Ansprüche 1 bis 18 zum Schutz von Pflanzen gegen den Befall durch phytopathogene Mikroorganismen.

**23.** Verfahren zum Schutz von Pflanzen gegen den Befall durch phytopathogene Mikroorganismen, dadurch gekennzeichnet, dass man als Wirkstoff eine Verbindung der Formel I gemäss einem der Ansprüche 1 bis 18 auf die Pflanze oder deren Standort appliziert.

**24.** Verfahren zum Schutz von Pflanzen gegen den Befall durch phytopathogene Mikroorganismen, dadurch gekennzeichnet, dass man als Wirkstoff eine Verbindung der Formel V gemäss Anspruch 19 auf die Pflanzen oder deren Standort appliziert.

**Patentansprüche für folgenden Vertragsstaat : ES**

**1.** Verfahren zur Herstellung der Verbindungen der Formel I

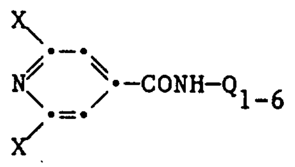

(I),

in welcher bedeuten:
X in ortho/ortho'-Stellung gleiche Atome Fluor, Chlor, Brom oder Jod;
$Q_1$ unsubstituiertes oder durch $R_1$, $R_2$ und $R_3$ substituiertes Pyrimidin-4-yl;
$Q_2$ unsubstituiertes oder durch $R_1$, $R_2$ und $R_3$ substituiertes Pyrimidin-2-yl;
$Q_3$ unsubstituiertes oder durch $R_1$, $R_2$ und $R_3$ substituiertes Pyrimidin-5-yl;
$Q_4$ unsubstituiertes oder durch $R_1$, $R_2$ und $R_3$ substituiertes Pyridin-2-yl, Pyridin-3-yl oder Pyridin-4-yl;
$Q_5$ unsubstituiertes oder durch $R_1$, $R_2$ und $R_3$ substituiertes Pyridazin-3-yl oder Pyridazin-4-yl;
$Q_6$ unsubstituiertes oder durch $R_1$, $R_2$ und $R_3$ substituiertes Pyrazin-2-yl;
$R_1$, $R_2$, $R_3$ Wasserstoff, Halogen, $C_1$-$C_6$-Alkyl, $C_1$-$C_6$-Haloalkyl mit 1 bis 3 Halogenatomen, $C_1$-$C_6$-Alkoxy, $C_2$-$C_6$-Alkoxyalkyl, $C_1$-$C_6$-Thioalkyl, $C_1$-$C_6$-Haloalkoxy mit 1 bis 5 Halogenatomen, $C_3$-$C_6$-Alkenyloxy, $C_3$-$C_6$-Alkinyloxy, $C_3$-$C_6$-Thioalkenyl, $C_3$-$C_6$-Cycloalkyl, mit Methyl substituiertes $C_3$-$C_6$-Cycloalkyl, Nitro, Cyano, die Reste $CH(OR_4)_2$ oder $COOR_4$, wobei $R_4$ $C_1$-$C_4$-Alkyl darstellt; ferner Phenyl, Phenoxy, Thiophenyl, Benzyl, Benzyloxy oder Thiobenzyl; oder mindestens einmal mit $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Phenyl, Phenoxy, Thiophenyl, Benzyl, Benzyloxy oder Thiobenzyl; sowie $N(R_5)R_6$, wobei $R_5$ und $R_6$ unabhängig voneinander $C_1$-$C_6$-Alkyl darstellt; darüber hinaus Piperidinyl, Pyrrolidinyl, Morpholinyl, Imidazolyl oder Triazolyl, oder durch $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Piperidinyl, Pyrrolidinyl, Morpholinyl, Imidazolyl oder Triazolyl, dadurch gekennzeichnet, dass man umsetzt:

a) ein Isonicotinsäurehalogenid der Formel II

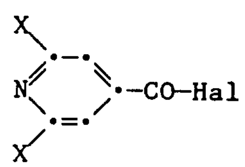

(II)

mit einem Amin der Formel III

$H_2N—Q_{1-6}$ (III)

in Gegenwart einer Base in einem inerten Lösungsmittel oder ohne Lösungsmittel; oder

b) ein Isonicotinsäureanhydrid der Formel IV

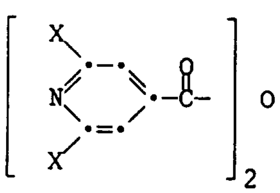

(IV)

mit einem Amin der Formel III

$H_2N—Q_{1-6}$ (III)

in Gegenwart einer Base in einem inerten Lösungsmittel oder ohne Lösungsmittel; oder
c) ein Isonicotinsäureazolid der Formel V

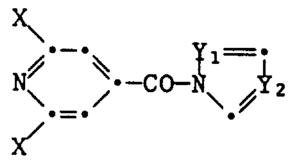

(V)

mit einem Amin der Formel III

$H_2N—Q_{1-6}$ (III)

in einem inerten Lösungsmittel unter Abscheidung eines Azols; wobei X und $Q_{1-6}$ die unter der Formel I angegebenen Bedeutungen besitzen, $Y_1$ und $Y_2$ unabhängig voneinander N oder CH darstellen, wobei $Y_2$ vorzugsweise N ist, und Hal für Halogen, vorzugsweise für Chlor, steht.

**2.** Verfahren nach Anspruch 1, dadurch gekennzeichnet, dass Verbindungen der Formel I

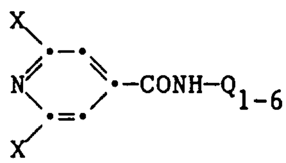

(I)

hergestellt werden,
in welcher bedeuten:
X in ortho/ortho'-Stellung gleiche Atome Fluor, Chlor, Brom oder Jod;
$Q_1$ unsubstituiertes oder durch $R_1$, $R_2$ und $R_3$ substituiertes Pyrimidin-4-yl;
$Q_2$ unsubstituiertes oder durch $R_1$, $R_2$ und $R_3$ substituiertes Pyrimidin-2-yl;
$Q_3$ unsubstituiertes oder durch $R_1$, $R_2$ und $R_3$ substituiertes Pyrimidin-5-yl;
$Q_4$ unsubstituiertes oder durch $R_1$, $R_2$ und $R_3$ substituiertes Pyridin-2-yl, Pyridin-3-yl oder Pyridin-4-yl;
$Q_5$ unsubstituiertes oder durch $R_1$, $R_2$ und $R_3$ substituiertes Pyridazin-3-yl oder Pyridazin-4-yl;
$Q_6$ unsubstituiertes oder durch $R_1$, $R_2$ und $R_3$ substituiertes Pyrazin-2-yl;
$R_1$, $R_2$, $R_3$ Wasserstoff, Halogen, $C_1$-$C_6$-Alkyl, $C_1$-$C_6$-Haloalkyl mit 1 bis 3 Halogenatomen, $C_1$-$C_6$-Alkoxy, $C_2$-$C_6$-Alkoxyalkyl, $C_1$-$C_6$-Thioalkyl, $C_1$-$C_6$-Haloalkoxy mit 1 bis 5 Halogenatomen, $C_3$-$C_6$-Alkenyloxy, $C_3$-$C_6$-Alkinyloxy, $C_3$-$C_6$-Thioalkenyl, $C_3$-$C_6$-Cycloalkyl, Nitro, Cyano oder $COOR_4$, wobei $R_4$ $C_1$-$C_4$-Alkyl darstellt; ferner Phenyl, Phenoxy, Thiophenyl, Benzyl, Benzyloxy oder Thiobenzyl; oder mindestens einmal mit $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Phenyl, Phenoxy, Thiophenyl, Benzyl, Benzyloxy oder Thiobenzyl; sowie $N(R_5)R_6$, wobei $R_5$ und $R_6$ unabhängig voneinander $C_1$-$C_6$-Alkyl darstellt; darüber hinaus Piperidinyl, Pyrrolidinyl, Morpholinyl, Imidazolyl oder Triazolyl, oder durch $C_1$-$C_3$-Alkyl, $C_1$-$C_3$-Alkoxy, $C_1$-$C_3$-Halogenalkyl mit 1 bis 3 Halogenatomen, Halogen, Nitro oder Cyano substituiertes Piperidinyl, Pyrrolidinyl, Morpholinyl, Imidazolyl oder Triazolyl.

**3.** Verfahren nach Anspruch 1, dadurch gekennzeichnet, dass Verbindungen der Formel I hergestellt werden, in denen $Q_1$ durch $R_1$, $R_2$ und $R_3$ substituiertes Pyrimidin-4-yl darstellt und X, $R_1$, $R_2$ und $R_3$ die unter Formel I angegebenen Bedeutungen besitzen.

**4.** Verfahren nach Anspruch 1, dadurch gekennzeichnet, dass Verbindungen der Formel I hergestellt werden, in denen $Q_2$ durch $R_1$, $R_2$ und $R_3$ substituiertes Pyrimidin-2-yl darstellt und X, $R_1$, $R_2$ und $R_3$ die unter Formel I angegebenen Bedeutungen besitzen.

5. Verfahren nach Anspruch 1, dadurch gekennzeichnet, dass Verbindungen der Formel I hergestellt werden, in denen $Q_3$ durch $R_1$, $R_2$ und $R_3$ substituiertes Pyrimidin-5-yl darstellt und X, $R_1$, $R_2$ und $R_3$ die unter Formel I angegebenen Bedeutungen besitzen.

6. Verfahren nach Anspruch 1, dadurch gekennzeichnet, dass Verbindungen der Formel I hergestellt werden, in denen $Q_4$ durch $R_1$, $R_2$ und $R_3$ substituiertes Pyridin-2-yl, Pyridin-3-yl oder Pyridin-4-yl darstellt und X, $R_1$, $R_2$ und $R_3$ die unter Formel I angegebenen Bedeutungen besitzen.

7. Verfahren nach Anspruch 1, dadurch gekennzeichnet, dass Verbindungen der Formel I hergestellt werden, in denen $Q_5$ durch $R_1$, $R_2$ und $R_3$ substituiertes Pyridazin-3-yl oder Pyridazin-4-yl darstellt und X, $R_1$, $R_2$ und $R_3$ die unter Formel I angegebenen Bedeutungen besitzen.

8. Verfahren nach Anspruch 1, dadurch gekennzeichnet, dass Verbindungen der Formel I hergestellt werden, in denen $Q_6$ durch $R_1$, $R_2$ und $R_3$ substituiertes Pyrazin-2-yl darstellt und X, $R_1$, $R_2$ und $R_3$ die unter Formel I angegebenen Bedeutungen besitzen.

9. Verfahren nach Anspruch 1, dadurch gekennzeichnet, dass Verbindungen der Formel I hergestellt werden, in denen X in ortho/ortho'-Stellung gleiche Atome Fluor, Chlor, Brom oder Jod darstellt, $Q_1$ Pyrimidin-4-yl bedeutet und $R_1$, $R_2$ und $R_3$ als Substituenten in 2-, 5-oder 6-Stellung stehen für Wasserstoff, Halogen, $C_1$-$C_6$-Alkyl, $C_1$-$C_3$-Haloalkyl mit 1 bis 3 Halogenatomen, $C_1$-$C_4$-Alkoxy, $C_2$-$C_4$-Alkoxyalkyl, $C_1$-$C_4$-Thioalkyl, $C_1$-$C_4$-Haloalkoxy mit 1 bis 3 Halogenatomen, $C_3$-$C_4$-Alkenyloxy, $C_3$-$C_4$-Alkinyloxy, $C_3$-$C_4$-Thioalkenyl, $C_3$-$C_5$-Cycloalkyl, Nitro, Cyano oder $COOR_4$, wobei $R_4$ $C_1$-$C_4$-Alkyl darstellt, stehen ferner für Phenyl, Phenoxy, Thiophenyl, Benzyl oder mit 1 bis 3 Halogenatomen substituiertes Phenyl oder Phenyloxy; darüber hinaus für $N(R_5)R_6$, wobei $R_5$ und $R_6$ unabhängig voneinander $C_1$-$C_4$-Alkyl darstellen; sowie für Piperidinyl, Pyrrolidinyl, Morpholinyl, Imidazolyl oder Triazolyl, oder durch $C_1$-$C_3$-Alkyl oder Halogen substituiertes Piperidinyl, Pyrrolidinyl, Morpholinyl, Imidazolyl oder Triazolyl.

10. Verfahren nach Anspruch 1, dadurch gekennzeichnet, dass Verbindungen der Formel I hergestellt werden, in denen X Fluor, Chlor oder Brom darstellt, $Q_1$ Pyrimidin-4-yl bedeutet und $R_1$, $R_2$ und $R_3$ in 2-, 5-oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Thioallylen, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino, Piperidinyl, Pyrrolidinyl, Morpholinyl, Imidazolyl oder 1,2,4-Triazolyl.

11. Verfahren nach Anspruch 1, dadurch gekennzeichnet, dass Verbindungen der Formel I hergestellt werden, in denen X Chlor darstellt, $Q_1$ Pyrimidin-4-yl bedeutet und $R_1$, $R_2$ und $R_3$ in 2-, 5-oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

12. Verfahren nach Anspruch 1, dadurch gekennzeichnet, dass Verbindungen der Formel I hergestellt werden, in denen X Fluor, Chlor, Brom oder Jod darstellen, $Q_2$ Pyrimidin-2-yl bedeutet und $R_1$, $R_2$ und $R_3$ in 4-, 5- oder 6-Stellung stehen für Wasserstoff, Halogen, $C_1$-$C_4$-Alkyl, $C_1$-$C_2$-Haloalkyl mit 1 bis 3 Halogenatomen, $C_1$-$C_3$-Alkoxy, $C_1$-$C_4$-Thioalkyl, $C_1$-$C_2$-Haloalkoxy mit 1 bis 3 Halogenatomen, $C_3$-$C_4$-Alkenyloxy, $C_3$-$C_4$-Alkinyloxy, $C_3$-$C_5$-Cycloalkyl, Phenoxy, Thiophenyl, Thiobenzyl; ferner $N(R_5)R_6$, wobei $R_5$ und $R_6$ unabhängig voneinander $C_1$-$C_2$-Alkyl darstellen; sowie Piperidinyl oder Morpholinyl oder durch $C_1$-$C_3$-Alkyl oder Chlor substituiertes Morpholinyl.

13. Verfahren nach Anspruch 1, dadurch gekennzeichnet, dass Verbindungen der Formel I hergestellt werden, in denen X Fluor, Chlor oder Brom darstellt, $Q_2$ Pyrimidin-2-yl bedeutet und $R_1$, $R_2$ und $R_3$ in 4-, 5- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**14.** Verfahren nach Anspruch 1, dadurch gekennzeichnet, dass Verbindungen der Formel I hergestellt werden, in denen X Chlor darstellt, $Q_2$ Pyrimidin-2-yl bedeutet und $R_1$, $R_2$ und $R_3$ in 4-, 5- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**15.** Verfahren nach Anspruch 1, dadurch gekennzeichnet, dass Verbindungen der Formel I hergestellt werden, in denen X Fluor, Chlor, Brom oder Jod darstellt, $Q_3$ Pyrimidin-5-yl bedeutet und $R_1$, $R_2$ und $R_3$ stehen für Wasserstoff, Halogen, $C_1$-$C_4$-Alkyl, $C_1$-$C_2$-Haloalkyl mit 1 bis 3 Halogenatomen, $C_1$-$C_5$-Alkoxy, $C_2$-$C_4$-Alkoxyalkyl, $C_1$-$C_4$-Thioalkyl, $C_1$-$C_3$-Haloalkoxy mit 1 bis 4 Halogenatomen, $C_3$-$C_4$-Alkenyloxy, $C_3$-$C_4$-Alkinyloxy, $C_3$-$C_5$-Cycloalkyl, Phenoxy, Thiophenyl oder Benzyloxy, mit Halogen substituiertes Phenoxy, Thiophenyl oder Benzyloxy; ferner für $N(R_5)R_6$, wobei $R_5$ und $R_6$ unabhängig voneinander $C_1$-$C_2$-Alkyl darstellen; sowie für Pyrrolidinyl oder Morpholinyl.

**16.** Verfahren nach Anspruch 1, dadurch gekennzeichnet, dass Verbindungen der Formel I hergestellt werden, in denen X Fluor, Chlor oder Brom darstellt, $Q_3$ Pyrimidin-5-yl bedeutet und $R_1$, $R_2$ und $R_3$ in 2-, 4- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**17.** Verfahren nach Anspruch 1, dadurch gekennzeichnet, dass Verbindungen der Formel I hergestellt werden, in denen X Chlor darstellt, $Q_3$ Pyrimidin-5-yl bedeutet und $R_1$, $R_2$ und $R_3$ in 2-, 4- oder 6-Stellung stehen für Wasserstoff, Fluor, Chlor, Brom, $C_1$-$C_4$-Alkyl, $C_1$-$C_2$-Haloalkyl mit 1 bis 3 Fluor- oder Chloratomen, $C_1$-$C_3$-Alkoxy, $C_2$-$C_4$-Alkoxyalkyl, $C_1$-$C_4$-Thioalkyl, $C_1$-$C_2$-Haloalkoxy mit 1 bis 3 Fluor-oder Chloratomen, $C_3$-$C_4$-Alkenyloxy, $C_3$-$C_4$-Alkinyloxy, Cyclopropyl, Nitro, Cyano oder $COOR_4$, wobei $R_4$ $C_1$-$C_4$-Alkyl darstellt; stehen ferner für Phenyl, Phenoxy, Thiophenyl, Benzyl, Dimethylamino.

**18.** Verfahren nach Anspruch 1, dadurch gekennzeichnet, dass eine Verbindung aus der Gruppe hergestellt wird:
N-(2-Cyclopropyl-5-ethyl-6-chlor-pyrimidin-4-yl)-2,6-dichlor-isonicotinsäureamid;
N-(2-Cyclopropyl-5-methyl-6-chlor-pyrimidin-4-yl)-2,6-dichlor-isonicotinsäureamid;
N-(2,5-Dimethyl-6-chlor-pyrimidin-4-yl)-2,6-dichlor-isonicotinsäureamid;
N-(2,5-Diethyl-6-chlor-pyrimidin-4-yl)-2,6-dichlor-isonicotinsäureamid;
N-(2-Clor-6-thiomethyl-pyrimidin-4-yl)-2,6-dichlor-isonicotinsäureamid;
N-(2-Methoxy-5-chlor-pyrimidin-4-yl)-2,6-dichlor-isonicotinsäureamid;
N-(2,5-Dichlor-pyrimidin-4-yl)-2,6-dichlor-isonicotinsäureamid;
N-(2,6-Dimethyl-pyrimidin-4-yl)-2,6-dichlor-isonicotinsäureamid;
N-(5-Methyl-6-chlor-pyrimidin-4-yl)-2,6-dichlor-isonicotinsäureamid;
N-(5-n-Propyl-6-chlor-pyrimidin-4-yl)-2,6-dichlor-isonicotinsäureamid;
N-(5-Ethyl-6-chlor-pyrimidin-4-yl)-2,6-dichlor-isonicotinsäureamid;
N-(2,5,6-Trichlor-pyrimidin-4-yl)-2,6-dichlor-isonicotinsäureamid;
N-(2-Dimethylamino-6-methoxy-pyrimidin-4-yl)-2,6-dichlor-isonicotinsäureamid;
N-(2-Ethyl-5-methyl-6-chlor-pyrimidin-4-yl)-2,6-dichlor-isonicotinsäureamid;
N-(2-Trifluormethoxy-5-chlor-pyrimidin-4-yl)-2,6-dichlor-isonicotinsäureamid;
N-(2-Methoxy-5-fluor-pyrimidin-4-yl)-2,6-dichlor-isonicotinsäureamid;
N-(2-Chlor-5-fluor-pyrimidin-4-yl)-2,6-dichlor-isonicotinsäureamid;
N-(2-Isopropoxy-5-chlor-pyrimidin-4-yl)-2,6-dichlor-isonicotinsäureamid;
N-(2-Isopropyl-6-chlor-pyrimidin-4-yl)-2,6-dichlor-isonicotinsäureamid;
N-(2-Chlor-6-n-propyl-pyrimidin-4-yl)-2,6-dichlor-isonicotinsäureamid;
N-(2-n-Propoxy-5-chlor-pyrimidin-4-yl)-2,6-dichlor-isonicotinsäureamid;
N-(2-Isopropyl-6-ethylthio-pyrimidin-4-yl)-2,6-dichlor-isonicotinsäureamid;
N-(2-Isopropyl-6-isopropylthio-pyrimidin-4-yl)-2,6-dichlor-isonicotinsäureamid;
N-(2-Chlor-6-methyl-pyrimidin-4-yl)-2,6-dichlor-isonicotinsäureamid;
N-Pyridin-2-yl-2,6-dichlor-isonicotinsäureamid;
N-(6-Methyl-pyridin-2-yl)-2,6-dichlor-isonicotinsäureamid;

N-(4-Methyl-pyridin-2-yl)-2,6-dichlor-isonicotinsäureamid;
N-(5-Chlor-pyridin-2-yl)-2,6-dichlor-isonicotinsäureamid;
N-Pyridin-4-yl-2,6-dichlor-isonicotinsäureamid.

**19.** Verfahren zur Herstellung der Verbindungen der Formel V

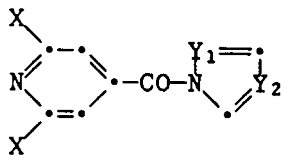

(V)

durch Umsetzung von Verbindungen der Formel II

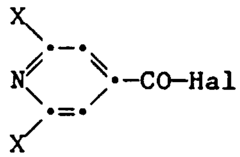

(II)

mit einem Azol der Formel VI

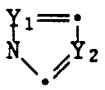

(VI)

in Gegenwart einer Base in einem inerten Lösungsmittel durchgeführt, wobei X die unter Formel I in Anspruch 1 angegebenen Bedeutungen hat, Hal für Halogen, vorzugsweise für Chlor, steht und $Y_1$ und $Y_2$ unabhängig voneinander N oder CH darstellt.

**Claims**

**Claims for the following Contracting States : AT, BE, CH, DE, FR, GB, IT, LI, NL**

**1.** A compound of formula I

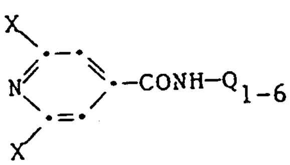

(I)

in which
X in ortho/ortho'-position are identical and represent fluorine, chlorine, bromine or iodine atoms;
$Q_1$ is unsubstituted pyrimidin-4-yl, or pyrimidin-4-yl substituted by $R_1$, $R_2$ and $R_3$;
$Q_2$ is unsubstituted pyrimidin-2-yl, or pyrimidin-2-yl substituted by $R_1$, $R_2$ and $R_3$;
$Q_3$ is unsubstituted pyrimidin-5-yl, or pyrimidin-5-yl substituted by $R_1$, $R_2$ and $R_3$;
$Q_4$ is pyridin-2-yl, pyridin-3-yl or pyridin-4-yl each of which is unsubstituted or substituted by $R_1$, $R_2$ and $R_3$;
$Q_5$ is pyridazin-3-yl or pyridazin-4-yl each of which is unsubstituted or substituted by $R_1$, $R_2$ and $R_3$;
$Q_6$ is unsubstituted pyrazin-2-yl, or pyrazin-2-yl substituted by $R_1$, $R_2$ and $R_3$;
$R_1$, $R_2$, $R_3$ are hydrogen, halogen, $C_1$-$C_6$alkyl, $C_1$-$C_6$haloalkyl having from 1 to 3 halogen atoms, $C_1$-$C_6$alkoxy, $C_2$-$C_6$alkoxyalkyl, $C_1$-$C_6$thioalkyl, $C_1$-$C_6$haloalkoxy having from 1 to 5 halogen atoms, $C_3$-$C_6$alkenyloxy, $C_3$-$C_6$alkynyloxy, $C_3$-$C_6$thioalkenyl, $C_3$-$C_6$cycloalkyl, $C_3$-$C_6$cycloalkyl substituted by methyl, or nitro, cyano, the radical $CH(OR_4)_2$ or $COOR_4$ in which $R_4$ is $C_1$-$C_4$alkyl; $R_1$, $R_2$, $R_3$ are also

phenyl, phenoxy, thiophenyl, benzyl, benzyloxy or thiobenzyl; or phenyl, phenoxy, thiophenyl, benzyl, benzyloxy or thiobenzyl each substituted at least once by $C_1$-$C_3$alkyl, $C_1$-$C_3$alkoxy, $C_1$-$C_3$haloalkyl having from 1 to 3 halogen atoms, halogen, nitro or by cyano; and $R_1$, $R_2$, $R_3$ are also $N(R_5)R_6$ in which $R_5$ and $R_6$, independently of one another, are each $C_1$-$C_6$alkyl; and furthermore piperidinyl, pyrrolidinyl, morpholinyl, imidazolyl or triazolyl, or piperidinyl, pyrrolidinyl, morpholinyl, imidazolyl or triazolyl each substituted by $C_1$-$C_3$alkyl, $C_1$-$C_3$alkoxy, $C_1$-$C_3$haloalkyl having from 1 to 3 halogen atoms, halogen, nitro or by cyano.

**2.** A compound of formula I according to claim 1

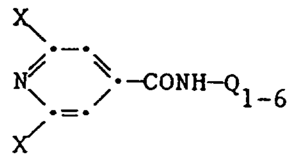

(I)

in which
X in ortho/ortho'-position are identical and represent fluorine, chlorine, bromine or iodine atoms;
$Q_1$ is unsubstituted pyrimidin-4-yl, or pyrimidin-4-yl substituted by $R_1$, $R_2$ and $R_3$;
$Q_2$ is unsubstituted pyrimidin-2-yl, or pyrimidin-2-yl substituted by $R_1$, $R_2$ and $R_3$;
$Q_3$ is unsubstituted pyrimidin-5-yl, or pyrimidin-5-yl substituted by $R_1$, $R_2$ and $R_3$;
$Q_4$ is pyridin-2-yl, pyridin-3-yl or pyridin-4-yl each of which is unsubstituted or substituted by $R_1$, $R_2$ and $R_3$;
$Q_5$ is pyridazin-3-yl or pyridazin-4-yl each of which is unsubstituted or substituted by $R_1$, $R_2$ and $R_3$;
$Q_6$ is unsubstituted pyrazin-2-yl, or pyrazin-2-yl substituted by $R_1$, $R_2$ and $R_3$;
$R_1$, $R_2$, $R_3$ are hydrogen, halogen, $C_1$-$C_6$alkyl, $C_1$-$C_6$haloalkyl having from 1 to 3 halogen atoms, $C_1$-$C_6$alkoxy, $C_2$-$C_6$alkoxyalkyl, $C_1$-$C_6$thioalkyl, $C_1$-$C_6$haloalkoxy having from 1 to 5 halogen atoms, $C_3$-$C_6$alkenyloxy, $C_3$-$C_6$alkynyloxy, $C_3$-$C_6$thioalkenyl, $C_3$-$C_6$cycloalkyl, nitro, cyano or $COOR_4$ in which $R_4$ is $C_1$-$C_4$alkyl; also phenyl, phenoxy, thiophenyl, benzyl, benzyloxy or thiobenzyl; or phenyl, phenoxy, thiophenyl, benzyl, benzyloxy or thiobenzyl each substituted at least once by $C_1$-$C_3$alkyl, $C_1$-$C_3$alkoxy, $C_1$-$C_3$haloalkyl having from 1 to 3 halogen atoms, halogen, nitro or by cyano; and also $N(R_5)R_6$ in which $R_5$ and $R_6$, independently of one another, are each $C_1$-$C_6$alkyl; and furthermore piperidinyl, pyrrolidinyl, morpholinyl, imidazolyl or triazolyl, or piperidinyl, pyrrolidinyl, morpholinyl, imidazolyl or triazolyl each substituted by $C_1$-$C_3$alkyl, $C_1$-$C_3$alkoxy, $C_1$-$C_3$haloalkyl having from 1 to 3 halogen atoms, halogen, nitro or by cyano.

**3.** A compound of formula I according to claim 2, in which $Q_1$ is pyrimidin-4-yl substituted by $R_1$, $R_2$ and $R_3$, and X, $R_1$, $R_2$ and $R_3$ are as defined for formula I.

**4.** A compound of formula I according to claim 2, in which $Q_2$ is pyrimidin-2-yl substituted by $R_1$, $R_2$ and $R_3$, and X, $R_1$, $R_2$ and $R_3$ are as defined for formula I.

**5.** A compound of formula I according to claim 2, in which $Q_3$ is pyrimidin-5-yl substituted by $R_1$, $R_2$ and $R_3$, and X, $R_1$, $R_2$ and $R_3$ are as defined for formula I.

**6.** A compound of formula I according to claim 2, in which $Q_4$ is pyridin-2-yl, pyridin-3-yl or pyridin-4-yl each substituted by $R_1$, $R_2$ and $R_3$, and X, $R_1$, $R_2$ and $R_3$ are as defined for formula I.

**7.** A compound of formula I according to claim 2, in which $Q_5$ is pyridazin-3-yl or pyridazin-4-yl each substituted by $R_1$, $R_2$ and $R_3$, and X, $R_1$, $R_2$ and $R_3$ are as defined for formula I.

**8.** A compound of formula I according to claim 2, in which $Q_6$ is pyrazin-2-yl substituted by $R_1$, $R_2$ and $R_3$, and X, $R_1$, $R_2$ and $R_3$ are as defined for formula I.

**9.** A compound of formula I according to claim 3, in which X in ortho/ortho'-position are identical and represent fluorine, chlorine, bromine or iodine atoms, $Q_1$ is pyrimidin-4-yl and $R_1$, $R_2$ and $R_3$ as substituents in the 2-, 5- and 6-positions are hydrogen, halogen, $C_1$-$C_6$alkyl, $C_1$-$C_3$haloalkyl having from

1 to 3 halogen atoms, $C_1$-$C_4$-alkoxy, $C_2$-$C_4$alkoxyalkyl, $C_1$-$C_4$thioalkyl, $C_1$-$C_4$haloalkoxy having from 1 to 3 halogen atoms, $C_3$-$C_4$alkenyloxy, $C_3$-$C_4$alkynyloxy, $C_3$-$C_4$thioalkenyl, $C_3$-$C_5$cycloalkyl, nitro, cyano or $COOR_4$ in which $R_4$ is $C_1$-$C_4$alkyl, and also phenyl, phenoxy, thiophenyl or benzyl, or phenyl or phenoxy each substituted by from 1 to 3 halogen atoms; and furthermore $N(R_5)R_6$ in which $R_5$ and $R_6$, independently of one another, are each $C_1$-$C_4$alkyl; as well as piperidinyl, pyrrolidinyl, morpholinyl, imidazolyl or triazolyl, or piperidinyl, pyrrolidinyl, morpholinyl, imidazolyl or triazolyl each substituted by $C_1$-$C_3$alkyl or by halogen.

**10.** A compound of formula I according to claim 3, in which X is fluorine, chlorine or bromine, $Q_1$ is pyrimidin-4-yl and $R_1$, $R_2$ and $R_3$ in the 2-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, thioallylene, cyclopropyl, nitro, cyano or $COOR_4$ in which $R_4$ is $C_1$-$C_4$alkyl; and also phenyl, phenoxy, thiophenyl, benzyl, dimethylamino, piperidinyl, pyrrolidinyl, morpholinyl, imidazolyl or 1,2,4-triazolyl.

**11.** A compound of formula I according to claim 10, in which X is chlorine, $Q_1$ is pyrimidin-4-yl and $R_1$, $R_2$ and $R_3$ in the 2-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**12.** A compound of formula I according to claim 4, in which X is fluorine, chlorine, bromine or iodine, $Q_2$ is pyrimidin-2-yl and $R_1$, $R_2$ and $R_3$ in the 4-, 5- and 6-positions are hydrogen, halogen, $C_1$-$C_4$alkyl, $C_1$-$C_2$haloalkyl having from 1 to 3 halogen atoms, $C_1$-$C_3$alkoxy, $C_1$-$C_4$thioalkyl, $C_1$-$C_2$haloalkoxy having from 1 to 3 halogen atoms, $C_3$-$C_4$alkenyloxy, $C_3$-$C_4$alkynyloxy, $C_3$-$C_5$cycloalkyl, phenoxy, thiophenyl, thiobenzyl; and furthermore $N(R_5)R_6$ in which $R_5$ and $R_6$, independently of one another, are each $C_1$-$C_2$alkyl; as well as piperidinyl or morpholinyl, or morpholinyl substituted by $C_1$-$C_3$alkyl or by chlorine.

**13.** A compound of formula I according to claim 12, in which X is fluorine, chlorine or bromine, $Q_2$ is pyrimidin-2-yl and $R_1$, $R_2$ and $R_3$ in the 4-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**14.** A compound of formula I according to claim 13, in which X is chlorine, $Q_2$ is pyrimidin-2-yl and $R_1$, $R_2$ and $R_3$ in the 4-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**15.** A compound of formula I according to claim 5, in which X is fluorine, chlorine, bromine or iodine, $Q_3$ is pyrimidin-5-yl and $R_1$, $R_2$ and $R_3$ are hydrogen, halogen, $C_1$-$C_4$alkyl, $C_1$-$C_2$haloalkyl having from 1 to 3 halogen atoms, $C_1$-$C_5$alkoxy, $C_2$-$C_4$alkoxyalkyl, $C_1$-$C_4$thioalkyl, $C_1$-$C_3$haloalkoxy having from 1 to 4 halogen atoms, $C_3$-$C_4$alkenyloxy, $C_3$-$C_4$alkynyloxy, $C_3$-$C_5$cycloalkyl, phenoxy, thiophenyl or benzyloxy, or phenoxy, thiophenyl or benzyloxy each substituted by halogen; and furthermore $N(R_5)R_6$ in which $R_5$ and $R_6$, independently of one another, are each $C_1$-$C_2$alkyl; as well as pyrrolidinyl or morpholinyl.

**16.** A compound of formula I according to claim 15, in which X is fluorine, chlorine or bromine, $Q_3$ is pyrimidin-5-yl and $R_1$, $R_2$ and $R_3$ in the 2-, 4- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**17.** A compound of formula I according to claim 16, in which X is chlorine, $Q_3$ is pyrimidin-5-yl and $R_1$, $R_2$ and $R_3$ in the 2-, 4- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**18.** A compound according to claim 1 from the group:
N-(2-cyclopropyl-5-ethyl-6-chloropyrimidin-4-yl)-2,6-dichloroisonicotinic acid amide;
N-(2-cyclopropyl-5-methyl-6-chloropyrimidin-4-yl)-2,6-dichloroisonicotinic acid amide;
N-(2,5-dimethyl-6-chloropyrimidin-4-yl)-2,6-dichloroisonicotinic acid amide;
N-(2,5-diethyl-6-chloropyrimidin-4-yl)-2,6-dichloroisonicotinic acid amide;
N-(2-chloro-6-thiomethylpyrimidin-4-yl)-2,6-dichloroisonicotinic acid amide;
N-(2-methoxy-5-chloropyrimidin-4-yl)-2,6-dichloroisonicotinic acid amide;
N-(2,5-dichloropyrimidin-4-yl)-2,6-dichloroisonicotinic acid amide;
N-(2,6-dimethylpyrimidin-4-yl)-2,6-dichloroisonicotinic acid amide;
N-(5-methyl-6-chloropyrimidin-4-yl)-2,6-dichloroisonicotinic acid amide;
N-(5-n-propyl-6-chloropyrimidin-4-yl)-2,6-dichloroisonicotinic acid amide;
N-(5-ethyl-6-chloropyrimidin-4-yl)-2,6-dichloroisonicotinic acid amide;
N-(2,5,6-trichloropyrimidin-4-yl)-2,6-dichloroisonicotinic acid amide;
N-(2-dimethylamino-6-methoxypyrimidin-4-yl)-2,6-dichloroisonicotinic acid amide;
N-(2-ethyl-5-methyl-6-chloropyrimidin-4-yl)-2,6-dichloroisonicotinic acid amide;
N-(2-trifluoromethoxy-5-chloropyrimidin-4-yl)-2,6-dichloroisonicotinic acid amide;
N-(2-methoxy-5-fluoropyrimidin-4-yl)-2,6-dichloroisonicotinic acid amide;
N-(2-chloro-5-fluoropyrimidin-4-yl)-2,6-dichloroisonicotinic acid amide;
N-(2-isopropoxy-5-chloropyrimidin-4-yl)-2,6-dichloroisonicotinic acid amide;
N-(2-isopropyl-6-chloropyrimidin-4-yl)-2,6-dichloroisonicotinic acid amide;
N-(2-chloro-6-n-propylpyrimidin-4-yl)-2,6-dichloroisonicotinic acid amide;
N-(2-n-propoxy-5-chloropyrimidin-4-yl)-2,6-dichloroisonicotinic acid amide;
N-(2-isopropyl-6-ethylthiopyrimidin-4-yl)-2,6-dichloroisonicotinic acid amide;
N-(2-isopropyl-6-isopropylthiopyrimidin-4-yl)-2,6-dichloroisonicotinic acid amide;
N-(2-chloro-6-methylpyrimidin-4-yl)-2,6-dichloroisonicotinic acid amide;
N-pyridin-2-yl-2,6-dichloroisonicotinic acid amide;
N-(6-methylpyridin-2-yl)-2,6-dichloroisonicotinic acid amide;
N-(4-methylpyridin-2-yl)-2,6-dichloroisonicotinic acid amide;
N-(5-chloropyridin-2-yl)-2,6-dichloroisonicotinic acid amide;
N-pyridin-4-yl-2,6-dichloroisonicotinic acid amide.

**19.** A compound of formula V

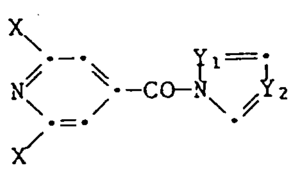

(V)

in which
X in ortho/ortho'-position are identical and represent fluorine, chlorine, bromine or iodine atoms and $Y_1$ and $Y_2$, independently of one another, are each N or CH.

**20.** A process for the preparation of a compound of formula I according to claim 1 which comprises reacting:

a) an isonicotinic acid halide of formula II

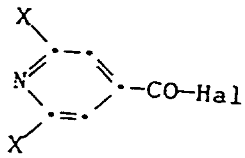

(II)

with an amine of formula III

$H_2N\text{-}Q_{1-6}$ (III)

in the presence of a base in an inert solvent or without a solvent; or
b) an isonicotinic acid anhydride of formula IV

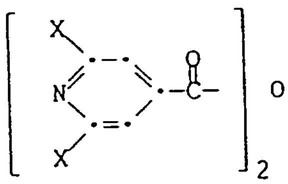

(IV)

with an amine of formula III

$H_2N\text{-}Q_{1-6}$ (III)

in the presence of a base in an inert solvent or without a solvent; or
c) an isonicotinic acid azolide of formula V

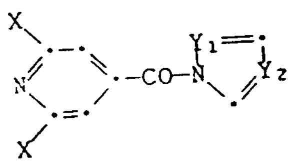

(V)

with an amine of formula III

$H_2N\text{-}Q_{1-6}$ (III)

in an inert solvent with the separation of an azole; wherein X and $Q_{1-6}$ are as defined for formula I, $Y_1$ and $Y_2$, independently of one another, are each N or CH with $Y_2$ preferably being N, and Hal is halogen, preferably chlorine.

**21.** A composition for protecting plants against attack by microorganisms that comprises as active component at least one compound of formula I according to any one of claims 1 to 18 together with customary carriers and adjuvants.

**22.** The use of a compound of formula I according to any one of claims 1 to 18 for protecting plants against attack by phytopathogenic microorganisms.

**23.** A method of protecting plants against attack by phytopathogenic microorganisms which comprises applying as active ingredient to the plant or the locus thereof a compound of formula I according to any

one of claims 1 to 18.

**24.** A method of protecting plants against attack by phytopathogenic microorganisms which comprises applying as active ingredient to the plants or the locus thereof a compound of formula V according to claim 19.

**Claims for the following Contracting State : ES**

**1.** A process for the preparation of a compound of formula I

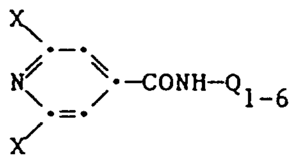

(I)

in which
X in ortho/ortho'-position are identical and represent fluorine, chlorine, bromine or iodine atoms;
$Q_1$ is unsubstituted pyrimidin-4-yl, or pyrimidin-4-yl substituted by $R_1$, $R_2$ and $R_3$;
$Q_2$ is unsubstituted pyrimidin-2-yl, or pyrimidin-2-yl substituted by $R_1$, $R_2$ and $R_3$;
$Q_3$ is unsubstituted pyrimidin-5-yl, or pyrimidin-5-yl substituted by $R_1$, $R_2$ and $R_3$;
$Q_4$ is pyridin-2-yl, pyridin-3-yl or pyridin-4-yl each of which is unsubstituted or substituted by $R_1$, $R_2$ and $R_3$;
$Q_5$ is pyridazin-3-yl or pyridazin-4-yl each of which is unsubstituted or substituted by $R_1$, $R_2$ and $R_3$;
$Q_6$ is unsubstituted pyrazin-2-yl, or pyrazin-2-yl substituted by $R_1$, $R_2$ and $R_3$;
$R_1$, $R_2$, $R_3$ are hydrogen, halogen, $C_1$-$C_6$alkyl, $C_1$-$C_6$haloalkyl having from 1 to 3 halogen atoms, $C_1$-$C_6$alkoxy, $C_2$-$C_6$alkoxyalkyl, $C_1$-$C_6$thioalkyl, $C_1$-$C_6$haloalkoxy having from 1 to 5 halogen atoms, $C_3$-$C_6$alkenyloxy, $C_3$-$C_6$alkynyloxy, $C_3$-$C_6$thioalkenyl, $C_3$-$C_6$cycloalkyl, $C_3$-$C_6$cycloalkyl substituted by methyl, or nitro, cyano, the radical $CH(OR_4)_2$ or $COOR_4$ in which $R_4$ is $C_1$-$C_4$alkyl; $R_1$, $R_2$, $R_3$ are also phenyl, phenoxy, thiophenyl, benzyl, benzyloxy or thiobenzyl; or phenyl, phenoxy, thiophenyl, benzyl, benzyloxy or thiobenzyl each substituted at least once by $C_1$-$C_3$alkyl, $C_1$-$C_3$alkoxy, $C_1$-$C_3$haloalkyl having from 1 to 3 halogen atoms, halogen, nitro or by cyano; and $R_1$, $R_2$, $R_3$ are also $N(R_5)R_6$ in which $R_5$ and $R_6$, independently of one another, are each $C_1$-$C_6$alkyl; and furthermore piperidinyl, pyrrolidinyl, morpholinyl, imidazolyl or triazolyl, or piperidinyl, pyrrolidinyl, morpholinyl, imidazolyl or triazolyl each substituted by $C_1$-$C_3$alkyl, $C_1$-$C_3$alkoxy, $C_1$-$C_3$haloalkyl having from 1 to 3 halogen atoms, halogen, nitro or by cyano, which comprises reacting:

a) an isonicotinic acid halide of formula II

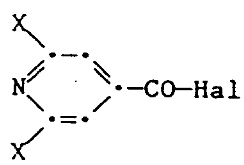

(II)

with an amine of formula III

$H_2N$-$Q_{1-6}$ (III)

in the presence of a base in an inert solvent or without a solvent; or

b) an isonicotinic acid anhydride of formula IV

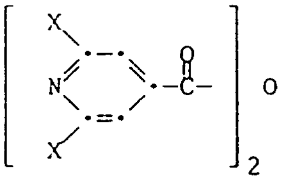

(IV)

with an amine of formula III

$H_2N\text{-}Q_{1-6}$ (III)

in the presence of a base in an inert solvent or without a solvent; or

c) an isonicotinic acid azolide of formula V

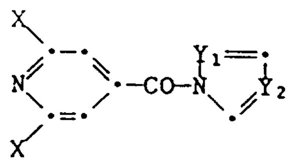

(V)

with an amine of formula III

$H_2N\text{-}Q_{1-6}$ (III)

in an inert solvent with the separation of an azole;

wherein X and $Q_{1-6}$ are as defined for formula I, $Y_1$ and $Y_2$, independently of one another, are each N or CH with $Y_2$ preferably being N, and Hal is halogen, preferably chlorine.

**2.** A process according to claim 1, wherein a compound of formula I

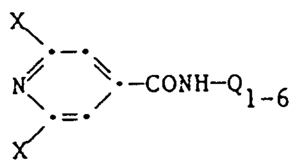

(I)

is prepared in which

X in ortho/ortho'-position are identical and represent fluorine, chlorine, bromine or iodine atoms;

$Q_1$ is unsubstituted pyrimidin-4-yl, or pyrimidin-4-yl substituted by $R_1$, $R_2$ and $R_3$;

$Q_2$ is unsubstituted pyrimidin-2-yl, or pyrimidin-2-yl substituted by $R_1$, $R_2$ and $R_3$;

$Q_3$ is unsubstituted pyrimidin-5-yl, or pyrimidin-5-yl substituted by $R_1$, $R_2$ and $R_3$;

$Q_4$ is pyridin-2-yl, pyridin-3-yl or pyridin-4-yl each of which is unsubstituted or substituted by $R_1$, $R_2$ and $R_3$;

$Q_5$ is pyridazin-3-yl or pyridazin-4-yl each of which is unsubstituted or substituted by $R_1$, $R_2$ and $R_3$;

$Q_6$ is unsubstituted pyrazin-2-yl, or pyrazin-2-yl substituted by $R_1$, $R_2$ and $R_3$;

$R_1$, $R_2$, $R_3$ are hydrogen, halogen, $C_1$-$C_6$alkyl, $C_1$-$C_6$haloalkyl having from 1 to 3 halogen atoms, $C_1$-$C_6$alkoxy, $C_2$-$C_6$alkoxyalkyl, $C_1$-$C_6$thioalkyl, $C_1$-$C_6$haloalkoxy having from 1 to 5 halogen atoms, $C_3$-$C_6$alkenyloxy, $C_3$-$C_6$alkynyloxy, $C_3$-$C_6$thioalkenyl, $C_3$-$C_6$cycloalkyl, nitro, cyano or $COOR_4$ in which $R_4$ is $C_1$-$C_4$alkyl; also phenyl, phenoxy, thiophenyl, benzyl, benzyloxy or thiobenzyl; or phenyl, phenoxy, thiophenyl, benzyl, benzyloxy or thiobenzyl each substituted at least once by $C_1$-$C_3$alkyl, $C_1$-$C_3$alkoxy, $C_1$-$C_3$haloalkyl having from 1 to 3 halogen atoms, halogen, nitro or by cyano; and also $N(R_5)R_6$ in which $R_5$ and $R_6$, independently of one another, are each $C_1$-$C_6$alkyl; and furthermore piperidinyl,

pyrrolidinyl, morpholinyl, imidazolyl or triazolyl, or piperidinyl, pyrrolidinyl, morpholinyl, imidazolyl or triazolyl each substituted by $C_1$-$C_3$alkyl, $C_1$-$C_3$alkoxy, $C_1$-$C_3$haloalkyl having from 1 to 3 halogen atoms, halogen, nitro or by cyano.

**3.** A process according to claim 1, wherein a compound of formula I is prepared in which $Q_1$ is pyrimidin-4-yl substituted by $R_1$, $R_2$ and $R_3$, and X, $R_1$, $R_2$ and $R_3$ are as defined for formula I.

**4.** A process according to claim 1, wherein a compound of formula I is prepared in which $Q_2$ is pyrimidin-2-yl substituted by $R_1$, $R_2$ and $R_3$, and X, $R_1$, $R_2$ and $R_3$ are as defined for formula I.

**5.** A process according to claim 1, wherein a compound of formula I is prepared in which $Q_3$ is pyrimidin-5-yl substituted by $R_1$, $R_2$ and $R_3$, and X, $R_1$, $R_2$ and $R_3$ are as defined for formula I.

**6.** A process according to claim 1, wherein a compound of formula I is prepared in which $Q_4$ is pyridin-2-yl, pyridin-3-yl or pyridin-4-yl each substituted by $R_1$, $R_2$ and $R_3$, and X, $R_1$, $R_2$ and $R_3$ are as defined for formula I.

**7.** A process according to claim 1, wherein a compound of formula I is prepared in which $Q_5$ is pyridazin-3-yl or pyridazin-4-yl each substituted by $R_1$, $R_2$ and $R_3$, and X, $R_1$, $R_2$ and $R_3$ are as defined for formula I.

**8.** A process according to claim 1, wherein a compound of formula I is prepared in which $Q_6$ is pyrazin-2-yl substituted by $R_1$, $R_2$ and $R_3$, and X, $R_1$, $R_2$ and $R_3$ are as defined for formula I.

**9.** A process according to claim 1, wherein a compound of formula I is prepared in which X in ortho/ortho'-position are identical and represent fluorine, chlorine, bromine or iodine atoms, $Q_1$ is pyrimidin-4-yl and $R_1$, $R_2$ and $R_3$ as substituents in the 2-, 5- and 6-positions are hydrogen, halogen, $C_1$-$C_6$alkyl, $C_1$-$C_3$haloalkyl having from 1 to 3 halogen atoms, $C_1$-$C_4$alkoxy, $C_2$-$C_4$alkoxyalkyl, $C_1$-$C_4$thioalkyl, $C_1$-$C_4$haloalkoxy having from 1 to 3 halogen atoms, $C_3$-$C_4$alkenyloxy, $C_3$-$C_4$alkynyloxy, $C_3$-$C_4$thioalkenyl, $C_3$-$C_5$cycloalkyl, nitro, cyano or $COOR_4$ in which $R_4$ is $C_1$-$C_4$alkyl, and also phenyl, phenoxy, thiophenyl or benzyl, or phenyl or phenoxy each substituted by from 1 to 3 halogen atoms; and furthermore $N(R_5)R_6$ in which $R_5$ and $R_6$, independently of one another, are each $C_1$-$C_4$alkyl; as well as piperidinyl, pyrrolidinyl, morpholinyl, imidazolyl or triazolyl, or piperidinyl, pyrrolidinyl, morpholinyl, imidazolyl or triazolyl each substituted by $C_1$-$C_3$alkyl or by halogen.

**10.** A process according to claim 1, wherein a compound of formula I is prepared in which X is fluorine, chlorine or bromine, $Q_1$ is pyrimidin-4-yl and $R_1$, $R_2$ and $R_3$ in the 2-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, thioallylene, cyclopropyl, nitro, cyano or $COOR_4$ in which $R_4$ is $C_1$-$C_4$alkyl; and also phenyl, phenoxy, thiophenyl, benzyl, dimethylamino, piperidinyl, pyrrolidinyl, morpholinyl, imidazolyl or 1,2,4-triazolyl.

**11.** A process according to claim 1, wherein a compound of formula I is prepared in which X is chlorine, $Q_1$ is pyrimidin-4-yl and $R_1$, $R_2$ and $R_3$ in the 2-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$-alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**12.** A process according to claim 1, wherein a compound of formula I is prepared in which X is fluorine, chlorine, bromine or iodine, $Q_2$ is pyrimidin-2-yl and $R_1$, $R_2$ and $R_3$ in the 4-, 5- and 6-positions are hydrogen, halogen, $C_1$-$C_4$alkyl, $C_1$-$C_2$haloalkyl having from 1 to 3 halogen atoms, $C_1$-$C_3$alkoxy, $C_1$-$C_4$thioalkyl, $C_1$-$C_2$haloalkoxy having from 1 to 3 halogen atoms, $C_3$-$C_4$alkenyloxy, $C_3$-$C_4$alkynyloxy, $C_3$-$C_5$cycloalkyl, phenoxy, thiophenyl, thiobenzyl; and furthermore $N(R_5)R_6$ in which $R_5$ and $R_6$, independently of one another, are each $C_1$-$C_2$alkyl; as well as piperidinyl or morpholinyl, or morpholinyl substituted by $C_1$-$C_3$alkyl or by chlorine.

**13.** A process according to claim 1, wherein a compound of formula I is prepared in which X is fluorine, chlorine or bromine, $Q_2$ is pyrimidin-2-yl and $R_1$, $R_2$ and $R_3$ in the 4-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**14.** A process according to claim 1, wherein a compound of formula I is prepared in which X is chlorine, $Q_2$ is pyrimidin-2-yl and $R_1$, $R_2$ and $R_3$ in the 4-, 5- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**15.** A process according to claim 1, wherein a compound of formula I is prepared in which X is fluorine, chlorine, bromine or iodine, $Q_3$ is pyrimidin-5-yl and $R_1$, $R_2$ and $R_3$ are hydrogen, halogen, $C_1$-$C_4$alkyl, $C_1$-$C_2$haloalkyl having from 1 to 3 halogen atoms, $C_1$-$C_5$alkoxy, $C_2$-$C_4$alkoxyalkyl, $C_1$-$C_4$thioalkyl, $C_1$-$C_3$haloalkoxy having from 1 to 4 halogen atoms, $C_3$-$C_4$alkenyloxy, $C_3$-$C_4$alkynyloxy, $C_3$-$C_5$cycloalkyl, phenoxy, thiophenyl or benzyloxy, or phenoxy, thiophenyl or benzyloxy each substituted by halogen; and furthermore $N(R_5)R_6$ in which $R_5$ and $R_6$, independently of one another, are each $C_1$-$C_2$alkyl; as well as pyrrolidinyl or morpholinyl.

**16.** A process according to claim 1, wherein a compound of formula I is prepared in which X is fluorine, chlorine or bromine, $Q_3$ is pyrimidin-5-yl and $R_1$, $R_2$ and $R_3$ in the 2-, 4- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**17.** A process according to claim 1, wherein a compound of formula I is prepared in which X is chlorine, $Q_3$ is pyrimidin-5-yl and $R_1$, $R_2$ and $R_3$ in the 2-, 4- and 6-positions are hydrogen, fluorine, chlorine, bromine, $C_1$-$C_4$alkyl, $C_1$-$C_2$haloalkyl having from 1 to 3 fluorine or chlorine atoms, $C_1$-$C_3$alkoxy, $C_2$-$C_4$alkoxyalkyl, $C_1$-$C_4$thioalkyl, $C_1$-$C_2$haloalkoxy having from 1 to 3 fluorine or chlorine atoms, $C_3$-$C_4$alkenyloxy, $C_3$-$C_4$alkynyloxy, cyclopropyl, nitro, cyano or $COOR_4$ in which $R_4$ is $C_1$-$C_4$alkyl; and also phenyl, phenoxy, thiophenyl, benzyl, dimethylamino.

**18.** A process according to claim 1 wherein there is prepared a compound from the group:
N-(2-cyclopropyl-5-ethyl-6-chloropyrimidin-4-yl)-2,6-dichloroisonicotinic acid amide;
N-(2-cyclopropyl-5-methyl-6-chloropyrimidin-4-yl)-2,6-dichloroisonicotinic acid amide;
N-(2,5-dimethyl-6-chloropyrimidin-4-yl)-2,6-dichloroisonicotinic acid amide;
N-(2,5-diethyl-6-chloropyrimidin-4-yl)-2,6-dichloroisonicotinic acid amide;
N-(2-chloro-6-thiomethylpyrimidin-4-yl)-2,6-dichloroisonicotinic acid amide;
N-(2-methoxy-5-chloropyrimidin-4-yl)-2,6-dichloroisonicotinic acid amide;
N-(2,5-dichloropyrimidin-4-yl)-2,6-dichloroisonicotinic acid amide;
N-(2,6-dimethylpyrimidin-4-yl)-2,6-dichloroisonicotinic acid amide;
N-(5-methyl-6-chloropyrimidin-4-yl)-2,6-dichloroisonicotinic acid amide;
N-(5-n-propyl-6-chloropyrimidin-4-yl)-2,6-dichloroisonicotinic acid amide;
N-(5-ethyl-6-chloropyrimidin-4-yl)-2,6-dichloroisonicotinic acid amide;
N-(2,5,6-trichloropyrimidin-4-yl)-2,6-dichloroisonicotinic acid amide;
N-(2-dimethylamino-6-methoxypyrimidin-4-yl)-2,6-dichloroisonicotinic acid amide;
N-(2-ethyl-5-methyl-6-chloropyrimidin-4-yl)-2,6-dichloroisonicotinic acid amide;
N-(2-trifluoromethoxy-5-chloropyrimidin-4-yl)-2,6-dichloroisonicotinic acid amide;
N-(2-methoxy-5-fluoropyrimidin-4-yl)-2,6-dichloroisonicotinic acid amide;
N-(2-chloro-5-fluoropyrimidin-4-yl)-2,6-dichloroisonicotinic acid amide;
N-(2-isopropoxy-5-chloropyrimidin-4-yl)-2,6-dichloroisonicotinic acid amide;
N-(2-isopropyl-6-chloropyrimidin-4-yl)-2,6-dichloroisonicotinic acid amide;
N-(2-chloro-6-n-propylpyrimidin-4-yl)-2,6-dichloroisonicotinic acid amide;
N-(2-n-propoxy-5-chloropyrimidin-4-yl)-2,6-dichloroisonicotinic acid amide;

N-(2-isopropyl-6-ethylthiopyrimidin-4-yl)-2,6-dichloroisonicotinic acid amide;
N-(2-isopropyl-6-isopropylthiopyrimidin-4-yl)-2,6-dichloroisonicotinic acid amide;
N-(2-chloro-6-methylpyrimidin-4-yl)-2,6-dichloroisonicotinic acid amide;
N-pyridin-2-yl-2,6-dichloroisonicotinic acid amide;
N-(6-methylpyridin-2-yl)-2,6-dichloroisonicotinic acid amide;
N-(4-methylpyridin-2-yl)-2,6-dichloroisonicotinic acid amide;
N-(5-chloropyridin-2-yl)-2,6-dichloroisonicotinic acid amide;
N-pyridin-4-yl-2,6-dichloroisonicotinic acid amide.

**19.** A process for the preparation of a compound of formula V

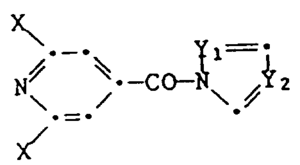

(V)

which is carried out by reacting a compound of formula II

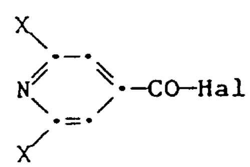

(II)

with an azole of formula VI

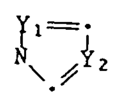

(VI)

in the presence of a base in an inert solvent, wherein X is as defined for formula I in claim 1, Hal is halogen, preferably chlorine, and $Y_1$ and $Y_2$, independently of one another, are each N or CH.

**Revendications**

**Revendications pour les Etats contractants suivants: AT, BE, CH, DE, FR, GB, IT, LI, NL**

**1.** Composés de formule I

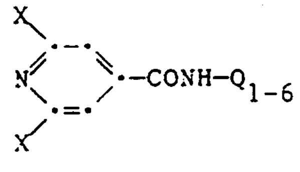

(I),

dans laquelle
X désigne des atomes identiques en position ortho/ortho' de fluor, chlore, brome ou iode;
$Q_1$ représente un radical pyrimidine-4-yle non substitué ou substitué par un groupe $R_1$, $R_2$ et $R_3$;
$Q_2$ représente un radical pyrimidine-2-yle non substitué ou substitué par un groupe $R_1$, $R_2$ et $R_3$;
$Q_3$ représente un radical pyrimidine-5-yle non substitué ou substitué par un groupe $R_1$, $R_2$ et $R_3$;
$Q_4$ représente un radical pyridine-2-yle, pyridine-3-yle ou pyridine-4-yle non substitué ou substitué par un groupe $R_1$, $R_2$ et $R_3$;
$Q_5$ représente un radical pyridazine-3-yle ou pyridazine-4-yle non substitué ou substitué par un groupe

$R_1$, $R_2$ et $R_3$;
$Q_6$ représente un radical pyrazine-2-yle non substitué ou substitué par un groupe $R_1$, $R_2$ et $R_3$;
$R_1$, $R_2$, $R_3$ représente l'hydrogène ou un groupe halogéno, alkyle en $C_1$-$C_6$, halogénoalkyle en $C_1$-$C_6$ ayant 1 à 3 atomes d'halogène, alcoxy en $C_1$-$C_6$, alcoxyalkyle en $C_2$-$C_6$, thioalkyle en $C_1$-$C_6$, halogénoalcoxy en $C_1$-$C_6$ ayant 1 à 5 atomes d'halogène, alcényloxy en $C_3$-$C_6$, alcynyloxy en $C_3$-$C_6$, thioalcényle en $C_3$-$C_6$, cycloalkyle en $C_3$-$C_6$, cycloalkyle en $C_3$-$C_6$ substitué par un radical méthyle, nitro, cyano, et les restes $CH(OR_4)_2$ ou $COOR_4$, où $R_4$ représente un radical alkyle en $C_1$-$C_4$; ou en outre un radical phényle, phénoxy, thiophényle, benzyle, benzyloxy ou thiobenzyle; ou un radical phényle, phénoxy, thiophényle, benzyle, benzyloxy ou thiobenzyle substitué au moins une fois par un radical alkyle en $C_1$-$C_3$, alcoxy en $C_1$-$C_3$, halogénoalkyle en $C_1$-$C_3$ avant 1 à 3 atomes d'halogène, halogéno, nitro ou cyano; ainsi que $N(R_5)R_6$, où $R_5$ et $R_6$ représentent, chacun indépendamment l'un de l'autre, un radical alkyle en $C_1$-$C_6$; ou en outre un radical pipéridinyle, pyrrolidinyle, morpholinyle, imidazolyle ou triazolyle, ou un radical pipéridinyle, pyrrolidinyle, morpholinyle, imidazolyle ou triazolyle substitué par un radical alkyle en $C_1$-$C_3$, alcoxy en $C_1$-$C_3$, halogénoalkyle en $C_1$-$C_3$ ayant 1 à 3 atomes d'halogène, halogéno, nitro ou cyano.

**2.** Composés de formule I selon la revendication 1,

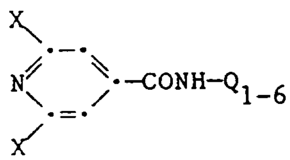

(I),

dans laquelle
X désigne des atomes identiques en position ortho/ortho' de fluor, chlore, brome ou iode;
$Q_1$ représente un radical pyrimidine-4-yle non-substitué ou substitué par des groupes $R_1$, $R_2$ et $R_3$;
$Q_2$ représente un un radical pyrimidine-2-yle non-substitué ou substitué par des groupes $R_1$, $R_2$ et $R_3$;
$Q_3$ représente un un radical pyrimidine-5-yle non-substitué ou substitué par des groupes $R_1$, $R_2$ et $R_3$;
$Q_4$ représente un un radical pyridine-2-yle, pyridine-3-yle ou pyridine-4-yle non-substitué ou substitué par des groupes $R_1$, $R_2$ et $R_3$;
$Q_5$ représente un un radical pyridazine-3-yle ou pyridazine-4-yle non-substitué ou substitué par des groupes $R_1$, $R_2$ et $R_3$;
$Q_6$ représente un un radical pyrazine-2-yle non-substitué ou substitué par des groupes $R_1$, $R_2$ et $R_3$;
$R_1$, $R_2$, $R_3$ représente l'hydrogène ou un groupe halogéno, alkyle en $C_1$-$C_6$, halogénoalkyle en $C_1$-$C_6$ avant 1 à 3 atomes d'halogène, alcoxy en $C_1$-$C_6$, alcoxyalkyle en $C_2$-$C_6$, thioalkyle en $C_1$-$C_6$, halogénoalcoxy en $C_1$-$C_6$ ayant 1 à 5 atomes d'halogène, alcényloxy en $C_3$-$C_6$, alcynyloxy en $C_3$-$C_6$, thioalcényle en $C_3$-$C_6$, cycloalkyle en $C_3$-$C_6$, nitro, cyano ou $COOR_4$, où $R_4$ représente un radical alkyle en $C_1$-$C_4$; ainsi qu'un radical phényle, phénoxy, thiophényle, benzyle, benzyloxy ou thiobenzyle; ou un radical phényle, phénoxy, thiophényle, benzyle, benzyloxy ou thiobenzyle substitué au moins une fois par un radical alkyle en $C_1$-$C_3$,alcoxy en $C_1$-$C_3$, halogénoalkyle en $C_1$-$C_3$ avant 1 à 3 atomes d'halogène, halogéno, nitro ou cyano; ainsi que $N(R_5)R_6$, où $R_5$ et $R_6$ représentent, chacun indépendamment l'un de l'autre, un radical alkyle en $C_1$-$C_6$; ou en outre un radical pipéridinyle, pyrrolidinyle, morpholinyle, imidazolyle ou triazolyle, ou un radical pipéridinyle, pyrrolidinyle, morpholinyle, imidazolyle ou triazolyle substitué par un radical alkyle en $C_1$-$C_3$, alcoxy en $C_1$-$C_3$, halogénoalkyle en $C_1$-$C_3$ avant 1 à 3 atomes d'halogène, halogéno, nitro ou cyano.

**3.** Composés de formule I selon la revendication 2, dans lesquels $Q_1$ représente un groupe pyrimidine-4-yle substitué par $R_1$, $R_2$ et $R_3$ et X, $R_1$, $R_2$ et $R_3$ ont les significations indiquées sous la formule I.

**4.** Composés de formule I selon la revendication 2, dans lesquels $Q_2$ représente un groupe pyrimidine-2-yle substitué par $R_1$, $R_2$ et $R_3$ et X, $R_1$, $R_2$ et $R_3$ ont les significations indiquées sous la formule I.

**5.** Composés de formule I selon la revendication 2, dans lesquels $Q_3$ représente un groupe pyrimidine-5-yle substitué par $R_1$, $R_2$ et $R_3$ et X, $R_1$, $R_2$ et $R_3$ ont les significations indiquées sous la formule I.

**6.** Composés de formule I selon la revendication 2, dans lesquels $Q_4$ représente un groupe pyridine-2-yle, pyridine-3-yle ou pyridine-4-yle substitué par $R_1$, $R_2$ et $R_3$ et X, $R_1$, $R_2$ et $R_3$ ont les significations indiquées sous la formule I.

**7.** Composés de formule I selon la revendication 2, dans lesquels $Q_5$ représente un groupe pyridazine-3-yle ou pyridazine-4-yle substitué par $R_1$, $R_2$ et $R_3$ et X, $R_1$, $R_2$ et $R_3$ ont les significations indiquées sous la formule I.

**8.** Composés de formule I selon la revendication 2, dans lesquels $Q_6$ représente un groupe pyrazine-2-yle substitué par $R_1$, $R_2$ et $R_3$ et X, $R_1$, $R_2$ et $R_3$ ont les significations indiquées sous la formule I.

**9.** Composés de formule I selon la revendication 3, dans lesquels X représente des atomes identiques de fluor, chlore, brome ou iode en position ortho/ortho', $Q_1$ désigne le radical pyrimidine-4-yle et $R_1$, $R_2$ et $R_3$ en tant que substituants en position 2, 5 ou 6 désignent l'hydrogène ou un groupe halogéno, alkyle en $C_1$-$C_6$, halogénoalkyle en $C_1$-$C_3$ ayant 1 à 3 atomes d'halogène, alcoxy en $C_1$-$C_4$, alcoxyalkyle en $C_2$-$C_4$, thioalkyle en $C_1$-$C_4$, halogénoalcoxy en $C_1$-$C_4$ ayant 1 à 3 atomes d'halogène, alcényloxy en $C_3$-$C_4$, alcynyloxy en $C_3$-$C_4$, thioalcényle en $C_3$-$C_4$, cycloalkyle en $C_3$-$C_5$, nitro, cyano ou $COOR_4$, où $R_4$ représente un radical alkyle en $C_1$-$C_4$, ainsi qu'un radical phényle, phénoxy, thiophényle, benzyle ou un radical phényle ou phénoxy substitué par 1 à 3 atomes d'halogène; ainsi que $N(R_5)R_6$, où $R_5$ et $R_6$ représentent, chacun indépendamment l'un de l'autre, un radical alkyle en $C_1$-$C_4$;ou encore un radical pipéridinyle, pyrrolidinyle, morpholinyle, imidazolyle ou triazolyle, ou un radical pipéridinyle, pyrrolidinyle, morpholinyle, imidazolyle ou triazolyle substitué par un radical alkyle en $C_1$-$C_3$ ou un halogène.

**10.** Composés de formule I selon la revendication 3, dans lesquels X représente des atomes identiques de fluor, chlore ou brome en position ortho/ortho', $Q_1$ désigne le radical pyrimidine-4-yle et $R_1$, $R_2$ et $R_3$ en position 2, 5 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxy-alkyle en $C_2$-$C_4$, thioalkyle en $C_1$-$C_4$, halogénoalcoxy en $C_1$-$C_2$ avant 1 à 3 atomes de fluor ou de chlore, alcényloxy en $C_3$-$C_4$, alcynyloxy en $C_3$-$C_4$, thioallylène, cyclopropyle, nitro, cyano ou $COOR_4$, où $R_4$ représente un radical alkyle en $C_1$-$C_4$, ainsi qu'un radical phényle, phénoxy, thiophényle, benzyle, diméthylamino, pipéridinyle, pyrrolidinyle, porpholinyle, imidazolyle ou 1,2,4-triazolyle.

**11.** Composés de formule I selon la revendication 10, dans lesquels X représente le chlore, $Q_1$ désigne le radical pyrimidine-4-yle et $R_1$, $R_2$ et $R_3$ en position 2, 5 ou 6 désignent l'hydrogène ou un radical fluoro, chloro, bromo, alkyle en $C_1$-$C_4$, halogénoalkyle en $C_1$-$C_2$ avant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$, ainsi qu'un radical phényle, phénoxy, thiophényle, benzyle ou diméthylamino.

**12.** Composés de formule I selon la revendication 4, dans lesquels X représente des atomes identiques de fluor, chlore, brome ou iode en position ortho/ortho', $Q_2$ désigne le radical pyrimidine-2-yle et $R_1$, $R_2$ et $R_3$ en position 4, 5 ou 6 désignent l'hydrogène ou un groupe halogéno, alkyle en $C_1$-$C_4$, halogénoalkyle en $C_1$-$C_2$ ayant 1 à 3 atomes d'halogène, alcoxy en $C_1$-$C_3$, thioalkyle en $C_1$-$C_4$, halogénoalcoxy en $C_1$-$C_2$ ayant 1 à 3 atomes d'halogène, alcényloxy en $C_3$-$C_4$, alcynyloxy en $C_3$-$C_4$, cycloalkyle en $C_3$-$C_5$, nitro, cyano ou $COOR_4$, où $R_4$ représente un radical alkyle en $C_1$-$C_4$; ou dans lesquels $R_1$, $R_2$ et $R_3$ désignent un radical phénoxy, thiophényle, thiobenzyle; ainsi que $N(R_5)R_6$, où $R_5$ et $R_6$ représentent, chacun indépendamment l'un de l'autre, un radical alkyle en $C_1$-$C_2$; ou encore un radical pipéridinyle ou morpholinyle ou un radical morpholinyle substitué par un radical alkyle en $C_1$-$C_3$ oule chlore.

**13.** Composés de formule I selon la revendication 12, dans lesquels X représente des atomes identiques de fluor, chlore ou brome en position ortho/ortho', $Q_2$ désigne le radical pyrimidine-2-yle et $R_1$, $R_2$ et $R_3$ en position 4, 5 ou 6 désignent l'hydrogène ou un radical fluoro, chloro ou bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$; ou encore désignent un radical phényle, phénoxy, thiophényle, benzyle ou diméthylamino.

14. Composés de formule I selon la revendication 13, dans lesquels X représente le chlore, $Q_2$ désigne le radical pyrimidine-2-yle et $R_1$, $R_2$ et $R_3$ en position 4, 5 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$; ou encore désignent un radical phényle, phénoxy, thiophényle, benzyle ou diméthylamino.

15. Composés de formule I selon la revendication 5, dans lesquels X représente des atomes identiques de fluor, chlore, brome ou iode en position ortho/ortho', $Q_3$ désigne le radical pyrimidine-5-yle et $R_1$, $R_2$ et $R_3$ désignent l'hydrogène ou un radical halogéno ou alkyle en $C_1$-$C_4$, halogénoalkyle en $C_1$-$C_2$ ayant 1 à 3 atomes d'halogène, alcoxy en $C_1$-$C_5$, alcoxyalkyle en $C_2$-$C_4$, thioalkyle en $C_1$-$C_4$, halogénoalcoxy en $C_1$-$C_3$ ayant 1 à 4 atomes d'halogène, alcényloxy en $C_3$-$C_4$, alcynyloxy en $C_3$-$C_4$, cycloalkyle en $C_3$-$C_5$, phénoxy, thiophényle ou benzyloxy ou phénoxy, thiophényle ou benzyloy substitué par des atomes d'halogène; ainsi que $N(R_5)R_6$, où $R_5$ et $R_6$ représentent, chacun indépendamment l'un de l'autre, un radical alkyle en $C_1$-$C_2$; ou encore un radical pyrrolidinyle ou morpholinyle.

16. Composés de formule I selon la revendication 15, dans lesquels X représente des atomes identiques de fluor, chlore, brome ou iode en position ortho/ortho', $Q_3$ désigne le radical pyrimidine-5-yle et $R_1$, $R_2$ et $R_3$ en position 2, 4 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$; ou encore désignent un radical phényle, phénoxy, thiophényle, benzyle ou diméthylamino.

17. Composés de formule I selon la revendication 16, dans lesquels X représente le chlore, $Q_3$ désigne le radical pyrimidine-5-yle et $R_1$, $R_2$ et $R_3$ en position 2, 4 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclo-propyle, nitro, cyano ou $COOR_4$, où $R_4$ représente un radical alkyle en $C_1$-$C_4$; ou encore désignent un radical phényle, phénoxy, thiophényle, benzyle ou diméthylamino.

18. Composé selon la revendication 1, choisi dans le groupe des composés suivants:
amide d'acide N-(2-cyclopropyl-5-éthyl-6-chloropyrimidine-4-yl)-2,6-dichloro-isonicotinique;
amide d'acide N-(2-cyclopropyl-5-méthyl-6-chloropyrimidine-4-yl)-2,6-dichloro-isonicotinique;
amide d'acide N-(2,5-diméthyl-6-chloro-pyrimidine-4-yl)-2,6-dichloro-isonicotinique;
amide d'acide N-(2,5-diéthyl-6-chloro-pyrimidine-4-yl)-2,6-dichloro-isonicotinique;
amide d'acide N-(2-chloro-6-thiométhyl-pyrimidine-4-yl)-2,6-dichloro-isonicotinique;
amide d'acide N-(2-méthoxy-5-chloro-pyrimidine-4-yl)-2,6-dichloro-isonicotinique;
amide d'acide N-(2,5-dichloro-pyrimidine-4-yl)-2,6-dichloro-isonicotinique;
amide d'acide N-(2,6-diméthyl-pyrimidine-4-yl)-2,6-dichloro-isonicotinique;
amide d'acide N-(5-méthyl-6-chloro-pyrimidine-4-yl)-2,6-dichloro-isonicotinique;
amide d'acide N-(5-n-propyl-6-chloro-pyrimidine-4-yl)-2,6-dichloro-isonicotinique;
amide d'acide N-(5-éthyl-6-chloro-pyrimidine-4-yl)-2,6-dichloro-isonicotinique;
amide d'acide N-(2,5,6-trichloro-pyrimidine-4-yl)-2,6-dichloro-isonicotinique;
amide d'acide N-(2-diméthylamino-6-méthoxy-pyrimidine-4-yl)-2,6-dichloro-isonicotinique;
amide d'acide N-(2-éthyl-5-méthyl-6-chloro-pyrimidine-4-yl)-2,6-dichloro-isonicotinique;
amide d'acide N-(2-trifluorométhoxy-5-chloro-pyrimidine-4-yl)-2,6-dichloro-isonicotinique;
amide d'acide N-(2-méthoxy-5-fluoro-pyrimidine-4-yl)-2,6-dichloro-isonicotinique;
amide d'acide N-(2-chloro-5-fluoro-pyrimidine-4-yl)-2,6-dichloro-isonicotinique;
amide d'acide N-(2-isopropoxy-5-chloro-pyrimidine-4-yl)-2,6-dichloro-isonicotinique;
amide d'acide N-(2-isopropyl-6-chloro-pyrimidine-4-yl)-2,6-dichloro-isonicotinique;
amide d'acide N-(2-chloro-6-n-propyl-pyrimidine-4-yl)-2,6-dichloro-isonicotinique;
amide d'acide N-(2-n-propoxy-5-chloro-pyrimidine-4-yl)-2,6-dichloro-isonicotinique;
amide d'acide N-(2-isopropyl-6-éthylthio-pyrimidine-4-yl)-2,6-dichloro-isonicotinique;
amide d'acide N-(2-isopropyl-6-isopropylthio-pyrimidine-4-yl)-2,6-dichloro-isonicotinique;

amide d'acide N-(2-chloro-6-méthyl-pyrimidine-4-yl)-2,6-dichloro-isonicotinique;
amide d'acide N-pyridine-2-yl-2,6-dichloro-isonicotinique;
amide d'acide N-(6-méthyl-pyridine-2-yl)-2,6-dichloroisonicotinique;
amide d'acide N-(4-méthyl-pyridine-2-yl)-2,6-dichloroisonicotinique;
amide d'acide N-(5-chloro-pyridine-2-yl)-2,6-dichloroisonicotinique;
amide d'acide N-pyridine-4-yl-2,6-dichloro-isonicotinique.

**19.** Composés de formule V

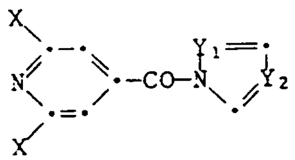

(V)

où X représente des atomes identiques de fluor, chlore, brome ou iode en position ortho/ortho' et $Y_1$ et $Y_2$ désignent, indépendamment l'un de l'autre, N ou CH.

**20.** Procédé pour la préparation de composés de formule I selon la revendication 1, caractérisé en ce qu'on fait réagir:

a) un halogénure d'acide isonicotinique de formule II

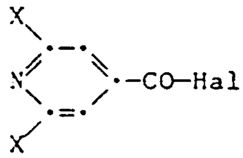

(II)

avec une amine de formule III

$H_2N—Q_{1-6}$ (III)

en présence d'une base dans un solvant inerte ou sans solvant; ou

b) un anhydride d'acide isonicotinique de formule IV

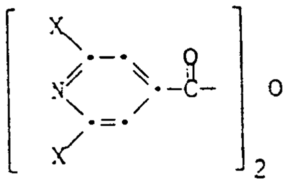

(IV)

avec une amine de formule III

$H_2N—Q_{1-6}$ (III)

en présence d'une base dans un solvant inerte ou sans solvant; ou

c) un azolide d'acide isonicotinique de formule V

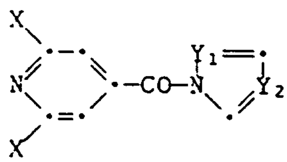

(V)

avec une amine de formule III

$H_2N—Q_{1-6}$ (III)

dans un solvant inerte, avec élimination d'un azol; tandis que X et $Q_{1-6}$ possèdent les significations indiquées sous la formule I, $Y_1$ et $Y_2$ représentent, indépendamment l'un de l'autre N ou CH, $Y_2$ étant de préférence N, et Hal désigne un halogène, de préférence le chlore.

**21.** Agent pour la protection des plantes contre l'attaque par des microorganismes, caractérisé en ce qu'il contient, outre les supports ou vecteurs et les adjuvants classiques, en tant que constituant actif au moins un composé de formule I selon l'une quelconque des revendications 1-18.

**22.** Utilisation de composés de formule I selon l'une quelconque des revendications 1 à 18 pour la protection des plantes contre l'attaque par des microorganismes phytopathogènes.

**23.** Procédé pour la protection des plantes contre l'attaque par des microorganismes phytopathogènes, caractérisé en ce qu'on applique en tant qu'agent actif un composé de formule I selon l'une quelconque des revendications 1 à 18 sur la plante ou sur le lieu où elle se trouve.

**24.** Procédé pour la protection des plantes contre l'attaque par des microorganismes phytopathogènes, caractérisé en ce qu'on applique en tant qu'agent actif un composé de formule V selon la revendication 19 sur les plantes ou sur le lieu où elles se trouvent.

**Revendications pour l'Etat contractant suivant : ES**

**1.** Procédé pour la préparation de composés de formule I

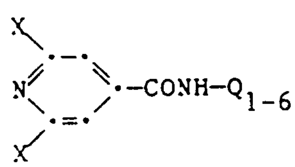

(I),

X désigne des atomes identiques en position ortho/ortho' de fluor, chlore, brome ou iode;
$Q_1$ représente un radical pyrimidine-4-yle non substitué ou substitué par un groupe $R_1$, $R_2$ et $R_3$;
$Q_2$ représente un radical pyrimidine-2-yle non substitué ou substitué par un groupe $R_1$, $R_2$ et $R_3$;
$Q_3$ représente un radical pyrimidine-5-yle non substitué ou substitué par un groupe $R_1$, $R_2$ et $R_3$;
$Q_4$ représente un radical pyridine-2-yle, pyridine-3-yle ou pyridine-4-yle non substitué ou substitué par un groupe $R_1$, $R_2$ et $R_3$;
$Q_5$ représente un radical pyridazine-3-yle ou pyridazine-4-yle non substitué ou substitué par un groupe $R_1$, $R_2$ et $R_3$;
$Q_6$ représente un radical pyrazine-2-yle non substitué ou substitué par un groupe $R_1$, $R_2$ et $R_3$;
$R_1$, $R_2$, $R_3$ représente l'hydrogène ou un groupe halogéno, alkyle en $C_1$-$C_6$, halogénoalkyle en $C_1$-$C_6$ ayant 1 à 3 atomes d'halogène, alcoxy en $C_1$-$C_6$, alcoxyalkyle en $C_2$-$C_6$, thioalkyle en $C_1$-$C_6$, halogénoalcoxy en $C_1$-$C_6$ ayant 1 à 5 dans laquelle $C_6$, thioalcényle en $C_3$-$C_6$, cycloalkyle en $C_3$-$C_6$, cycloalkyle en $C_3$-$C_6$ substitué par un radical méthyle, nitro, cyano, et les restes $CH(OR_4)_2$ ou $COOR_4$, où $R_4$ représente un radical alkyle en $C_1$-$C_4$; ou en outre un radical phényle, phénoxy, thiophényle, benzyle, benzyloxy ou thiobenzyle; ou un radical phényle, phénoxy, thiophényle, benzyle, benzyloxy ou

thiobenzyle substitué au moins une fois par un radical alkyle en $C_1$-$C_3$, alcoxy en $C_1$-$C_3$, halogénoalkyle en $C_1$-$C_3$ ayant 1 à 3 atomes d'halogène, halogéno, nitro ou cyano; ainsi que $N(R_5)R_6$, où $R_5$ et $R_6$ représentent, chacun indépendamment l'un de l'autre, un radical alkyle en $C_1$-$C_6$; ou en outre un radical pipéridinyle, pyrrolidinyle, morpholinyle, imidazolyle ou triazolyle, ou un radical pipéridinyle, pyrrolidinyle, morpholinyle, imidazolyle ou triazolyle substitué par un radical alkyle en $C_1$-$C_3$, alcoxy en $C_1$-$C_3$, halogénoalkyle en $C_1$-$C_3$ ayant 1 à 3 atomes d'halogène, halogéno, nitro ou cyano, caractérisé en ce qu'on fait réagir:

a) un halogénure d'acide isonicotinique de formule II

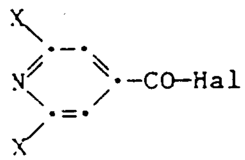

(II)

avec une amine de formule III

$H_2N—Q_{1-6}$ (III)

en présence d'une base dans un solvant inerte ou sans solvant; ou

b) un anhydride d'acide isonicotinique de formule IV

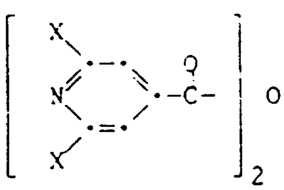

(IV)

avec une amine de formule III

$H_2N—Q_{1-6}$ (III)

en présence d'une base dans un solvant inerte ou sans solvant; ou

c) un azolide d'acide isonicotinique de formule V

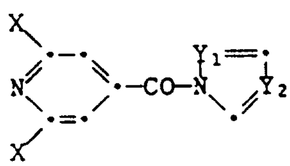

(V)

avec une amine de formule III

$H_2N—Q_{1-6}$ (III)

dans un solvant inerte, avec élimination d'un azol; tandis que X et $Q_{1-6}$ possèdent les significations indiquées sous la formule I, $Y_1$ et $Y_2$ représentent, indépendamment l'un de l'autre N ou CH, $Y_2$ étant de préférence N, et Hal désigne un halogène, de préférence le chlore.

2. Procédé selon la revendication 1, caractérisé en ce qu'on prépare des composés de formule I,

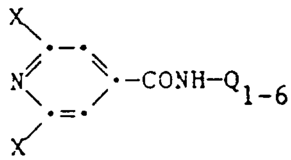

(I)

dans laquelle
X désigne des atomes identiques en position ortho/ortho' de fluor, chlore, brome ou iode;
$Q_1$ représente un radical pyrimidine-4-yle non-substitué ou substitué par des groupes $R_1$, $R_2$ et $R_3$;
$Q_2$ représente un un radical pyrimidine-2-yle non-substitué ou substitué par des groupes $R_1$, $R_2$ et $R_3$;
$Q_3$ représente un un radical pyrimidine-5-yle non-substitué ou substitué par des groupes $R_1$, $R_2$ et $R_3$;
$Q_4$ représente un un radical pyridine-2-yle, pyridine-3-yle ou pyridine-4-yle non-substitué ou substitué par des groupes $R_1$, $R_2$ et $R_3$;
$Q_5$ représente un un radical pyridazine-3-yle ou pyridazine-4-yle non-substitué ou substitué par des groupes $R_1$, $R_2$ et $R_3$;
$Q_6$ représente un un radical pyrazine-2-yle non-substitué ou substitué par des groupes $R_1$, $R_2$ et $R_3$;
$R_1$, $R_2$, $R_3$ représente l'hydrogène ou un groupe halogéno, alkyle en $C_1$-$C_6$, halogénoalkyle en $C_1$-$C_6$ ayant 1 à 3 atomes d'halogène, alcoxy en $C_1$-$C_6$, alcoxyalkyle en $C_2$-$C_6$, thioalkyle en $C_1$-$C_6$, halogénoalcoxy en $C_1$-$C_6$ ayant 1 à 5 atomes d'halogène, alcényloxy en $C_3$-$C_6$, alcynyloxy en $C_3$-$C_6$, thioalcényle en $C_3$-$C_6$, cycloalkyle en $C_3$-$C_6$, nitro, cyano ou $COOR_4$, où $R_4$ représente un radical alkyle en $C_1$-$C_4$; ainsi qu'un radical phényle, phénoxy, thiophényle, benzyle, benzyloxy ou thiobenzyle; ou un radical phényle, phénoxy, thiophényle, benzyle, benzyloxy ou thiobenzyle substitué au moins une fois par un radical alkyle en $C_1$-$C_3$,alcoxy en $C_1$-$C_3$, halogénoalkyle en $C_1$-$C_3$ ayant 1 à 3 atomes d'halogène, halogéno, nitro ou cyano; ainsi que $N(R_5)R_6$, où $R_5$ et $R_6$ représentent, chacun indépendamment l'un de l'autre, un radical alkyle en $C_1$-$C_6$; ou en outre un radical pipéridinyle, pyrrolidinyle, morpholinyle, imidazolyle ou triazolyle, ou un radical pipéridinyle, pyrrolidinyle, morpholinyle, imidazolyle ou triazolyle substitué par un radical alkyle en $C_1$-$C_3$, alcoxy en $C_1$-$C_3$, halogénoalkyle en $C_1$-$C_3$ ayant 1 à 3 atomes d'halogène, halogéno, nitro ou cyano.

3. Procédé selon la revendication 1, caractérisé en ce qu'on prépare des composés de formule I dans lesquels $Q_1$ représente un groupe pyrimidine-4-yle substitué par $R_1$, $R_2$ et $R_3$ et X, $R_1$, $R_2$ et $R_3$ ont les significations indiquées sous la formule I.

4. Procédé selon la revendication 1, caractérisé en ce qu'on prépare des composés de formule I dans lesquels $Q_2$ représente un groupe pyrimidine-2-yle substitué par $R_1$, $R_2$ et $R_3$ et X, $R_1$, $R_2$ et $R_3$ ont les significations indiquées sous la formule I.

5. Procédé selon la revendication 1, caractérisé en ce qu'on prépare des composés de formule I dans lesquels $Q_3$ représente un groupe pyrimidine-5-yle substitué par $R_1$, $R_2$ et $R_3$ et X, $R_1$, $R_2$ et $R_3$ ont les significations indiquées sous la formule I.

6. Procédé selon la revendication 1, caractérisé en ce qu'on prépare des composés de formule I dans lesquels $Q_4$ représente un groupe pyridine-2-yle, pyridine-3-yle ou pyridine-4-yle substitué par $R_1$, $R_2$ et $R_3$ et X, $R_1$, $R_2$ et $R_3$ ont les significations indiquées sous la formule I.

7. Procédé selon la revendication 1, caractérisé en ce qu'on prépare des composés de formule I dans lesquels $Q_5$ représente un groupe pyridazine-3-yle ou pyridazine-4-yle substitué par $R_1$, $R_2$ et $R_3$ et X, $R_1$, $R_2$ et $R_3$ ont les significations indiquées sous la formule I.

8. Procédé selon la revendication 1, caractérisé en ce qu'on prépare des composés de formule I dans lesquels $Q_6$ représente un groupe pyrazine-2-yle substitué par $R_1$, $R_2$ et $R_3$ et X, $R_1$, $R_2$ et $R_3$ ont les significations indiquées sous la formule I.

9. Procédé selon la revendication 1, caractérisé en ce qu'on prépare des composés de formule I dans lesquels X représente des atomes identiques de fluor, chlore, brome ou iode en position ortho/ortho',

$Q_1$ désigne le radical pyrimidine-4-yle et $R_1$, $R_2$ et $R_3$ en tant que substituants en position 2, 5 ou 6 désignent l'hydrogène ou un groupe halogéno, alkyle en $C_1$-$C_6$, halogénoalkyle en $C_1$-$C_3$ ayant 1 à 3 atomes d'halogène, alcoxy en $C_1$-$C_4$, alcoxyalkyle en $C_2$-$C_4$, thioalkyle en $C_1$-$C_4$, halogénoalcoxy en $C_1$-$C_4$ ayant 1 à 3 atomes d'halogène, alcényloxy en $C_3$-$C_4$, alcynyloxy en $C_3$-$C_4$, thioalcényle en $C_3$-$C_4$, cycloalkyle en $C_3$-$C_5$, nitro, cyano ou $COOR_4$, où $R_4$ représente un radical alkyle en $C_1$-$C_4$, ainsi qu'un radical phényle, phénoxy, thiophényle, benzyle ou un radical phényle ou phénoxy substitué par 1 à 3 atomes d'halogène; ainsi que $N(R_5)R_6$, où $R_5$ et $R_6$ représentent, chacun indépendamment l'un de l'autre, un radical alkyle en $C_1$-$C_4$;ou encore un radical pipéridinyle, pyrrolidinyle, morpholinyle, imidazolyle ou triazolyle, ou un radical pipéridinyle, pyrrolidinyle, morpholinyle, imidazolyle ou triazolyle substitué par un radical alkyle en $C_1$-$C_3$ ou un halogène.

**10.** Procédé selon la revendication 1, caractérisé en ce qu'on prépare des composés de formule I dans lesquels X représente des atomes identiques de fluor, chlore ou brome en position ortho/ortho', $Q_1$ désigne le radical pyrimidine-4-yle et $R_1$, $R_2$ et $R_3$ en position 2, 5 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, thioallylène, cyclopropyle, nitro, cyano ou $COOR_4$, où $R_4$ représente un radical alkyle en $C_1$-$C_4$, ainsi qu'un radical phényle, phénoxy, thiophényle, benzyle, diméthylamino, pipéridinyle, pyrrolidinyle, porpholinyle, imidazolyle ou 1,2,4-triazolyle.

**11.** Procédé selon la revendication 1, caractérisé en ce qu'on prépare des composés de formule I dans lesquels X représente le chlore, $Q_1$ désigne le radical pyrimidine-4-yle et $R_1$, $R_2$ et $R_3$ en position 2, 5 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$, ainsi qu'un radical phényle, phénoxy, thiophényle, benzyle ou diméthylamino.

**12.** Procédé selon la revendication 1, caractérisé en ce qu'on prépare des composés de formule I dans lesquels X représente des atomes identiques de fluor, chlore, brome ou iode en position ortho/ortho', $Q_2$ désigne le radical pyrimidine-2-yle et $R_1$, $R_2$ et $R_3$ en position 4, 5 ou 6 désignent l'hydrogène ou un groupe halogéno, alkyle en $C_1$-$C_4$, halogénoalkyle en $C_1$-$C_2$ ayant 1 à 3 atomes d'halogène, alcoxy en $C_1$-$C_3$, thioalkyle en $C_1$-$C_4$, halogénoalcoxy en $C_1$-$C_2$ ayant 1 à 3 atomes d'halogène, alcényloxy en $C_3$-$C_4$, alcynyloxy en $C_3$-$C_4$, cycloalkyle en $C_3$-$C_5$, nitro, cyano ou $COOR_4$, où $R_4$ représente un radical alkyle en $C_1$-$C_4$; ou dans lesquels $R_1$, $R_2$ et $R_3$ désignent un radical phénoxy, thiophényle, thiobenzyle; ainsi que $N(R_5)R_6$, où $R_5$ et $R_6$ représentent, chacun indépendamment l'un de l'autre, un radical alkyle en $C_1$-$C_2$; ou encore un radical pipéridinyle ou morpholinyle ou un radical morpholinyle substitué par un radical alkyle en $C_1$-$C_3$ oule chlore.

**13.** Procédé selon la revendication 1, caractérisé en ce qu'on prépare des composés de formule I dans lesquels X représente des atomes identiques de fluor, chlore ou brome en position ortho/ortho', $Q_2$ désigne le radical pyrimidine-2-yle et $R_1$, $R_2$ et $R_3$ en position 4, 5 ou 6 désignent l'hydrogène ou un radical fluoro, chloro ou bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$; ou encore désignent un radical phényle, phénoxy, thiophényle, benzyle ou diméthylamino.

**14.** Procédé selon la revendication 1, caractérisé en ce qu'on prépare des composés de formule I dans lesquels X représente le chlore, $Q_2$ désigne le radical pyrimidine-2-yle et $R_1$, $R_2$ et $R_3$ en position 4, 5 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$; ou encore désignent un radical phényle, phénoxy, thiophényle, benzyle ou diméthylamino.

**15.** Procédé selon la revendication 1, caractérisé en ce qu'on prépare des composés de formule I dans lesquels X représente des atomes identiques de fluor, chlore, brome ou iode en position ortho/ortho', $Q_3$ désigne le radical pyrimidine-5-yle et $R_1$, $R_2$ et $R_3$ désignent l'hydrogène ou un radical halogéno ou alkyle en $C_1$-$C_4$, halogénoalkyle en $C_1$-$C_2$ ayant 1 à 3 atomes d'halogène, alcoxy en $C_1$-$C_5$, alcoxyalkyle en $C_2$-$C_4$, thioalkyle en $C_1$-$C_4$, halogénoalcoxy en $C_1$-$C_3$ ayant 1 à 4 atomes d'halogène, alcényloxy en $C_3$-$C_4$, alcynyloxy en $C_3$-$C_4$, cycloalkyle en $C_3$-$C_5$, phénoxy, thiophényle ou benzyl-oxy ou phénoxy, thiophényle ou benzyloy substitué par des atomes d'halogène; ainsi que $N(R_5)R_6$, où $R_5$ et $R_6$ représentent, chacun indépendamment l'un de l'autre, un radical alkyle en $C_1$-$C_2$; ou encore un radical pyrrolidinyle ou morpholinyle.

**16.** Procédé selon la revendication 1, caractérisé en ce qu'on prépare des composés de formule I dans lesquels X représente des atomes identiques de fluor, chlore, brome ou iode en position ortho/ortho', $Q_3$ désigne le radical pyrimidine-5-yle et $R_1$, $R_2$ et $R_3$ en position 2, 4 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclopropyle, nitro, cyano ou $COOR_4$, où $R_4$ représente un radical alkyle en $C_1$-$C_4$; ou encore désignent un radical phényle, phénoxy, thiophényle, benzyle ou diméthylamino.

**17.** Procédé selon la revendication 1, caractérisé en ce qu'on prépare des composés de formule I dans lesquels X représente le chlore, $Q_3$ désigne le radical pyrimidine-5-yle et $R_1$, $R_2$ et $R_3$ en position 2, 4 ou 6 désignent l'hydrogène ou un radical fluoro, chloro, bromo, alkyle en $C_1$-$C_4$, halogénoalkyle en $C_1$-$C_2$ ayant 1 à 3 atomes de fluor ou de chlore, alcoxy en $C_1$-$C_3$, alcoxyalkyle en $C_2$-$C_4$, thioalkyle en $C_1$-$C_4$, halogénoalcoxy en $C_1$-$C_2$ ayant 1 à 3 atomes de fluor ou de chlore, alcényloxy en $C_3$-$C_4$, alcynyloxy en $C_3$-$C_4$, cyclo-propyle, nitro, cyano ou $COOR_4$, où $R_4$ représente un radical alkyle en $C_1$-$C_4$; ou encore désignent un radical phényle, phénoxy, thiophényle, benzyle ou diméthylamino.

**18.** Procédé selon la revendication 1, caractérisé en ce qu'on prépare un composé choisi dans le groupe des composés suivants:
amide d'acide N-(2-cyclopropyl-5-éthyl-6-chloro-pyrimidine-4-yl)-2,6-dichloro-isonicotinique;
amide d'acide N-(2-cyclopropyl-5-méthyl-6-chloro-pyrimidine-4-yl)-2,6-dichloro-isonicotinique;
amide d'acide N-(2,5-diméthyl-6-chloro-pyrimidine-4-yl)-2,6-dichloro-isonicotinique;
amide d'acide N-(2,5-diéthyl-6-chloro-pyrimidine-4-yl)-2,6-dichloro-isonicotinique;
amide d'acide N-(2-chloro-6-thiométhyl-pyrimidine-4-yl)-2,6-dichloro-isonicotinique;
amide d'acide N-(2-méthoxy-5-chloro-pyrimidine-4-yl)-2,6-dichloro-isonicotinique;
amide d'acide N-(2,5-dichloro-pyrimidine-4-yl)-2,6-dichloro-isonicotinique;
amide d'acide N-(2,6-diméthyl-pyrimidine-4-yl)-2,6-dichloro-isonicotinique;
amide d'acide N-(5-méthyl-6-chloro-pyrimidine-4-yl)-2,6-dichloro-isonicotinique;
amide d'acide N-(5-n-propyl-6-chloro-pyrimidine-4-yl)-2,6-dichloro-isonicotinique;
amide d'acide N-(5-éthyl-6-chloro-pyrimidine-4-yl)-2,6-dichloro-isonicotinique;
amide d'acide N-(2,5,6-trichloro-pyrimidine-4-yl)-2,6-dichloro-isonicotinique;
amide d'acide N-(2-diméthylamino-6-méthoxy-pyrimidine-4-yl)-2,6-dichloro-isonicotinique;
amide d'acide N-(2-éthyl-5-méthyl-6-chloro-pyrimidine-4-yl)-2,6-dichloro-isonicotinique;
amide d'acide N-(2-trifluorométhoxy-5-chloro-pyrimidine-4-yl)-2,6-dichloro-isonicotinique;
amide d'acide N-(2-méthoxy-5-fluoro-pyrimidine-4-yl)-2,6-dichloro-isonicotinique;
amide d'acide N-(2-chloro-5-fluoro-pyrimidine-4-yl)-2,6-dichloro-isonicotinique;
amide d'acide N-(2-isopropoxy-5-chloro-pyrimidine-4-yl)-2,6-dichloro-isonicotinique;
amide d'acide N-(2-isopropyl-6-chloro-pyrimidine-4-yl)-2,6-dichloro-isonicotinique;
amide d'acide N-(2-chloro-6-n-propyl-pyrimidine-4-yl)-2,6-dichloro-isonicotinique;
amide d'acide N-(2-n-propoxy-5-chloro-pyrimidine-4-yl)-2,6-dichloro-isonicotinique;
amide d'acide N-(2-isopropyl-6-éthylthio-pyrimidine-4-yl)-2,6-dichloro-isonicotinique;
amide d'acide N-(2-isopropyl-6-isopropylthio-pyrimidine-4-yl)-2,6-dichloro-isonicotinique;
amide d'acide N-(2-chloro-6-méthyl-pyrimidine-4-yl)-2,6-dichloro-isonicotinique;
amide d'acide N-pyridine-2-yl-2,6-dichloro-isonicotinique;
amide d'acide N-(6-méthyl-pyridine-2-yl)-2,6-dichloro-isonicotinique;
amide d'acide N-(4-méthyl-pyridine-2-yl)-2,6-dichloro-isonicotinique;
amide d'acide N-(5-chloro-pyridine-2-yl)-2,6-dichloro-isonicotinique;
amide d'acide N-pyridine-4-yl-2,6-dichloro-isonicotinique.

**19.** Procédé pour la préparation de composés de formule V

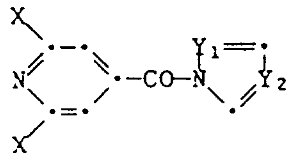

(V)

par réaction de composés de formule II

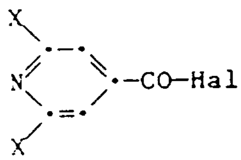

(II)

avec un azole de formule VI

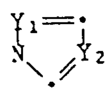

(VI)

en présence d'une base dans un solvant inerte, tandis que X a les significations indiquées sous la formule I, Hal désigne un atome d'halogène, de préférence le chlore, et $Y_1$ et $Y_2$ représentent, indépendamment l'un de l'autre, N ou CH.